# EUROPÄISCHE PATENTANMELDUNG

(11) **EP 1 619 196 A1**
(43) Veröffentlichungstag der Anmeldung: **25.01.2006**
(21) Anmeldenummer: 04017542.4
(22) Anmeldetag: 23.07.2004
(51) Int. Cl.: C07D 495/14, C07D 491/14, A61K 31/519, A61P 9/10

(54) **Substituierte Pyrido[3',2':4,5]thieno[3,2-d]pyrimidine und Pyrido[3',2':4,5]furo[3,2-d]-pyrimidine zur Verwendung als Inhibitoren der PDA-4 und/oder TNF-alpha Freisetzung**

(71) Anmelder: Curacyte Discovery GmbH, 04103 Leipzig (DE)
(72) Erfinder: Reichelt, Claudia, 04299 Leipzig (DE); Ludwig, Alexander, 04275 Leipzig (DE); Schulze, Alexander, 04317 Leipzig (DE); Daghish, Mohammed, 04107 Leipzig (DE); Leistner, Siegfried, 04340 Leipzig (DE)
(74) Vertreter: Schüssler, Andrea

(57) **Zusammenfassung**

Die Erfindung betrifft substituierte Pyrido[3',2':4,5]thieno[3,2-d]pyrimidine (Y=S) und substituierte Pyrido[3',2':4,5]furo[3,2-d]pyrimidine (Y=O) der allgemeinen Formel1

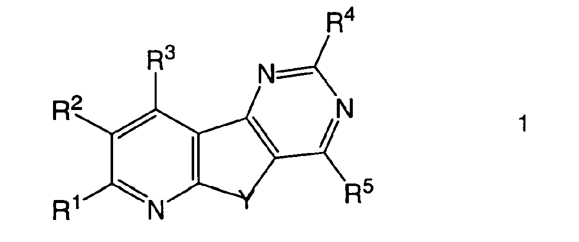

Verfahren zu deren Herstellung, pharmazeutische Zubereitungen, die diese Verbindungen und/oder daraus herstellbare physiologisch verträgliche Salze und/oder deren Solvate enthalten sowie die pharmazeutische Verwendung dieser Verbindungen, deren Salze oder Solvate als Inhibitoren der Phosphodiesterase 4 und/oder TNF alpha-Freisetzung. Die Verbindungen stellen Wirkstoffe zur Behandlung jener Erkrankungen dar, die durch Hemmung der Aktivität von Phosphodiesterase 4 und/oder TNF alpha-Freisetzung, z.B. in Lymphozyten, eosinophilen und basophilen Granulozyten, Makrophagen und Mastzellen, positiv zu beeinflussen sind.

## Beschreibung

### Technisches Gebiet

Die Erfindung betrifft substituierte Pyrido[3',2':4,5]thieno[3,2-d]pyrimidine (Y=S) und substituierte Pyrido[3',2':4,5]furo[3,2-d]pyrimidine (Y=O) der allgemeinen Formel 1, 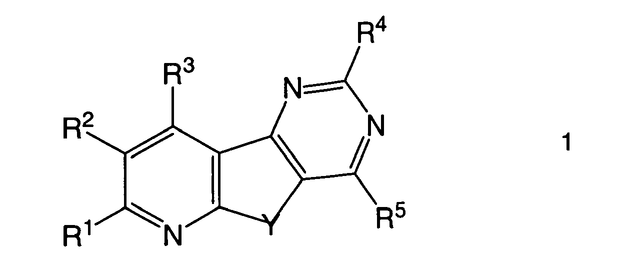 Verfahren zu deren Herstellung, pharmazeutische Zubereitungen, die diese Verbindungen und/oder daraus herstellbare physiologisch verträgliche Salze und/oder deren Solvate enthalten sowie die pharmazeutische Verwendung dieser Verbindungen, deren Salze oder Solvate als Inhibitoren der Phosphodiesterase 4. Die Verbindungen stellen Wirkstoffe zur Behandlung jener Erkrankungen dar, die durch Hemmung der Aktivität von Phosphodiesterase 4 und/oder TNF alpha-Freisetzung, z.B. in Lymphozyten, eosinophilen und basophilen Granulozyten, Makrophagen und Mastzellen, positiv zu beeinflussen sind.

### Stand der Technik

Die zyklischen Nukleotide cAMP und cGMP werden als intrazelluläre sekundäre Botenstoffe (second messengers) zusammengefasst und spielen bei vielfältigsten physiologischen aber auch bei pathophysiologischen Prozessen in lebenden Organismen eine zentrale Rolle. Bei hohen intrazellulären Konzentrationen dieser Nukleotide binden sich diese an spezifische Proteinkinasen, insbesondere an die Proteinkinase A und die Proteinkinase B, und aktivieren diese. Die so aktivierten Proteinkinasen sind nun in der Lage, eine Vielzahl von intrazellulären Proteinen zu phosphorylieren, die dann ihrerseits den zellulären Stoffwechsel und die Antwort dieser Zellen auf innere und äußere Signale maßgeblich beeinflussen.

In der lebenden Zelle wird die Konzentration an cAMP und cGMP hauptsächlich durch die Synthese und den enzymatischen Abbau dieser Nukleotide bestimmt. Die Synthese von cAMP und cGMP erfolgt aus ATP und GTP über die Enzyme Adenylatzyklase und Guanylatzyklase, die durch G-Protein-gekoppelte Rezeptoren in der Zellmembran aktiviert werden. Der Abbau dieser zyklischen Nukleotide erfolgt durch spezifische Phosphodiesterasen in der Zelle.

Die Phosphodiesterasen (PDEs) umfassen eine große Superenzymfamilie mit derzeit 11 bekannten Typen, die ihrerseits in zahlreiche Subtypen (isoformen) und noch wesentlich mehr Splicevarianten unterteilt werden. Diese lsoformen unterscheiden sich in ihrer Substratspezifität, Enzymkinetik, Gewebespezifität, Sensitivität gegenüber Inhibitoren und Aktivatoren sowie in ihrer intrazellulären Kompartmentierung *(Giernbycz MA. Phosphodiesterase 4 inhibitors and the treatment of asthma: where are we now and where do we go from here? Drugs [2000]; 59, 193-212).*

Seit 1990 ist ein wachsendes Interesse der pharmazeutischen Industrie zu registrieren, selektive PDE Inhibitoren zu identifizieren und als Wirkstoffkandidaten zur Behandlung von z.B. chronischen Atemwegserkrankungen zu entwickeln.

Die cAMP spezifische PDE-4 stellt nach heutigem Wissensstand das wichtigste lsoenzym von immunkompetenten Zellen und von glatten Muskelzellen des Lungengewebes dar. Die PDE-4 Familie umfasst derzeit vier Gene mit den Genprodukten PDE-4A, PDE-4B, PDE-4C und PDE-4D. Erstmals wurden PDE-4 spezifische Isotypen von Nemoz et al. beschrieben *(Nemoz G, Prigent AF, Moueqqit M, Fougier S, Macovschi O, Pacheco H. Selective inhibition of one of the cyclic AMP phosphodiesterases from rat brain by the neurotropic compound rolipram. Biochem Pharmacol [1985]; 34, 2997-3000).* Alle Subtypen setzen sich aus einer konservierten katalytischen Domäne mit einer Länge von ca. 270 Aminosäuren und zwei in der Länge variierenden zusätzlichen Regionen (UCR1 und UCR2; upstream conserved region) innerhalb des N-Terminus des Moleküls zusammen *(Engels P, Fichtel K, Lubbert H. Expression and regulation of human and rat phosphodiesterase IV. FEBS Letters [1994]; 350, 291-295).* Eine weitere Diversivität in der Expression der PDE-4 lsoformen resultiert durch alternative Splicevarianten und unterschiedliches posttranslationales Prozessing *(Beavo JA, Conti M, Heaslip RJ. Multiple cyclic nucleotide phosphodiesterases. Mol Pharmakol [1994]; 46, 399-405).* Diese vier Subtypen werden in unterschiedlichen Geweben und Zellen differentiell exprimiert. Die PDE-4A bis -4D sind in den meisten immunkompetenten Zellen und in Entzündungszellen vorhanden. Mit Ausnahme der PDE-4B sind die anderen PDE-4 Typen auch in den Epithelzellen der Luftwege nachweisbar. Im menschlichen Gehirn ist gleichfalls eine differentielle Expression der vier Subtypen in Abhängigkeit von bestimmten anatomischen Regionen beschrieben.

In den vergangenen Jahren haben zahlreiche präklinische und klinische Studien die Modulation von Entzündungs- und Immunkompetenten Zellen durch selektive PDE-4 Inhibitoren belegen können und führten damit zu der wissenschaftlich abgesicherten Auffassung über das hohe therapeutische Potential von spezifischen PDE-4 Inhibitoren bei ganz unterschiedlichen entzündlichen Erkrankungen, wie z.B. chronisch obstruktiven Lungenerkrankungen (COPD), Asthma bronchiale, atopischer Dermatitis, Rheumatoider Arthritis (RA), Multiple Sklerose (MS) und zahlreichen neurologischen Erkrankungen *(Doherty AM. Phosphodiesterase 4 inhibitors as novel anti-inflammatory agents. Curr Opin Chem Biol [1999]; 3, 466-473; Essayan DM. Cyclic nucleotide phosphodiesterase (PDE) inhibitors and immunomodulation. Biochem Pharmacol [1999]; 57, 565-573; Dal Piaz V, Giovannoni MP. Phosphodiesterase 4 inhibitors, structurally unrelated to Rolipram, as promising agents for treatment of asthma and other pathologies. Eur J Med Chem [2000]; 35, 463-480).*

Eine zentrale Eigenschaft von selektiven PDE-4 Inhibitoren ist die Hemmung der Freisetzung des wichtigen pro-inflammatorischen Zytokins TNFα aus Entzündungszellen (Makrophagen, Mastzellen, T-Lymphozyten, basophilen und eosinophilen Granulozyten) aber auch aus Fibroblasten, Endothelzellen und Astrozyten. TNFα wiederum ist selbst in der Lage, weitere proi-nflammatorische Zytokine wie den granulocyte-macrophage colony-stimulating factor (GM-CSF) und Interleukine, besonders IL-1 und IL-8, zu stimulieren. Desweiteren aktiviert TNFα neutrophile und eosinophile Granulozyten, Fibroblasten und Endothelzellen, die ihrerseits pro-inflammatorische Zytokine und Proteasen, hauptsächlich MMP's, sezernieren. So stellen auch alle Krankheiten, bei denen TNFα eine zentrale Rolle im Erkrankungsprozess spielt, ein Target für PDE-4 Inhibitoren dar.

In eosinophilen Granulozyten konnten sowohl PDE-3 als auch PDE-4A, B, D, jedoch keine PDE-4C Subtypen nachgewiesen werden. Diese Zellen spielen eine bedeutende Rolle in der Pathophysiologie des Asthmas und sind deshalb auch hinsichtlich der PDE-4 Hemmung intensiv untersucht worden. Die selektive PDE-4 Hemmung bewirkt eine Verminderung der Freisetzung von reaktiven Sauerstoffradikalen und von Leukotrien C₄ *(Sturton RG, Butt NM, Palfai SP, Tudhope SR, Abram TS, Fisher R, Braunlich G, Es-Sayed M. Am J Respir Crit Care Med [2000]; 161, A200).* Die Synthese des eosinophil-derived neurotoxin (EDN), der Komplementkomponente C5ₐ und des platelet activating factor (PAF) wird gleichfalls durch selektive Inhibition der PDE-4 vermindert *(Hatzelmann A, Tenor H, Schudt C. Differential effects of non-selective and selective phosphodiesterase inhibitors on human eosinophil functions. Br J Pharmacol. [1995];114, 821-831).* PDE-4 Inhibitoren blockieren, untersucht in verschiedensten Tiermodellen für Asthma, signifikant das Einwandern von eosinophilen Granulozyten, hemmen die Akkumulation der Zytokine IL-4 und IL-5 in der broncho-alveolären Lavage (BAL) und verbessern die Funktion der Luftwege *(Kanehiro A, Ikemura T, Makela MJ, Lahn M, Joetham A, Dakhama A, Gelfand EW. Inhibition of phosphodiesterase 4 attenuates airway hyperresponsiveness and airway inflammation in a model of secondary allergen challenge. Am J Respir Crit Care Med. [2001];163, 173-184).*

Neutrophile Granulozyten spielen eine Schlüsselrolle bei entzündlichen Erkrankungen. Sie akkumulieren sehr schnell im entzündlichen Gewebe und sezernieren eine Vielzahl biologisch aktiver Mediatoren und Enzyme.
In diesen Zellen wird als cAMP-abbauendes Enzym hauptsächlich die PDE-4 nachgewiesen (*Wang P, Wu P, Ohleth KM, Egan RW, Billah MM. Phosphodiesterase 4B2 is the predominant phosphodiesterase species and undergoes differential regulation of gene expression in human monocytes and neutrophils. Mol Pharmacol. [1999]; 56,170-174*). PDE-4 Inhibitoren suprimieren eine Vielzahl von Abwehrfunktionen dieser Zellen, wie Superoxid-Bildung, Degranulierung, IL-8-Freisetzung, Adhesionsmolekül-Expression, eine stark verminderte Expression der humanen Lungen-Elastase, der MMP-9 und der Leukotrien B₄ Synthese (*Barnette MS, Christensen SB, Essayan DM, Grous M, Prabhakar U, Rush JA, Kagey-Sobotka A, Torphy TJ. SB 207499 (Ariflo), a potent and selective second-generation phosphodiesterase 4 inhibitor: in vitro anti-inflammatory actions. J Pharmacol Exp Ther. [1998]; 284, 420-426; Berends C, Dijkhuizen B, de Monchy JG, Dubois AE, Gerritsen J, Kauffman HF. Inhibition of PAF-induced expression of CD11b and shedding of L-selectin on human neutrophils and eosinophils by the type IV selective PDE inhibitor, rolipram. Eur Respir J. [1997]; 10, 1000-1007).* Eine pathophysiologische Hauptkonsequenz dieser Zellfunktionsänderung durch PDE-4 Inhibitoren ist in deren stark verminderter Adhäsion und Chemotaxis in Richtung des Entzündungsherdes zu sehen, es kommt somit nicht zum Einwandern der Entzündungszellen.

PDE-4 Inhibitoren blockieren in vivo in zahlreichen COPD Tiermodellen die Neutrophilie in der BAL und damit einen wesentlichen pathologischen Entzündungsweg *(Spond J, Chapman R, Fine J, Jones H, Kreutner W, Kung TT, Minnicozzi M. Comparison of PDE 4 inhibitors, rolipram and SB 207499 (ariflo), in a rat model of pulmonary neutrophilia. Pulm Pharmacol Ther [2001]; 14, 157-164).*

Das gleichfalls verstärktes Einwandern und die Akkumulation von Monozyten in das entzündliche Lungengewebe ist ein typischer pathophysiologischer Regulationsmechanismus bei diesen Erkrankungen. Als Leitphänomen kann hierbei die Freisetzung von pro-inflammatorischen Zytokinen, besonders von TNFα und GM-CSF und zahlreicher proteolytischer Enzyme angesehen werden. Selektive PDE-4 Inhibitoren blockieren die Synthese und Freisetzung von TNFα, GM-CSF und Proteasen und stimulieren die PGE₂ Produktion. Überraschenderweise wird die Synthese des anti-inflammatorische Zytokin IL-10 durch selektive PDE-4 Inhibitoren stark stimuliert *(Suda Y, Tamura G, Ohno I, Maeda K, Liu Y, Yamauchi K, Kurimoto F, Shirato K. Effects of phosphodiesterase inhibitors on secretions of human monokines. Allergol Int [1998]; 47, 219-224*).

Die im entzündlichen Lungengewebe akkumulierten T-Lymphozyten sezernieren hauptsächlich pro-inflammatorische Zytokine wie IL-2, IL-4, IL-5, IL-13, TNFα, IFNγ und GM-CSF, deren Synthese durch PDE-4 Inhibitoren sehr effektiv gehemmt wird.

Die besonders bei Asthma eingewanderten B-Lymphozyten werden durch spezifische PDE-4 Inhibitoren in ihrer lgE Synthese gehemmt und damit direkt Einfluss auf den IgE-vermittelten allergischen Pathogeneseweg *(Coqueret O, Boichot E, Lagente V. Selective type IV phosphodiesterase inhibitors prevent IL-4-induced lgE production by human peripheral blood mononuclear cells. Clin Exp Allergy [1997]; 27, 816-823).*

Die Epithelzellen der Luftwege sind gleichfalls stark in die entzündlichen Reaktionen bei COPD und Asthma eingebunden. Bei ihrer Aktivierung setzen sie eine Vielzahl von biologisch hochaktiven Substanzen, wie z.B. Metabolite der Arachidonsäure und proinflammatorische Zytokine, wie das TNFα und GM-CSF, frei *(Chipappara G, Merendino AM, Chimenti L, Rilcobono L, Mirabella F, La Rocca AM, Weck PK, Bonsignore G, Vignola AM. In vitro and ex vivo effects of the phosphodiesterase 4 inhibitors. Am J Resp Crit Cara Med [2001]; 163, A278).* In vivo zeigen PDE-4 Inhibitoren durch eine signifikante Hemmung der Synthese und Freisetzung des proinflammatorischen Enzyms MMP-9, einen starken protektiven Effekt auf Epithelzellen der Luftwege, sowohl in Tiermodellen als auch in klinischen Studien *(Rabinovici R, Feuerstein G, Abdullah F, Whiteford M, Borboroglu P, Sheikh E, Phillip DR, Ovadia P, Bobroski L, Bagasra O, Neville LF. Locally produced tumor necrosis factor-alpha mediates interleukin-2-induced lung injury. Circ Res (1996); 78, 329-236; Ortiz JL, Cortijo J, Valles JM, Bou J, Morcillo EJ. Rolipram inhibits airway microvascular leakage induced by platelet-activating factor, histamine and bradykinin in guinea-pigs. J Pharm Pharmacol (1993); 45, 1090-1092).*

Ein erhöhter intrazellulärer cAMP Spiegel senkt die Aktivität von Immun- und Entzündungszellen sowohl in vitro als auch in vivo, wirkt protektiv auf Epithel- und Endothelzellen und vermittelt eine Relaxation der glatten Muskelzellen im Lungengewebe. Diese pharmakologischen Eigenschaften belegen nachhaltig die Bedeutung von PDE Hemmung als relevantes und abgesichertes Target für die therapeutische Intervention bei chronischen entzündlichen Lungenerkrankungen wie COPD und Asthma *(Barnes PJ, Shapiro SD, Pauwels RA. Chronic obstructive pulmonary disease: molecular and cellular mechanisms. Eur Respir J (2003); 22, 672-88).*

Unter COPD wird eine Gruppe von chronisch progressiven, entzündlichen Lungenerkrankungen zusammengefasst, die hauptsächlich durch ein Einwandern von neutrophilen Granulozyten und weiterer immunkompetenter Zellen in das Lungengewebe charakterisiert ist. Hauptsymptome sind hierbei chronischer Husten und Auswurf und die damit verbundene progrediente und irreversible Verschlechterung der Lungenfunktion bis hin zur Maximalvariante, dem Lungenemphysem. Die Erkrankung verläuft schubförmig und ist häufig mit bakteriellen Sekundärinfektionen der Lunge verbunden. Wichtigste Risikofaktoren sind das Rauchen und die Umweltverschmutzung. Durch die teilweise extreme Kurzatmigkeit und die geringe Belastbarkeit der Patienten auf Grund eines pulmonalen Rückstaus und der daraus resultierenden kardialen Probleme ist die Lebensqualität und Lebenserwartung der Patienten stark eingeschränkt. Weltweit leiden ca. 600 Mill. an dieser Erkrankung und ist laut WHO sechsthäufigste Erkrankung weltweit, die auch die vierthäufigste Todesursache ist. Mit einer jährlichen Wachstumsrate von 12% (Visiongain, 2004), wird sie global in den nächsten 20 Jahren die dritthäufigste Todesursache sein. Analysten schätzen deshalb ein, dass es sich bei dieser Erkrankungsgruppe um den mit am schnellsten wachsenden globalen therapeutischen Markt handelt. Die gegenwärtige Standardtherapie setzt sich aus der Gabe von β₂-Agonisten, muscarinergen Antagonisten, Kortikosteroiden und Antihistaminika zusammen. Diese Behandlungsstrategien sind jedoch nur symptomatischer Natur, ohne kausal in den progressiven Verlauf der Erkrankung einzugreifen. Es besteht deshalb seit Jahren die berechtigte Forderung mittels neuer Therapieansätze kausal in den Krankheitsprozess einzugreifen und damit insbesondere den fortschreitenden Charakter von COPD zu bekämpfen.

Beim Asthma bronchiale handelt es sich um eine anfallsweise, nichtinfektiöse und meist allergisch ausgelöste Lungenerkrankung, die hauptsächlich durch ein Einwandern von eosinophilen Granulozyten und weiteren immunkompetenten Zellen in das Lungengewebe und durch eine temporäre Bronchokonstriktion charakterisiert ist. Nach längerer Krankheitsdauer tritt vielfach eine chronische Bronchitis mit einem Lungenemphysem und Bronchiektasien bis hin zum Cor pulmonale auf. Die Lebensqualität der Patienten ist durch die Atemnot bis hin zu Dyspnoe Attacken, die erhebliche Schleimsekretion und starke Angstgefühle merklich eingeschränkt. In den Industrieländern leiden ca. 5% der erwachsenen Bevölkerung und nahezu 10% der Kinder an dieser Erkrankung. Weltweit geht man von einer Prävalenz von 155 Mill. Erkrankten bei einer jährlichen Steigerungsrate von 10 - 15% (aus Lead Discovery, 2003). Die seit 25 Jahren übliche Standardtherapie mit β₂-Adeno-rezeptor-Agonisten (Bronchodilatation) und Kortikosteroide (antientzündlich) zeigt neben einem guten therapeutischen Profil auch teilweise erhebliche unerwünschte Nebenwirkungen, besonders im Kindesalter (bekannte Glukokortikoid-Nebenwirkungen und Tachykardie, Herzklopfen, Kopfschmerz bei β₂- Adenorezeptor Agonisten). Es besteht auch bei diesem Krankheitsbild die Forderung nach neuen wirksameren Therapiestrategien, die nicht nur symptomatisch, sondern kausal in den Krankheitsprozess eingreifen.

Selektive PDE-4 Inhibitoren sollten daher zusammengefasst zumindest folgende invivo Wirkungen haben:
1. effektive Hemmung der TNFa-Synthese und -Freisetzung in Blutzellen;
2. signifikante Verminderung der TNFα Konzentration in der Broncho-alveolären Lavageflüssigkeit (BAL);
3. signifikante Verminderung von IL-4 und IL-5 in der BAL Flüssigkeit;
4. signifikante Hemmung des Einwanderns von eosinophilen Granulozyten in die Lunge;
5. signifikante Hemmung des Einwanderns von neutrophilen Granulozyten in die Lunge;
6. Verhinderung des "oxidativen Burst";
7. Verhinderung der Bronchokonstriktion;
8. Verhinderung der Ausbildung von Lungenödemen (Emphysem);
9. Verhinderung der Proliferation und Hyperplasie von Zellen der Luftwege;
10. signifikante Hemmung der MMP-9 Aktivität in der BAL Flüssigkeit;
11. signifikante Hemmung der TGF-β Aktivität in der BAL Flüssigkeit.

In neuerer Zeit belegen experimentelle Daten auch den erfolgversprechenden Einsatz von PDE-4 Inhibitoren als immunmodulatorische Wirkstoffe. Das therapeutische Potential von PDE-4 Inhibitoren wurde erfolgreich in Tiermodellen für die atopische Dermatitis (*Hanifin JM, Chan SC, Cheng JB, Tofte SJ, Henderson WR Jr, Kirby DS, Weiner ES. Type 4 phosphodiesterase inhibitors have clinical and in vitro anti-inflammatory effects in atopic dermatitis. J Invest Dermatol (1996);107, 51-*56), die Rheumatoide Arthritis *(Laemont KD, Schaefer CJ, Juneau PL, Schrier DJ. Effects of the phosphodiesterase inhibitor rolipram on streptococcal cell wall-induced arthritis in rats. lnt J Immunopharmacol (1999); 21,* 711-725), Colitis ulcerosa *(Hartmann G, Bidlingmaier C, Siegmund B, Albrich S, Schulze J, Tschoep K, Eigler A, Lehr HA, Endres S. Specific type IV phosphodiesterase inhibitor rolipram mitigates experimental colitis in mice. J Pharmacol Exp Ther (2000); 292, 22-30),* Multiple Sklerose *(Dinter H, Onuffer J, Faulds D, Perez HD. Phosphodiesterase type IV inhibitors in the treatment of multiple sclerosis. J Mol Med (1997); 75, 95-102),* Crohn's Disease *(Prehn JL, Landers C, Muller GW, Man HW, Stirling DI, Targan SR. Potent inhibition of cytokine production from intestinal lamina propria T cells by phosphodiesterase-4 inhibitory thalidomide analogues. J Clin Immunol (2001); 21,* 357-364), Entzündungsschmerz *(Cunha FQ, Teixeira MM, Ferreira SH. Pharmacological modulation of secondary mediator systems--cyclic AMP and cyclic GMP--on inflammatory hyperalgesia. Br J Pharmacol (1999);127, 671-678),* Septischer Schock *(Cardelus I, Gras J, Jauregui J, Llenas J, Palacios JM. Inhibition of lipopolysaccharide-induced bowel erythrocyte extravasation in rats, and of mesenteric hypoperfusion in dogs, by phosphodiesterase inhibitors. Eur J Pharmacol (1996); 299, 153-159),* Leishmaniose *(Rascon A. Cyclic nucleotide phosphodiesterases: diversity, classification, structure and function. Acta Cient Venez (1997); 48, 145-153)* und HIV Infektionen *(Sun Y, Li L, Lau F, Beavo JA, Clark EA. lnfection of CD4+ memory T cells by HIV-1 requires expression of phosphodiesterase 4. J Immunol (2000); 165, 1755-1761)* nachgewiesen.

Klinisch zeigten die meisten der bisher getesteten PDE-4 Inhibitoren, mit Rolipram als Leitstruktur, unerwünschte Arzneimittelwirkungen (UAW) und fehlende in vivo Wirksamkeit, die ihren klinischen Einsatz bisher limitierten. Dazu zählen emetische Wirkungen wie Übelkeit und Erbrechen, aber auch gastro-intestinale Beschwerden, kardiovaskuläre und zentrale Nebenwirkungen *(Zeller E, Stief HJ, Pflug B, Sastre-y-Hernandez M. Results of a phase II study of the antidepressant effect of rolipram. Pharmacopsychiatry (1984); 17, 188-190; Puurunen J, Lucke C, Schwabe U. Effect of the phosphodiesterase inhibitor 4-(3-cyclopentyloxy-4-methoxyphenyl)-2-pyrrolidone (ZK 62711) on gastric secretion and gastric mucosal cyclic AMP. Naunyn Schmiedebergs Arch Pharmacol (1978); 304, 69-75; Nicholson CD. Cyclic nucleotide phosphodiesterase isoenzymes and asthma--outstanding issues. Agents Actions Suppl (1993); 43, 3-12).*

Ausgehend von Rolipram wurden in der Folgezeit zahlreiche strukturbasierte Abwandlungen entwickelt, die aber alle ein ähnliches Nebenwirkungsprofil aufweisen *(Schneider HH, Schmiechen R, Brezinski M, Seidler J. Stereospecific binding of the antidepressant rolipram to brain protein structures. Eur J Pharmacol (1986); 127, 105-115).* Die heute am weitesten entwickelten PDE-4 Inhibitoren sind Cilomilast (Fa. **Glaxo-SmithKline)** *(Compton CH, Gubb J, Nieman R, Edelson J, Amit O, Bakst A, Ayres JG, Creemers JP, Schultze-Werninghaus G, Brambilla C, Barnes NC; International Study Group. Cilomilast, a selective phosphodiesterase-4 inhibitor for treatment of patients with chronic obstructive pulmonary disease: a randomised, dose-ranging study. Lancet (2001); 358, 265-270; Compton C, Edelson JD, Cedar E et al. Cilomilast (Ariflo) 15mg bid safety in a six month clinical trial program. Am J Resp Crit Care Med (2001); 163, A909; Zussman BD, Benincosa LJ, Webber DM, Clark DJ, Cowley H, Kelly J, Murdoch RD, Upward J, Wyld P, Port A, Fuder H. An overview of the pharmacokinetics of cilomilast (Ariflo), a new, orally active phosphodiesterase 4 inhibitor, in healthy young and elderly volunteers. J Clin Pharmacol (2001); 41, 950-958; Giembycz MA. Cilomilast: a second generation phosphodiesterase 4 inhibitor for asthma and chronic obstructive pulmonary disease. Expert Opin Investig Drugs (2001); 10, 1361-1379)* und Roflumilast (Fa. Altana) ( *Schmidt BM, Kusma M, Feuring M, Timmer WE, Neuhauser M, Bethke T, Stuck BA, Hormann K, Wehling M. The phosphodiesterase 4 inhibitor roflumilast is effective in the treatment of allergic rhinitis. J Allergy Clin Immunol (2001); 108, 530-536; SCRIP 2644, p.25*), die strukturell große Ähnlichkeiten zu Rolipram aufweisen, aber offensichtlich ein besseres Wirkprofil besitzen. Roflumilast ist im Vergleich zu Cilomilast mit einem IC₅₀ Wert von 0,8 nM ein wesentlich potenterer PDE-4 Inhibitor und zeigt auch in zahlreichen in vitro und in vivo Untersuchungen eine stärkere anti-inflammatorische Aktivität *(Hatzelmann A, Schudt C. Anti-inflammatory and immunomodulatory potential of the novel PDE4 inhibitor roflumilast in vitro. J Pharmacol Exp Ther (2001); 297, 267-79).* Beide Wirkstoffe haben trotz eines Nebenwirkungsprofils erfolgreich die klinische Studie Phase III für COPD und Asthma abgeschlossen, sind jedoch noch nicht von den zuständigen Behörden zugelassen worden.

Die bisher entwickelten PDE-4 Inhibitoren, die zum Teil ein umfangreiches Nebenwirkungsprofil aufweisen, lassen sich hauptsächlich in folgende chemische Hauptklassen untergliedern:
1. Catecholetherverbindungen mit struktureller Ähnlichkeit zu Rolipram, Cilomilast und Roflumilast,
2. Chinazolindione mit struktureller Ähnlichkeit zu Nitraquazone,
3. Xanthin-Derivate mit struktureller Ähnlichkeit zu Theophyllin und
4. Benzofuran-Derivate.

Es besteht deshalb der dringende Bedarf, neuartige nebenwirkungsarme PDE-4 Inhibitoren mit besserer therapeutischer Breite zu entwickeln und in die Klinik einzuführen.

Die Aufgabe der vorliegenden Erfindung ist daher neue Verbindungen bereitzustellen, die selektiv die PDE-4 hemmen.

Gelöst wird diese Aufgabe durch die nachfolgend gezeigten Verbindungen aus der Klasse der Pyrido[3',2':4,5]thieno[3,2-d]pyrimidin- und Pyrido[3',2':4,5]furo[3,2-d]pyrimidin-Derivate,

In bisher nur wenigen Fällen ist über biologisch aktive Pyrido[3',2':4,5]thieno-[3,2-d]pyrimidin-Derivate berichtet worden:
So wurden von *J.M. Quintela et al.: Bioorg. Med. 6 (1998) 1911-1925,* die Synthese von mehreren 2-Dimethylamino-(oder 2-H)-4-sek.amino-7-ethoxy-8-cyano-(oder 8-H)-9-phenyl- und auch das strukturanaloge 4-Ethoxy-pyrido[3',2':4,5]thieno-[3,2-d]pyrimidin-Derivat beschrieben. Einige dieser Verbindungen zeigten eine die Histamin-Freisetzung aus Mastzellen von Ratten inhibierende Wirkung. *Abdel-Rahmann et al.: Pharmazie 58 (2003) 372-377* berichteten über die Synthese von 8-Acetyl-3-amino-7-methyl-4-imino-9-subst.phenyl-pyrido[3',2':4,5]thieno-[3,2-d]-pyrimidinen mit antimikrobieller Wirksamkeit. Weiterhin wurden Derivate dieses Heterosystems u.a mit cholesterinsenkender *(C.J.Shishoo, M.B.Devani und V.S.Bhadti: Indian Patent151.456 (1983); Chem. Abstr.: 100, 209858 (1984);* und *V.P.Arya; Drugs Future, 10, 123 (1985)), mit analgetischer (C.G.Dave et al.: J. Indian Chem.Soc. 66, 48 (1989)),* mit antipyretischer *(E.Bousquet et al.: Farmaco Ed.Sci. 40, 869(1985); und E.Bousquet et al.: Farmaco Ed.Sci. 39, 110 (1984)),* mit antianaphylaktischer *(H. Vieweg, S.Leistner, G. Wagner* et al.: *Patent DD 257830 (1988); Chem. Abstr.: 110, 95262p (1989); und H. Vieweg, S.Leistner, G. Wagner et al.: East German Patent DD 258234 (1988)),* mit antiinflammatorischer *(E.F.Elslager, P.W.Jacob* und *M.Leslic: J.Heterocyclic Chem. 9, 775 (1972);* und *M.Chaykovsky* et al.: *J.Med.Chem. 10, 188 (1973);* und *L.A.Radinovskaya* und *A.Sharanin: Khim.Geterotsikl.Soedin. 805 (1988);* und *S.Leistner* et al.: *Pharmazie 41, 54 (1986));* mit klinisch effektiver antiallergischer *(G.D.Madding* und *M.D.Thompson: J.Heterocyclic Chem. 24, 581 (1987))* und mit potentiell antineoplastischer Wirksamkeit *(C.C.Cheng in Progress in Medicinal Chemistry 25, 35 (1989) beschrieben.*

Als Inhibitoren der PDE 4 sind Pyrido[3',2':4,5]thieno[3,2-d]pyrimidin- und Pyrido[3',2':4,5]furo[3,2-d]pyrimidin-Derivate bisher jedoch völlig unbekannt.

### Detailierte Beschreibung der Erfindung

Die Erfindung betrifft substituierte Pyrido[3',2':4,5]thieno[3,2-d]pyrimidine und Pyrido[3',2':4,5]furo[3,2-d]pyrimidine der allgemeinen Formel 1 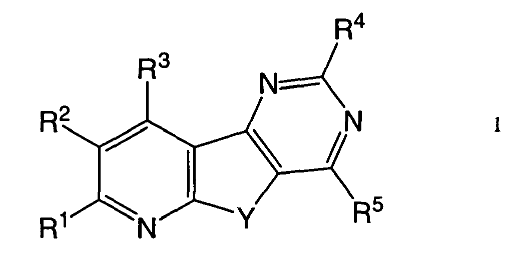
worin bedeuten:
- Y: Schwefel oder Sauerstoff und
- R¹ und R³,: gleich oder ungleich unabhängig voneinander,
- Wasserstoff,
- C₁₋₁₀Alkyl (ggf. mit R^{§} substituiert),
- C_{2-!2} Alkenyl und C_{2-!2} Alkinyl (jeweils ggf. mit R^{§} substituiert ),
- Difluormethyl, Trifluormethyl,
- Benzyl (ggf. mit R^{§} substituiert), Phenyl-(C₂₋₆)alkyl , Phenyl, 4-R^{§} -
- Phenyl, 3-R^{§}-Phenyl, 2-R^{§}- Phenyl, 3-R^{§},4-R^{§}-Phenyl, 3-R^{§},4-R^{§}-,5-R^{§} -Phenyl, 2R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H SO₂OAlk (mit "Alk": C₁₋₅),
- Chlor, Brom, Iod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
- _{R}²: - Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAlk und CONAlk₂ (mit "Alk" jeweils C₁₋₆),
- R*CO- (worin R* Wasserstoff, C₁-C₁₂Alkyl bedeuten sowie ggf. mit R^{§} substituiert),
- Cyano, Nitro, Amino, C₁₋₆Alkylamino, Di(C₁₋₆)alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
- _{R}⁴: - C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen Reste mit R§ substituiert);
- Phenyl, 4-R^{§} -Phenyl, 3-R^{§} -Phenyl, 2-R^{§}-Phenyl (jedoch ausgenommen: 2-NO₂-Phenyl), 3-R^{§},4-R^{§}-Phenyl, 3-R^{§},4-R^{§}-,5-R^{§-}Phenyl,
- 1-Naphthyl, 2-Naphthyl (jeweils ggf. mit R^{§} substituiert);
- S-Alkyl, SO-Alkyl, SO₂-Alkyl (jeweils C₁-C₈), S-Alkenyl, SO-Alkenyl, SO₂-Alkenyl (jeweils C₂-C₈) S-Alkinyl, SO-Alkinyl, SO₂-Alkinyl (jeweils C₂-C₆) (ggf. jeweils am C-Skelett der vorgenannten aliphatischen Reste mit -OH, -CN, -SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert;
- OR⁶, worin R⁶ bedeutet
   - CH₃, C₂H₅, (CH₂) ₂CH₃, (CH₂) ₃CH₃, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
   - C₃₋₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
   - C₂-₅Alkenyl und C₂-₅Alkinyl,
   - C₃-₇ Cycloalkenyl,
   - Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
   - 1-Naphthyl, 2-Naphthyl (ggf. jeweils mit R^{§} substituiert),
   - Pyridyl, Isochinolinyl, Chinolinyl, Acridinyl;
- NR⁷R⁸, worin dieser Substituent insgesamt bedeutet:
   - Morpholino, Thiomorpholino, Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
   - weitere Amino-Reste sekundärer, mono- oder polycyclischer cycloaliphatischer Amine mit insgesamt 5-14 Ringatomen, einschließlich der am C-Skelett mit R^{§} substituierte Vertreter;
   - Amino-Reste sekundärer aliphatischer und aromatischer Amine, R⁷R⁸NH,

   worin R⁷ und R⁸ unabhängig voneinander gleich oder ungleich sein können und bedeuten:
   - C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
   - Aminoreste primärer Amine, R⁷NH₂, ( worin R⁷ dasselbe wie oben bedeutet),
   - Amino, NH₂, mit der Einschränkung, dass dann R⁵ nur die Bedeutung von OR⁶ hat ;
- R⁵: - OR⁶, wobei R⁶ dasselbe wie oben bedeutet,
- NR⁷R⁸, mit gleicher Bedeutung wie oben, jedoch mit der Einschränkung, dass R⁴ dabei nicht ebenfalls die Bedeutung von NR ⁷R⁸ aufweist,
- Azido, Hydroxylamino, O-(C₁₋₃)Alkylhydroxylamino,
- N-(C₁₋₃)Alkylhydroxylamino, N,N-Di(C₁-₃)alkylhydroxylamino,
- Hydrazino, (C₁-₄)Alkyl- und Di(C₁-₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;

Ausgenommen vom Schutz ist folgende Verbindung der Formel 1:
Y = S, R¹ = OC₂H₅, R² = CN, R³ = C₆H₅, R⁴ = N(C₂H₅)₂, R⁵ = OC₂H₅

Der oben erwähnte Ausdruck "ggf. mit R^{§} substituiert" bedeutet, daß die genannten Reste einfach oder mehrfach, gleich oder ungleich unabhängig voneinander, substituiert sein können, wobei R^{§} folgende Bedeutung hat:
- OH, -SH, -O-C₁₋₈Alkyl, -O-C₆₋₁₄Aryl, -S-C₁₋₄Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl, -(CO)C₁₋₈Alkyl,
- COOH, -COOC_{1...8}Alkyl, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- CH₃, -CHF₂, -CF₃, -C₂H₅, -C(CH₃)₂, -(CH₂) ₂CH₃, -(CH₂) ₃CH₃, -CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -Cylopropyl, -Cylopropylmethyl,
- Cylobutyl, -Cyclobutylmethyl, -Cyclopentyl, -Cyclopentylmethyl,
- Cylohexyl, -Cyclohexylmethyl,
- F, -Cl, -Br, -I, -CN, -NO₂, und -SCN.

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die verzweigten möglichen Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

Von den Verbindungen der allgemeinen Formel 1 sind als besondere Ausführungsform der Erfindung die Verbindungen der allgemeinen Formeln 1 a und 1 b hervorzuheben, 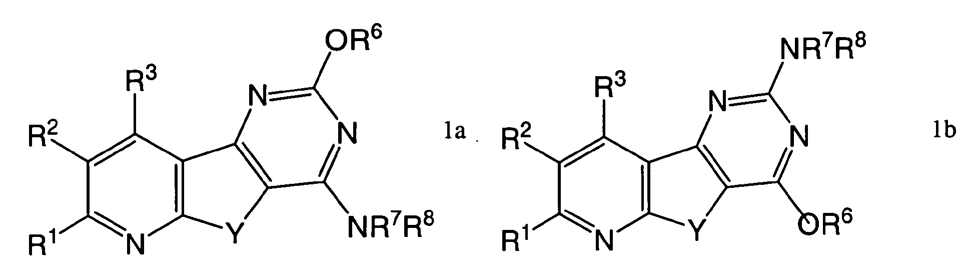 worin R¹, R², R³, R⁶, R⁷, R⁸ und Y die oben genannten Bedeutungen besitzen.

Die Erfindung betrifft auch physiologisch verträgliche Salze der Verbindungen der allgemeinen Formeln 1, 1 a und 1 b.

Die physiologisch verträglichen Salze werden auf übliche Weise durch Umsetzung basischer Verbindungen der allgemeinen Formeln 1, 1 a und 1b mit anorganischen oder organischen Säuren, ggf. auch bei Vorliegen von Verbindungen mit aciden Eigenschaften, wenn z.B. einer der Substituenten R¹, R², R³, R⁴ oder R⁵ in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxybenzoesäuren, Ascorbinsäure, C₁-C₃,Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formeln 1, 1 a und 1b dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quarternären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formeln 1, 1 a und 1b einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Insbesondere, wenn ein solches Prodrug dieser erfindungsgemäßen Verbindungen einem Patienten appliziert wurde, und dieses Prodrug in eine Substanz der allgemeinen Formel 1, 1 a, und 1b transformiert wird, wodurch der gewünschte pharmakologische Effekt erzielt wird.

Die durch die erfindungsgemäßen Verbindungen behandelbaren Erkrankungen schließen alle ein, bei denen das Enzym Phosphodiesterase (PDE4) eine Rolle spielt, z.B. Asthma bronchiale, COPD, Rheumatoide Arthritis (RA), Osteoarthritis, Multiple Sklerose, Guillain-Barré Syndrom, Mb. Crohn, Colitis ulcerosa, Psoriasis, atopische Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ 1, neurodegenerative Erkrankungen, insbesondere Mb. Alzheimer und Mb Parkinson, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen und hier insbesondere Tumoren des blutbildenden Systems wie z.B. Leukämien und Lymphome, virale Erkrankungen und hier insbesondere retrovirale Erkrankungen wie z.B. das erworbene Immundefizienz Syndrom (AIDS) und Myastenia Gravis.

Die durch die erfindungsgemäßen Verbindungen behandelbaren Erkrankungen schließen auch die in der Veterinärmedizin auftretenden mit ein, insbesondere das Asthma bronchiale, die COPD und Dermatiditen unterschiedlicher Genese.

Die erfindungsgemäßen Verbindungen können auf verschiedenen Wegen verabreicht werden, z.B. oral, parenteral, kutan, subkutan, intravenös, intramuskulär, rektal oder inhalativ. Bevorzugt ist die orale oder inhalative Verabreichung. Die Verbindung wird einem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 100.000 mg/kg Körpergewicht, bevorzugt 0,01 bis 10.000 mg/kg Körpergewicht und ganz bevorzugt 0,1 bis 1.000 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Pulver zur Rekonstitution vor Gebrauch, Aerosolen, Inhalationssprays, Wirkstoffpflastern, Granulaten, Suppositorien, Ovula, Injektionspräparaten, Cremes, Salben, Gels, Mikrospheren, Implantaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polytethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol, Ethylenglycol oder andere Additive, die in der Pharmazie üblich sind, verwendet werden.

Bei der Verwendung von Infusions- oder Injektionslösungen sind diese bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Weiter bevorzugt werden Inhalationszusammensetzungen, z.B. in Form von Aerosolen, Sprays, oder als mikronisiertes Pulver hergestellt. Dazu werden die erfindungsgemäßen Verbindungen entweder in pharmazeutisch üblichen Lösungsmitteln gelöst bzw. suspendiert und mittels Überdruck in einem bestimmten Volumen fein verteilt und inhaliert. Ein entsprechendes Vorgehen erfolgt bei den zu inhalierenden Festsubstanzen, die gleichfalls mittels Überdruck fein verteilt und inhaliert werden. Ebenfalls andere als mit Überdruck funktionierende Applikatoren sind hierbei eingeschlossen.

Die Erfindung betrifft auch pharmazeutische Zubereitungen, die eine therapeutisch wirksame Menge des aktiven Inhaltsstoffs (erfindungsgemäße Verbindung der Formel (1)) zusammen mit organischen oder anorganischen festen oder flüssigen pharmazeutisch verträglichen Trägern, die für die beabsichtigte Verabreichung geeignet sind, und die mit den aktiven Inhaltsstoffen nicht nachteilig Wechselwirken, enthalten.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt zur erfindungsgemäßen pharmazeutischen Zusammensetzung mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- β₂-Adrenoceptor Agonisten (z.B. Terbutalin, Salbutanol, Salmetanol, Fenoterol, Formoterol)
- Dinatriumcromoglycat
- Korticosteroide
- Leukotrien-Antagonisten (entweder Enzym-Inhibitoren [wie 5-Lipoxygenaseinhibitoren oder Arachidonsäure-Enzyminhibitoren] oder Rezeptorantagonisten) , z.B. Pramkulast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, , Olopatadin
- Theophyllin
- Muscarinrezeptor-Antagonisten, z.B. Spiriva
- (monoklonale) Antikörper gegen TNF-alpha oder andere Wirkstoffe, die die Bildung bzw. Freisetzung von TNF-alpha oder die Aktivität von TNF-alpha hemmen (z.B. rekombinante lösliche Rezeptorkonstrukte)

Die Kombination mit Korticosteroiden, Leukotrien-Antagonisten, Antihistaminika, Theophylin, Muscarinrezeptor-Antagonisten und/oder TNF-alpha-Hemmern dient besonders dazu, den akuten zu behandelnden Krankheitszustand nicht chronisch werden zu lassen, da die erfindungsgemäßen Verbindungen und die anderen Wirkstoffe komplementäre Aspekte der der Erkrankung zugrundeliegenden pathophysiologischen Mechanismen angehen. Erfindungsgemäß sollte insbesondere die Kombination der erfindungsgemäßen Verbindungen mit ß₂₋Adrenoceptoragonisten und/oder Dinatriumcromogycat als Inhalationstherapie bei milden Formen von Asthma, insbesondere im Kindesalter, wirksam sein. In der Kombination mit Glucocorticoiden ergibt sich ein positiver Effekt daraus, daß weniger Glucocorticoide angewendet werden müssen und damit ein Spareffekt zu erreichen ist und die von Glucocorticoiden bekannten Nebenwirkungen vermindert werden bzw. ganz ausbleiben. Die Kombination der erfindungsgemäßen Verbindungen mit Glucocorticoiden und/oder Theophyllin hat sich insbesondere bei persistierendem Asthma bewährt.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1 vorliegen.

Die Erfindung betrifft weiterhin Verfahren zur Herstellung der erfindungsgemäßen Verbindungen.

Die erfindungsgemäßen Verfahren zur Herstellung der Verbindungen der allgemeinen Formeln 1, 1 a und 1b mit den zuvor aufgeführten Bedeutungen von R¹, R², R³, R⁴, R⁵, R⁶ R⁷, R⁸ und Y sind gekennzeichnet durch folgende Verfahrensweisen:
**A)**
   - Umsetzung der bekannten bzw. nach bekannten Methoden analog dargestellten Pyridin-3-carbonitrile der allgemeinen Formel 2 (mit identischer Bedeutung von R¹, R², R³ und Y wie vorstehend) 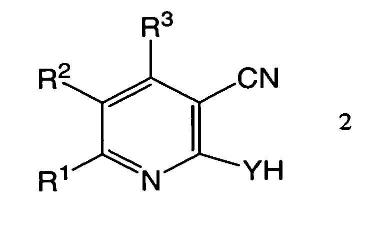 in an sich bekannter Weise mit Chloracetamid, ClCH₂CONH₂, in methanolischer oder ethanolischer Lösung in Gegenwart eines Natriumalkoxides, vorzugsweise Natriummethoxid oder Natriumethoxid, in die analogen 3-Amino-thieno[2,3-b]pyridin-2-carbonsäureamide der allgemeinen Formel 4 (mit identischer Bedeutung von R¹, R², R³ wie vorstehend und worin Y die Bedeutung von S aufweist), bzw. in die analogen 3-Amino-furo[2,3-b]pyridin-2-carbonsäureamide der allgemeinen Formel 4 (mit identischer Bedeutung von R¹, R² und R³ und wobei Y die Bedeutung von O aufweist);

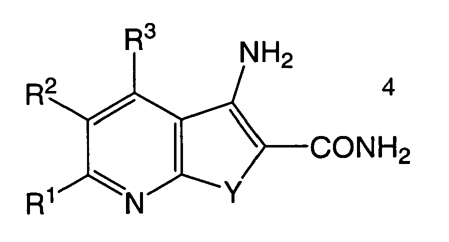

Die Verbindungen der allgemeinen Formel 4 sind aus den Verbindungen der allgemeinen Formel 2 (wobei R¹, R² und R³ und Y die oben genannten Bedeutungen aufweisen) auch dadurch darstellbar, dass diese Verbindungen mit Choracetamid zunächst in vorzugsweise ethanolischer Lösung in Gegenwart von vorzugsweise Triethylamin oder einem sekundären cycloaliphatischen Amin wie Morpholin, Piperidin oder Pyrrolidin zu den Verbindungen der allgemeinen Formel 3 (wobei R¹, R² und R³ und Y die oben genannten Bedeutungen aufweisen), 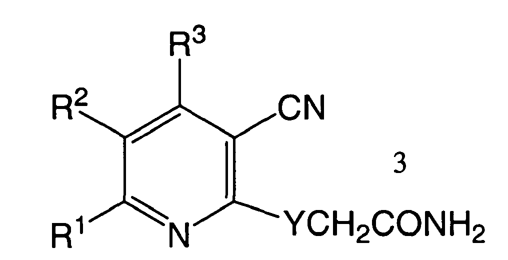 umgesetzt werden und diese Verbindungen in einem weiteren Syntheseschritt in vorzugsweise wasserfreier ethanolischer Lösung mit einer katalytischen Menge Natriummethoxid oder Natriumethoxid durch Erhitzen unter Rückfluss gleichfalls in die oben genannten Verbindungen der allgemeinen Formel 4 übergeführt werden.]
- Umsetzung der Verbindungen der allgemeinen Formel 4 mit Phosgen, Trichlormethylchlorformiat oder mit Alkylchlorformiaten der allgemeinen Formel 5,

   CICOOR^{§§} 5,

   worin R^{§§} C₁₋₃-Alkyl bedeutet,

in einem vorzugsweise aprotischen, hochsiedenden Lösungsmittel wie Dioxan, Tetrahydrofuran oder Toluol bzw. deren Mischungen unter Erhitzen, ggf. in Anwesenheit katalytischer Mengen eines Natriumalkoxides, wie zum Beispiel Natriumethylat, zu Verbindungen der allgemeinen Formel 6, 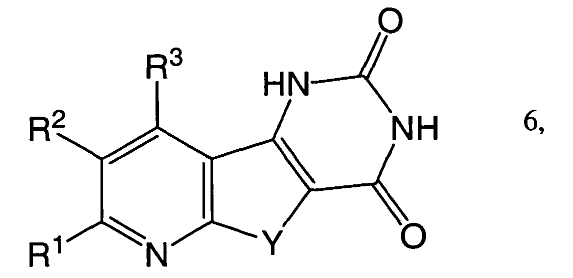
worin R¹, R², R³ und Y dasselbe wie oben bedeuten;
- Umsetzung der tricyclischen Pyrimidin-2,4-dione der allgemeinen Formel 6
   mit Phosphortrichlorid, Phosphorpentachlorid, Phosphoroxidchlorid oder vorzugsweise Dichlorphenylphosphinoxid bzw. deren Mischungen, jeweils in der Siedehitze,
   zu den tricyclischen 2,4-Dichlorpyrimidinen der allgemeinen Formel 7,

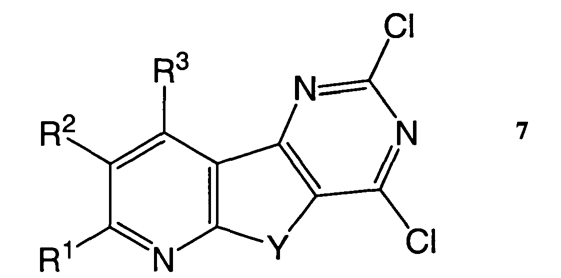

worin R¹, R², R³ und Y dasselbe wie oben bedeuten;
- Umsetzung der 2,4-Dichlorpyrimidine der allgemeinen Formel 7
   mit Morpholin, Thiomorpholin, Thiomorpholin-S,S-Dioxid, Pyrrolidin, Piperidin, Homopiperazin, Piperazin, N-Alkylpiperazinen, C₁-₆, N-Hydroxyalkylpiperazinen, C₁₋₃, N-Benzylpiperazin, oder N-Aryl-piperazinen,
   mit ein oder mehrfach, gleich oder ungleich am heterocycloaliphatischen Ring substituierten Aminen,
   mit weiteren sekundären, mono- oder polycyclischen cycloaliphatischen Aminen mit insgesamt 5-14 Ringatomen, einschließlich der am C-Skelett mit R^{§} ein oder mehrmals, gleich oder ungleich substituierten Vertreter,
   mit sekundären aliphatischen oder aromatischen Aminen, R⁷R⁸NH [worin R⁷ und R⁸ gleich oder ungleich sein können und C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl usw. (wobei alle vorstehend genannten Reste auch mit R^{§} ein oder mehrmals, gleich oder ungleich substituiert sein können) bedeuten];
   mit primären Aminen, R⁷NH₂ (worin R⁷ dasselbe wie oben bedeutet),
   mit Natriumazid,
   mit Ammoniak, Hydroxylamin, O-C₁₋₃Alkylhydroxylaminen, N-C₁₋₃Alkylhydroxyl-aminen, N,N-Di (C₁-₃)alkylhydroxylaminen, Hydrazin, C₁₋₄Alkyl- und Di-(C₁₋₄) alkylhydrazinen, Benzylhydrazinen, Carbonsäurehydraziden, N,N-Diacylhydrazinen,
   mit Harnstoff, N-Alkyl-, N,N-Dialkyl- und N,N'-Dialkylharnstoffen,
   mit Imidazol, 1,2,4-Triazol, Pyridin, Pyrazin, Pyridazin, einschliesslich der mit R^{§} substituierten Vertreter dieser Azaheterocyclen;
   mit Natrium- oder Kaliumalkoholaten von
   - C₁-₄Alkanolen,
   - mit -OH, -SH, -SCH₃, -F, -Cl, -Br, -F₂, -Cl₂, -F₃ oder -Cl₃
      substituierter C₁₋₄Alkanole,
   - C₂-₅Alkenolen oder C₂-₅Alkinolen,
   - C₄-₇Cycloalkanolen und C₄-₇Cycloalkenolen (ggf. am C- Skelett
      substituiert),

mit Natrium- oder Kaliumphenolaten von mono-, bi- oder tricyclischen C₆₋₁₄ Aromaten oder Heteroaromaten,

in Alkanolen oder gegebenenfalls aprotischen, dipolaren Lösungsmitteln unter Erwärmen, in Ausnahmefällen auch bei Raumtemperatur,
zu den tricyclischen 2-Chlor-pyrimidinen der allgemeinen Formeln 8a bzw. 8b, 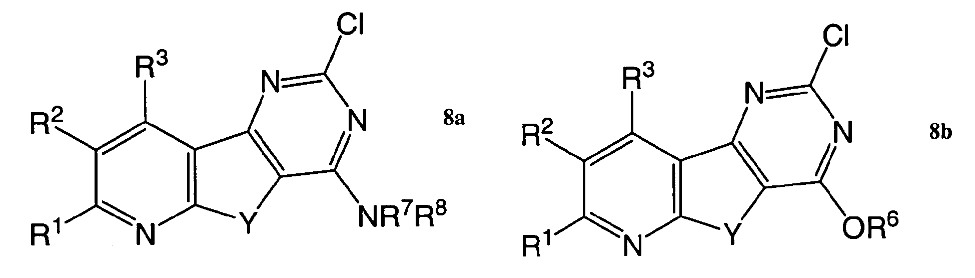
worin R¹, R², R³, R⁶, R⁷, R⁸ und Y dasselbe wie oben bedeuten;
- Umsetzung der 2-Chlor-4-NR⁷R⁸-pyrimidine der allgemeinen Formel 8a,
   worin R¹, R², R³, R⁷, R⁸ und Y dasselbe wie oben bedeuten,
   mit Natrium- oder Kaliumalkoholaten von
   - C₁-₄Alkanolen,
   - mit -OH, -SH, -SCH₃, -F, -Cl, -Br, -F₂, -Cl₂, -F₃ oder -Cl₃
      substituierter C₁-₄Alkanole,
   - C₂-₅Alkenolen oder C₂-₅Alkinolen,
   - C₄-₇Cycloalkanolen und C₄-₇Cycloalkenolen (ggf. am C- Skelett substituiert),
      mit Natrium- oder Kaliumphenolaten von mono-, bi- oder tricyclischen C₆₋₁₄ Aromaten oder Heteroaromaten,

in aprotischen, dipolaren Lösungsmitteln oder ggf. Alkanolen unter Erwärmen,
zu den Verbindungen der allgemeinen Formel 1 a, worin R¹, R², R³, R⁶, R⁷, R⁸ und Y dasselbe wie oben bedeuten;
- Umsetzung der 2-Chlor-4-OR⁶-pyrimidine der allgemeinen Formel 8b, worin R¹, R², R³, R⁶ und Y dasselbe wie oben bedeuten,
   mit Morpholin, Thiomorpholin, Thiomorpholin-S,S-Dioxid, Pyrrolidin, Piperidin, Homopiperazin, Piperazin, N-Alkylpiperazinen, C₁-₆, N-Hydroxyalkylpiperazinen, C₁-₃, N-Benzylpiperazin, oder N-Aryl-piperazinen,
   mit ein oder mehrfach, gleich oder ungleich am heterocycloaliphatischen Ring substituierten Aminen,
   mit weiteren sekundären, mono- oder polycyclischen cycloaliphatischen Aminen mit insgesamt 5-14 Ringatomen, einschließlich der am C-Skelett mit R^{§} ein oder mehrmals, gleich oder ungleich substituierten Vertreter,
   mit sekundären aliphatischen oder aromatischen Aminen, R⁷R⁸NH [worin R⁷ und R⁸ gleich oder ungleich sein können und C₁-₆Alkyl, Benzyl, Phenyl, 1- und 2-Naphthyl, 2-, 3- und 4-Pyridyl, Chinolinyl, Isochinolinyl, 2-Thienyl, 2-Furyl usw. (wobei alle vorstehend genannten Reste auch mit R^{§} ein oder mehrmals, gleich oder ungleich substituiert sein können) bedeuten];
   mit primären Aminen, R⁷NH₂ (worin R⁷ dasselbe wie oben bedeutet),
   mit Natriumazid,
   mit Ammoniak, Hydroxylamin, O-C₁-₃Alkylhydroxylaminen, N-C₁₋₃Alkylhydroxylaminen, N,N-Di(C₁₋₃)alkylhydroxylaminen, Hydrazin, C₁₋₄Alkyl- und Di-(C₁₋₄)alkylhydrazinen, Benzylhydrazinen, Carbonsäurehydraziden, N,N-Diacylhydrazinen,
   durch Erhitzen in aprotischen Lösungsmitteln unter Rückfluss
   zu den Verbindungen der allgemeinen Formel 1 b,
   worin R¹, R², R³, R⁶, R⁷, R⁸ und Y dasselbe wie oben bedeuten;
   oder

**B)**
   - Umsetzung der Verbindungen der allgemeinen Formel 4, worin R¹, R², R³ und Y dasselbe wie oben bedeuten,
      mit Carbonsäurehalogeniden der allgemeinen Formel 10,

      R^{&}-CO-Z 10

   worin bedeuten:
   - R^{&}: C₂₋₁₄Alkyl, C₃₋₁₄Cycloalkyl, C₂₋₁₄Alkenyl, C₃₋₁₄Cycloalkenyl, C₂₋₁₄Alkinyl,
   mono-, bi- oder tricyclische gesättigte oder ein- oder mehrfach ungesättigte heterocyclische Reste mit insgesamt 4-14 Ring-Atomen, davon 1-5 Heteroatomen, die vorzugsweise N, O, S und Se sind, ggf. ein- oder mehrfach gleich oder verschieden substituiert mit
   -OH, -SH, -O-C₁₋₈Alkyl, -OC₆₋₁₄Aryl, -S-C₁₋₄Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC₁₋₈Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl), -F, -CI, -Br, -I, -CN, -NO₂, -SCN
   - Z: Chlor oder Brom

oder
- Umsetzung der Verbindungen der allgemeinen Formel 4, worin R¹, R², R³ und Y dasselbe wie oben bedeuten,
   mit Carbonsäureanhydriden der allgemeinen Formel 11,

   (R^{&}CO)₂O 11

worin R^{&} dasselbe wie bei den Verbindungen der allgemeinen Formel 10 bedeutet,
jeweils im Überschuss und in der Siedehitze,
gegebenenfalls zusammen mit Pyridin oder einem aprotischen, hochsiedenden Lösungsmittel, wie zum Beispiel Toluol oder Xylol,
- Behandlung des gebildeten Niederschlages mit wässerig-ethanolischer Natronlauge oder wässriger Ammoniak-Lösung unter Erwärmen,
   nachfolgendem Ansäuern mit verdünnter Salzsäure unter Bildung der Verbindungen der allgemeinen Formel 12,

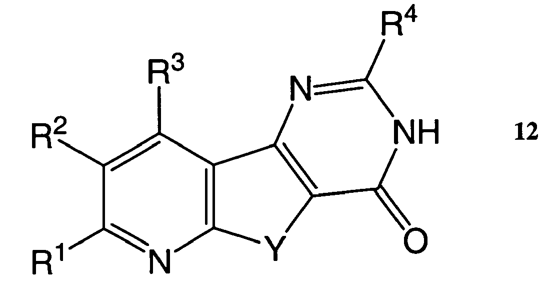

worin R¹, R², R³ und Y dasselbe wie oben bedeuten
und R⁴ die bei den Verbindungen der allgemeinen Formel 10 aufgeführte Bedeutung von R^{&} aufweist,
- Umsetzung der tricyclischen Pyrimidin-4-one der allgemeinen Formel 12
   mit Phosphortrichlorid, Phosphorpentachlorid, Phosphoroxidchlorid oder Dichlorphenylphosphinoxid, jeweils in der Siedehitze, zu den tricyclischen 4-Chlor-pyrimidinen der allgemeinen Formel 13,

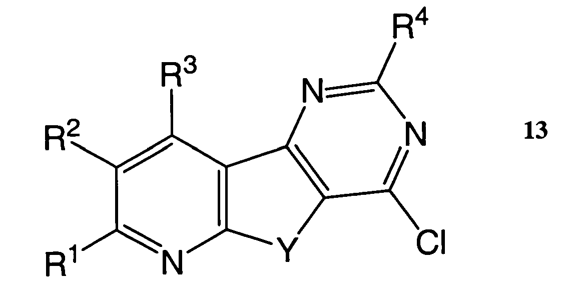

worin R¹, R², R³ und Y dasselbe wie oben bedeuten
und R⁴ die bei den Verbindungen der allgemeinen Formel 10 aufgeführte Bedeutung von R^{&} aufweist,
- Umsetzung der tricyclischen 4-Chlorpyrimidine der allgemeinen Formel 13
   mit jenen ionogen und nicht-ionogen N-, O-, und S-Nukleophilen, die auch für die Umsetzung der Verbindungen der allgemeinen Formel 7 zu den Verbindungen der allgemeinen Formeln 8a und 8b Verwendung finden,
   zu Verbindungen der allgemeinen Formel 1,

worin R¹, R², R³, R⁵ und Y dasselbe wie oben bedeuten
und R⁴ die bei den Verbindungen der allgemeinen Formel 10 aufgeführte Bedeutung von R& aufweist,
oder
**C)**
   - Umsetzung der aus den Verbindungen der allgemeinen Formel 2 und Chloressigsäurealkylestern, C₁-C₃, darstellbaren bekannten

   oder nach prinzipiell bekannten Methoden darstellbaren Verbindungen der allgemeinen Formel 14, 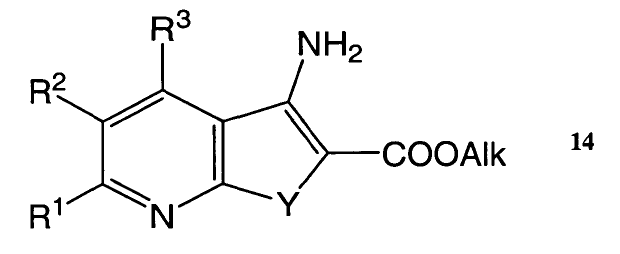
   worin R¹, R², R³, Y dasselbe wie oben und Alk Alkyl, C₁-C₃, bedeuten,
   mit Benzoylisothiocyanat, PhCONCS, in aprotischen, dipolaren Lösungsmitteln, vorzugsweise Aceton,
   unter Erwärmen zu den Verbindungen der allgemeinen Formel 15, 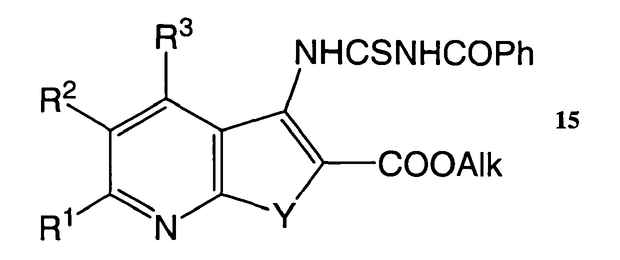
   worin R¹, R², R³, Y dasselbe wie oben und Alk Alkyl, C₁-C₃, bedeuten,
   - Umsetzung der Verbindungen der allgemeinen Formel 15 mit Natrium- oder Kaliumhydroxid in protischen oder aprotischen polaren Lösungsmitteln oder Lösungsmittelgemischen unter Erwärmen,

   Zugabe von Brom- oder lodalkanen, -alkenen oder alkinen, Alk*Z,
   (worin Alk* bedeutet: C₁-C₈Alkyl, C₂-C₈Alkenyl, C₃-C₆Alkinyl, ggf. mit einem Rest wie -CN, -SCN, NO₂, Phenyl, C₃₋₇Cycloalkyl, substituiert ),
   zu der erkalteten alkalischen Reaktionslösung,
   mäßiges Erwärmen bis zur Beendigung der Umsetzung,
   Ansäuern mit verdünnter Salzsäure unter Entstehung der Verbindungen der allgemeinen Formel 16, 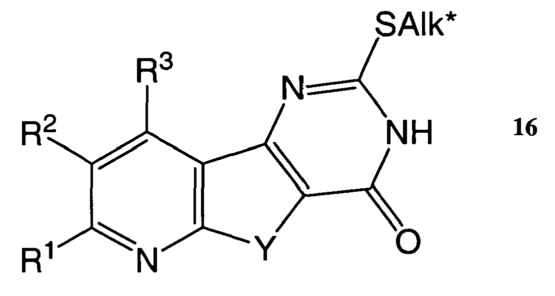
   worin R¹, R², R³, Y dasselbe wie oben und Alk* bedeutet: C₁-C₈Alkyl, C₂-C₈ Alkenyl, C₃-C₆Alkinyl, und ggf. mit einem Rest wie -CN, -SCN, NO₂, Phenyl und C₃₋C₇Cycloalkyl, , substituiert,
   - Umsetzung der tricyclischen 2-Alkylthio-pyrimidin-4-one der allgemeinen Formel 16
      mit Phosphortrichlorid, Phosphorpentachlorid, Phosphoroxidchlorid oder Dichlorphenylphosphinoxid, jeweils in der Siedehitze,
      zu den tricyclischen 2-Alkylthio-4-chlor-pyrimidinen der allgemeinen Formel 17,

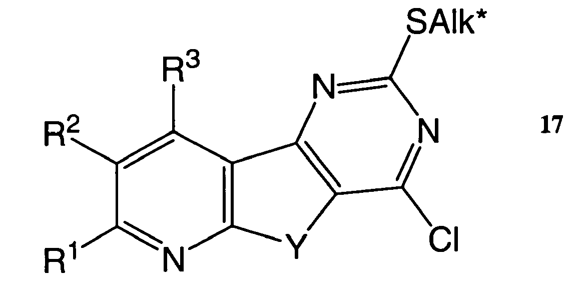

   worin R¹, R², R³, Y und Alk* dasselbe wie bei den Verbindungen der allgemeinen Formel 16 bedeuten,
   - Umsetzung der tricyclischen 2-Alkylthio-4-chlor-pyrimidine der allgemeinen Formel 17, mit jenen ionogen und nicht-ionogen N-, O-, und S-Nukleophilen, die bei der Umsetzung der Verbindungen der allgemeinen Formel 7 zu den Verbindungen der allgemeinen Formeln 8a und 8b Verwendung finden, zu Verbindungen der allgemeinen Formel 1, worin R¹, R², R³, R⁵ und Y dasselbe wie oben bedeuten und R⁴ SAlk* bedeutet (worin Alk* bedeutet: C₁-C₈Alkyl, C₂-C₈Alkenyl, C₃-C₆Alkinyl, ggf. mit einem Rest wie -CN, -SCN, NO₂, Phenyl oder C₃-C₇Cycloalkyl substituiert ),
**D)**
   - Umsetzung von Verbindungen der allgemeinen Formel 1 bei denen R⁴ SAlk* bedeutet (worin Alk* bedeutet: C₁-C₈Alkyl, C₂-C₈Alkenyl, C₃-C₆Alkinyl, ggf. mit einem Rest wie -CN, -SCN, NO₂, Phenyl oder C₃-C₇Cycloalkyl substituiert)
      mit annähernd äquimolarer Menge Dihydrogenperoxid in Essigsäure oder Ameisensäure unter dc-Kontrolle bei Raumtemperatur
      oder mit KlO₄ in protischen oder aprotischen, dipolaren Lösungsmitteln unter Erwärmen zu den Verbindungen der allgemeinen Formel 1, worin R¹, R², R³, R⁵ und Y dasselbe wie oben bedeuten und R⁴ SOAlk* bedeutet (worin Alk* bedeutet: C₁₋C₈A1kyl, C₂-C₈Alkenyl, C₃-C₆Alkinyl, ggf. mit einem Rest wie -CN, -SCN, NO₂, Phenyl oder C₃-C₇Cycloalkyl substituiert)
**E)**
   - Umsetzung jener Verbindungen der allgemeinen Formel 1 bei denen R⁴ SAlk* oder SOAlk* bedeutet (wobei Alk*: C₁-C₈Alkyl, C₂-C₈Alkenyl, C₃-C₆Alkinyl, ggf. mit einem Rest wie -CN, -SCN, NO₂, Phenyl oder C₃-C₇Cycloalkyl substituiert)
      mit überschüssigem Dihydrogenperoxid in Essigsäure oder Ameisensäure unter dc-Kontrolle bei Raumtemperatur, gegebenenfalls auch unter mäßigem Erwärmen
      oder mit KMnO₄ in protischen oder aprotischen, dipolaren Lösungsmitteln unter Erwärmen
      zu den Verbindungen der allgemeinen Formel 1, worin R¹, R², R³, R⁵ und Y dasselbe wie oben bedeuten
      und R⁴ SO₂Alk* bedeutet (worin Alk*: C₁-C₈Alkyl, C₂-C₈Alkenyl, C₃-C₆Alkinyl, ggf. mit einem Rest wie -CN, -SCN, NO₂, Phenyl oder C₃-C₇Cycloalkyl substituiert).
      t
      Entsprechend der vorliegenden Erfindung können die unter A) bis E) beschriebenen Verfahren innerhalb weiter Grenzen variiert werden.

So können die nach dem Verfahren A) aus definierten Verbindungen der allgemeinen Formel 8a oder 8b mit Natrium- oder Kaliumalkoxiden erhältlichen Verbindungen der allgemeinen Formel 1 a oder 1b, gegebenenfalls unter Verwendung von Kronenethern dargestellt werden.

Weitere Ausführungsformen der Erfindung können beispielsweise darin bestehen, dass die Darstellung der tricyclischen Pyrimidindione der allgemeinen Formel 6 bzw. die der tricyclischen 2,4-Dichlorpyrimidine der allgemeinen Formel 7 unter Verwendung einer Mikrowelle realisiert wird,

### Untersuchungen zur PDE4A-Hemmung und Hemmung der TNFα-Freisetzung

### Methodenbeschreibung

Die Verbindungen der allgemeinen Formel 1 sind starke Inhibitoren der Phosphodiesterase 4 und der Freisetzung von TNFalpha.

Die Volllängen-Formen der Phosphodiesterasen wurden aus zytosolischen Präparationen der humanen monozytischen Zellinien U937 oder HL60 erhalten und durch Anionen-Austauschchromatographie an einer Poros HQ Säule (starker Anionen-Austauscher) entsprechend einer adaptierten Methode von Lavan B. E., Lakey T., Houslay M. D. Biochemical Pharmacology, (1989), 38(22), 4123-4136., und Silver P. J et al., Eur. J. Pharmacol. (1988) 150: 85-94, and T. J. Torphy et al., J. Pharm. Exp. Ther. (1992), 263: 1195-1205 teilweise gereinigt.

Humane rekombinante PDE4A katalytische Domäne, die die Aminosäuren 342 - 704 umfasst, wurde in Escherichia coli kloniert und exprimiert, wie beschrieben durch W. Richter et al., Protein Expression and Purification (2000) 19: 375-383. Die anderen Phosphodiesterasen wurden entweder aus Zellinien humanen Ursprungs gewonnen (TPH1 Monozyten Linie für PDE1, MCF7 für PDE5), oder in SF9 Insektenzellen rekombinant exprimiert (für PDE3A, PDE3B, PDE7A und PDE4D), entsprechend einer adaptierten Methode von Luckow, V. A. et al., in: Recombinant DNA Technology&Applications., (1991) eds. Prokop, Bajpai, R. K.&Ho, C. S., pp 97-152.

Die Phosphodiesterase Aktivität wurde wie beschrieben durch den Hersteller Amersham Biosciences und durch Horton J.K and Baxendale P. M., Methods Mol. Biol. (1995) 41: 91-105 mittels des SPA-Assays bestimmt.

Die Reaktionsmischungen enthielten 100 mM Tris-HCI (pH 7.5), 5 mM MgCl₂, 3.8 mM 2-mercaptoethanol, und 1µM cAMP oder cGMP als Substrat, die Inhibitoren in unterschiedlichen Konzentrationen und ferner weitere Komponenten, die für die Detektion der individuellen Isoenzyme benötigt wurden (s. unten). Die Reaktion wurde durch Zugabe des Enzyms gestartet. Das Arbeitsvolumen beträgt 100 µl in den wells einer weißen 96-well Mikrotiterplatte. Die Testsubstanzen wurden als Stock-Lösungen in DMSO hergestellt. Die DMSO-Konzentration im Reaktionsansatz war 1 % v/v. Bei dieser DMSO-Konzentration wird die PDE-Aktivität nicht beeinträchtigt. Nach Start der Reaktion durch Zugabe von Enzym, wurden die Proben bei 22° C für 20 Minuten inkubiert. Die Reaktion wurde gestoppt durch Zugabe von SPA-Bead Suspension, die nach Hersteller vorgegebene Zinksulfatkonzentration enthält. Danach lässt man die Beads für 30 Minuten sedimentieren und wertet die Platten am Lumineszenz Mikrotiterplattenreader Fluostar Optima (BMG Labtechologies) aus.

[³H]-cAMP wurde als Substrat für die Bestimmung der Aktivität von PDE 2, 3, 4 und 7 eingesetzt und [³H]-cGMP für die Bestimmung der Aktivität von PDE 5. Die unspezifischen Enzymaktivitäten wurden in Anwesenheit von 100 µM rolipram (für PDE 4) und in Anwesenheit von 100 µM IBMX (für PDE 3 und 5) bestimmt und von den Testwerten subtrahiert. Die Inkubationsproben des PDE 3 Assays enthalten 10 µM rolipram, um mögliche Kontaminationen durch PDE 4 zu inhibieren. PDE 2-Aktivität wird unter Verwendung eines SPA-Assays von Amersham Biosciences in Gegenwart des PDE 2 Aktivators 5 µM cGMP getestet.

IC₅₀-Werte im Bereich von 10⁻⁹ bis 10⁻⁵ M wurden für die in der Erfindung beschriebenen Inhibitoren bezüglich ihrer inhibitorischen Wirkung auf Phosphodiesterase 4 beobachtet. Die Selektivität zu den PDE's 2, 3 und 5 weist einen Faktor von 100 bis 10 000 auf.

Die Stimulierung isolierter Leukozyten für die Freisetzung von Zytokinen kann auf verschiedenen Wegen erfolgen. Lipopolysaccharide (LPS) stellen einen Stimulus für die Untersuchung der Freisetzung von TNFα dar. LPS ist Bestandteil bakterieller Zellwände und wird beim Abtöten der Bakterien (durch Antibiotika oder das natürliche Immunsystem) freigesetzt. LPS stimuliert insbesondere die Aktivität phagozytierender Leukozyten (Gewebsmakrophagen, Granulozyten, Monozyten) und verursacht die Infiltration von Leukozyten vom peripheren Blut in das betroffene Gewebe. Ein Zytokin von besonderer Bedeutung für diese Mechanismen ist TNFα, das in großen Mengen durch die betroffenen Zellen sezerniert wird. Hauptquelle dabei sind Monozyten und Makrophagen. TNFα initiiert und prolongiert den Entzündungsprozess im Zusammenspiel mit anderen Mediatoren.

Für die Untersuchung des Effektes auf die LPS-induzierte TNFα-Freisetzung wurde eine Methode verwendet, die von Marx, D., Tassabehji, M., Heer, S., Hüttenbrink, K.-B., and 1. Szelenyi, Pulmonary Pharmacology & Therapeutics (2002) 15, 7-15 beschrieben wurde. Die Methode in Kürze: Humanes Blut wurde von verschiedenen Spendern entnommen, durch Zusatz von 10 mM Na-Citrat ungerinnbar gemacht und 1:5 mit RPMI 1640 Zellkulturmedium verdünnt. Die Testsubstanzen wurden den Blutproben in verschiedenen Konzentrationen zugefügt. 15 Minuten später wurden die Leukozyten durch Zusatz von Lipopolysacchariden (LPS) aus Salmonella abortus equi in einer Endkonzentration von l,ug/ml stimuliert. Nach Inkubation der Testansätze für 24 Stunden bei 37°C. und unter 5% CO₂ in wassergesättigter Luft, wurde das Blut zentrifugiert und die Konzentration an TNFα im zellfreien Überstand unter Verwendung eines käuflichen ELISA (BD Biosciences) nach Angaben des Herstellers exakt vermessen.

Die Verbindungen der allgemeinen Formel 1 sind starke Inhibitoren der Phosphodiesterase 4 und der Freisetzung von TNFalpha. Ihr therapeutisches Potential in vivo ist erwiesen durch die Inhibierung der asthmatischen Spät-Phasen Reaktion (Eosinophilie) in der Lungen-Spülflüssigkeit des Meerschweinchens und durch die Beeinflussung der Allergen-induzierten Gefäßpermeabilität in aktiv sensitierten braunen Ratten (R. Norwegicus).

Die nachfolgende Auflistung beinhaltet erfindungsgemäße Substanzen der allgemeinen Formel 1, die im PDE-4A-Hemmassay einen IC₅₀-Wert zwischen >50 und <500 nM aufweisen:
2-Ethoxy-7,9-diphenyl-4-(piperazin-1-yl)-pyrido[3',2':4,5]thieno[3,2-d]pyrimidin;
2-Methoxy-7-methyl-9-phenyl-4-(piperazin-1-yl)-pyrido[3',2':4,5]thieno[3,2-d]pyrimidin;
4-Ethoxy-2-(homopiperazin-1-yl)-9-methyl-7-phenyl-pyrido[3',2':4,5]thieno[3,2-d]pyrimidin;
4-Cyclopropylmethoxy-9-methyl-7-phenyl-2-(piperazin-1-yl)-pyrido[3',2':4,5]thieno[3,2-d]pyrimidin;
2-(2,2-Difluor-ethoxy)-9-methyl-7-phenyl-4-(piperazin-1-yl)-pyrido[3',2':4,5]thieno[3,2-d]pyrimidin;
2-Methoxy-7,9-dimethyl-4-(piperazin-1-yl)-pyrido[3',2':4,5]thieno[3,2-d]pyrimidin;
2-Ethoxy-4-(homopiperazin-1-yl)-9-methyl-7-phenyl-pyrido[3',2':4,5]thieno[3,2-d]pyrimidin;
4-Ethoxy-7-methyl-9-phenyl-2-(piperazin-1-yl)-pyrido[3',2':4,5]thieno[3,2-d]pyrimidin;
2-Ethyl-9-methyl-7-phenyl- 4-(piperazin-1-yl)-pyrido[3',2':4,5]thieno[3,2-d]pyrimidin;
2-Methoxy-9-methyl-7-phenyl-4-(piperazin-1-yl)-pyrido[3',2':4,5]thieno[3,2-d]pyrimidin;
9-Chlormethyl-2-ethylthio-7-phenyl- 4-(piperazin-1-yl)-pyrido[3',2':4,5]thieno[3,2-d]pyrimidin;
2-Ethylthio-9-methyl-7-phenyl-4-(piperazin-1-yl)-pyrido[3',2':4,5]thieno[3,2-d]pyrimidin;

Die nachfolgende Auflistung beinhaltet erfindungsgemäße Substanzen der allgemeinen Formel 1, die im PDE-4A-Hemmassay einen IC₅₀-Wert von <50 nM aufweisen:
4-Ethoxy-7-morpholino-9-phenyl-2-(piperazin-1-yl)-pyrido[3',2':4,5]thieno[3,2-d]pyrimidin;
4-Ethoxy-7-(4-trifluo-rmethyl-phenyl)-9-methyl-2-(piperazin-1-yl)-pyrido[3',2':4,5]thieno[3,2-d]pyrimidin;
8-(4-Amino-benzyl)-4-ethoxy-7,9-dimethyl-2-(piperazin-1-yl)-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;
9-Methyl-7-phenyl-2-(piperazin-1-yl)-4-n-propoxy-pyrido[3',2':4,5]thieno[3,2-d]pyrimidin;
4-Ethoxy-7-thien-2-yl-9-trifluor-methyl-2-(piperazin-1-yl)-pyrido[3',2':4,5]thieno[3,2-d]pyrimidin;
2-Ethoxy-7-(4-trifluor-methyl-phenyl)-9-methyl-4-(piperazin-1-yl)-pyrido[3',2':4,5]thieno[3,2-d]pyrimidin;
2-(2-Hydroxy-ethoxy)-9-methyl-7-phenyl-4-(piperazin-1-yl)-pyrido[3',2':4,5]thieno[3,2-d]pyrimidin;
2-Ethoxy-7-methyl-9-phenyl-4-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin-hydrochlorid;
4-Ethoxy-7-(4-methoxy-phenyl)-9-methyl-2-(piperazin-1-yl)-pyrido[3',2':4,5]thieno[3,2-d]pyrimidin;
4-Ethoxy-7-phenyl-9-methyl-2-(piperazin-1-yl)-pyrido[3',2':4,5]furo[3,2-d]pyrimidin;
2-(2,2-Difluor-ethoxy)-9-methyl-7-phenyl-4-(piperazin-1-yl)-pyrido[3',2':4,5]thieno[3,2-d]pyrimidin;
2-Ethoxy-7,9-di-iso-propyl-4-(piperazin-1-yl)-pyrido[3',2':4,5]thieno[3,2-d]pyrimidin;
7-(3,4-Dimethoxy-phenyl)-9-methyl-4-(piperazin-1-yl)-2-n-propyl-pyrido[3',2':4,5]thieno[3,2-d]pyrimidin;
2-Ethoxy-9-methyl-7-(4-methyl-phenyl)-4-(piperazin-1-yl)-pyrido[3',2':4,5]thieno[3,2-d]pyrimidin;
9-Methyl-7-phenyl-2-(piperazin-1-yl)-4-*iso*-propoxy-pyrido[3',2':4,5]thieno[3,2-d]pyrimidin;
4-(2-Hydroxy-ethoxy)-9-methyl-7-phenyl-2-(piperazin-1-yl)-pyrido[3',2':4,5]thieno[3,2-d]pyrimidin;
2-Ethoxy-7-thien-2-yl-9-trifluor-methyl-4-(piperazin-1-yl)-pyrido[3',2':4,5]thieno[3,2-d]pyrimidin;
2-Ethoxy-7-(3,4-dimethoxy-phenyl)-9-methyl-4-(piperazin-1-yl)-pyrido[3',2':4,5]thieno[3,2-d]pyrimidin;
2-Ethoxy-7-(4-methoxy-phenyl)-9-methyl-4-(piperazin-1-yl)-pyrido[3',2':4,5]thieno[3,2-d]pyrimidin;
2-Ethoxy-9-methyl-7-thien-2-yl-9-methyl-4-(piperazin-1-yl)-pyrido[3',2':4,5]thieno[3,2-d]pyrimidin;
4-Ethoxy-9-methyl-7-(4-methyl-phenyl)-2-(piperazin-1-yl)-pyrido[3',2':4,5]thieno[3,2-d]pyrimidin;
4-Ethoxy-7,9-dimethyl-8-(4-nitro-benzyl)-2-(piperazin-1-yl)-pyrido[3',2':4,5]thieno[3,2-d]pyrimidin;
4-Ethoxy-7-(4-fluor-phenyl)-9-methyl-2-(piperazin-1-yl)-pyrido[3',2':4,5]thieno[3,2-d]pyrimidin;
2-Ethoxy-7,9-dimethyl-8-(4-nitro-benzyl)-4-(piperazin-1-yl)-pyrido[3',2':4,5]thieno[3,2-d]pyrimidin;
4-Ethoxy-7,9-diphenyl-2-(piperazin-1-yl)-pyrido[3',2':4,5]thieno[3,2-d]pyrimidin;
4-Ethoxy-7-methyl-9-phenyl-2-(piperazin-1-yl)-pyrido[3',2':4,5]thieno[3,2-d]pyrimidin;
2-Ethoxy-9-trifluor-methyl-7-phenyl-4-(piperazin-1-yl)-pyrido[3',2':4,5]thieno[3,2-d]pyrimidin;
4-Ethoxy-7,9-di-*iso*-propyl-2-(piperazin-1-yl)-pyrido[3',2':4,5]thieno[3,2-d]pyrimidin;
9-Methyl-7-phenyl-2-(piperazin-1-yl)-4-*iso*-propoxy-pyrido[3',2':4,5]thieno[3,2-d]pyrimidin-hydrochlorid;
9-Methyl-7-phenyl-4-(piperazin-1-yl)-2-*iso*-propoxy-pyrido[3',2':4,5]thieno[3,2-d]pyrimidin-hydrochlorid;
9-Methyl-7-phenyl-4-(piperazin-1-yl)-2-*n*-propyl-pyrido[3',2':4,5]thieno[3,2-d]pyrimidin;
4-Ethoxy-9-methyl-8-(4-nitro-benzyl)-7-phenyl-2-(piperazin-1-yl)-pyrido[3',2':4,5]thieno[3,2-d]pyrimidin;
4-Ethoxy-9-methyl-7-thien-2-yl-2-(piperazin-1-yl)-pyrido[3',2':4,5]thieno[3,2-d]pyrimidin;
4-Ethoxy-9-trifluor-methyl-7-phenyl-2-(piperazin-1-yl)-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin;
4-Ethoxy-7,9-diphenyl-2-(piperazin-1-yl)-pyrido[3',2':4,5]thieno[3,2-d]pyrimidin-hydrochlorid;
2-Ethoxy-7-methyl-9-phenyl-4-(piperazin-1-yl)-pyrido[3',2':4,5]thieno[3,2-d]pyrimidin-hydrochlorid;
4-Ethoxy-7-(4-methoxy-phenyl)-9-methyl-2-(piperazin-1-yl)-pyrido[3',2':4,5]thieno[3,2-d]pyrimidin-hydrochlorid;

Die nachfolgende Auflistung beinhaltet erfindungsgemäße Substanzen der allgemeinen Formel 1, die im TNFα-Hemmassay einen IC₅₀-Wert zwischen 2 µM bis <10 µM aufweisen:
2-Ethoxy-7-methyl-9-phenyl-4-(piperazin-1-yl)-pyrido[3',2':4,5]thieno[3,2-d]pyrimidin;
2-Ethyl-9-methyl-7-phenyl- 4-(piperazin-1-yl)-pyrido[3',2':4,5]thieno[3,2-d]pyrimidin;
2-Methoxy-9-methyl-7-phenyl-4-(piperazin-1-yl)-pyrido[3',2':4,5]thieno[3,2-d]pyrimidin;
9-Chlormethyl-2-ethylthio-7-phenyl- 4-(piperazin-1-yl)-pyrido[3',2':4,5]thieno[3,2-d]pyrimidin;
2-Ethylthio-9-methyl-7-phenyl-4-(piperazin-1-yl)-pyrido[3',2':4,5]thieno[3,2-d]pyrimidin;
4-Ethoxy-2-(homopiperazin-1-yl)-9-methyl-7-phenyl-pyrido[3',2':4,5]thieno[3,2-d]pyrimidin;
4-Cyclopropylmethoxy-9-methyl-7-phenyl-2-(piperazin-1-yl)-pyrido[3',2':4,5]thieno[3,2-d]pyrimidin;
2-(2,2-Difluor-ethoxy)-9-methyl-7-phenyl-4-(piperazin-1-yl)-pyrido[3',2':4,5]thieno[3,2-d]pyrimidin;
2-Methoxy-7,9-dimethyl-4-(piperazin-1-yl)-pyrido[3',2':4,5]thieno[3,2-d]pyrimidin;
2-Methoxy-7-methyl-9-phenyl-4-(piperazin-1-yl)-pyrido[3',2':4,5]thieno[3,2-d]pyrimidin;
2-Ethoxy-4-(homopiperazin-1-yl)-9-methyl-7-phenyl-pyrido[3',2':4,5]thieno[3,2-d]pyrimidin;
4-Ethoxy-7-methyl-9-phenyl-2-(piperazin-1-yl)-pyrido[3',2':4,5]thieno[3,2-d]pyrimidin;
2-Ethoxy-7,9-diphenyl-4-(piperazin-1-yl)-pyrido[3',2':4,5]thieno[3,2-d]pyrimidin;
4-Ethoxy-7-(4-trifluo-rmethyl-phenyl)-9-methyl-2-(piperazin-1-yl)-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;
4-Ethoxy-7-phenyl-9-methyl-2-(piperazin-1-yl)-pyrido[3',2':4,5]furo[3,2-d]pyrimidin;
2-(2,2-Difluor-ethoxy)-9-methyl-7-phenyl-4-(piperazin-1-yl)-pyrido[3',2':4,5]thieno[3,2-d]pyrimidin;
9-Methyl-7-phenyl-2-(piperazin-1-yl)-4-*iso*-propoxy-pyrido[3',2':4,5]thieno[3,2-d]pyrimidin;
4-(2-Hydroxy-ethoxy)-9-methyl-7-phenyl-2-(piperazin-1-yl)-pyrido[3',2':4,5]thieno[3,2-d]pyrimidin;
7-(3,4-Dimethoxy-phenyl)-9-methyl-4-(piperazin-1-yl)-2-n-propyl-pyrido[3',2':4,5]thieno[3,2-d]pyrimidin;
9-Methyl-7-phenyl-2-(piperazin-1-yl)-4-iso-propoxy-pyrido[3',2':4,5]thieno[3,2-d]pyrimidin-hydrochlorid;
9-Methyl-7-phenyl-4-(piperazin-1-yl)-2-iso-propoxy-pyrido[3',2':4,5]thieno[3,2-d]pyrimidin-hydrochlorid;
4-Ethoxy-7,9-diphenyl-2-(piperazin-1-yl)-pyrido[3',2':4,5]thieno[3,2-d]pyrimidin-hydrochlorid;
9-Methyl-7-phenyl-4-(piperazin-1-yl)-2-n-propyl-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;

Die nachfolgende Auflistung beinhaltet erfindungsgemäße Substanzen der allgemeinen Formel 1, die im TNFα-Hemmassay einen IC₅ₒ-Wert zwischen 20 nM und <2 µM aufweisen:
4-Ethoxy-7-(3,4-dimethoxy-phenyl)-9-methyl-2-(piperazin-1-yl)-pyrido[3',2':4,5]thieno[3,2-d]pyrimidin;
4-Ethoxy-7-(4-methoxy-phenyl)-9-methyl-2-(piperazin-1-yl)-pyrido[3',2':4,5]thieno[3,2-d]pyrimidin-hydrochlorid;
2-Ethoxy-7-methyl-9-phenyl-4-(piperazin-1-yl)-pyrido[3',2':4,5]thieno[3,2-d]pyrimidin-hydrochlorid;
2-Ethoxy-7-(4-trifluor-methyl-phenyl)-9-methyl-4-(piperazin-1-yl)-pyrido[3',2':4,5]thieno[3,2-d]pyrimidin;
2-(2-Hydroxy-ethoxy)-9-methyl-7-phenyl-4-(piperazin-1-yl)-pyrido[3',2':4,5]thieno[3,2-d]pyrimidin;
8-(4-Amino-benzyl)-4-ethoxy-7,9-dimethyl-2-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin-hydrochlorid;
4-Ethoxy-7-morpholino-9-phenyl-2-(piperazin-1-yl)-pyrido[3',2':4,5]thieno[3,2-d]pyrimidin;
2-Ethoxy-7-(3,4-dimethoxy-phenyl)-9-methyl-4-(piperazin-1-yl)-pyrido[3',2':4,5]thieno[3,2-d]pyrimidin;
2-Ethoxy-7-(4-methoxy-phenyl)-9-methyl-4-(piperazin-1-yl)-pyrido[3',2':4,5]thieno[3,2-d]pyrimidin;
2-Ethoxy-7,9-dimethyl-8-(4-nitro-benzyl)-4-(piperazin-1-yl)-pyrido[3',2':4,5]thieno[3,2-d]pyrimidin;
4-Ethoxy-7,9-diphenyl-2-(piperazin-1-yl)-pyrido[3',2':4,5]thieno[3,2-d]pyrimidin;
4-Ethoxy-7-(4-methoxy-phenyl)-9-methyl-2-(piperazin-1-yl)-pyrido[3',2':4,5]thieno[3,2-d]pyrimidin;
9-Methyl-7-phenyl-2-(piperazin-1-yl)-4-*n*-propoxy-pyrido[3',2':4,5]thieno[3,2-d]pyrimidin;
4-Ethoxy-7,9-dimethyl-8-(4-nitro-benzyl)-2-(piperazin-1-yl)-pyrido[3',2':4,5]thieno[3,2-d]pyrimidin;
4-Ethoxy-7-(4-fluor-phenyl)-9-methyl-2-(piperazin-1-yl)-pyrido[3',2':4,5]thieno[3,2-d]pyrimidin;

Die Erfindung wird durch die nachfolgenden Beispiele näher erläutert.

### Beispiel 1

### 4-Ethoxy-7-(3,4-dimethoxyphenyl)-9-methyl-2-piperazin-1-yl-pyrido[3',2':4,5]thieno[3,2-d]pyrimidin (Verbindung 1; allgemeine Formel 1: R₁=3,4-(MeO)₂-Ph, R₂=H, R₃=Me, R₄=Piperazin-1-yl, R₅=OEt, Y=S)

1.0 g (2.4 mmol) 2-Chlor-4-ethoxy-7-(3,4-dimethoxyphenyl)-9-methyl-pyrido[3',2':4,5]thieno[3,2-*d*]pyrimidin (Beispiel 8g; allgemeine Formel 8: R₁=3,4-(MeO)₂-Ph, R₂=H, R₃=Me, R₅=OEt, Y=S) und 0.83 g (9.6 mmol) Piperazin werden in 20 ml Toluol 4 Stunden unter Rückfluß erhitzt. Anschließend wird das Lösungsmittel entfernt und der Rückstand in Dichlormethan suspendiert. Die organische Phase wird mit gesättigter Natriumcarbonat-Lösung gewaschen, über Natriumsulfat getrocknet und das Lösungsmittel entfernt. Der Rückstand wird durch Flash-Chromatographie (Kieselgel, Ethanol/Chloroform 1:5) gereinigt.
Ausbeute: 0.98 g (87 %); HPLC t_{R}: 16.9 min, Reinheit: 97.0 % (s. a in Tab. 1);
MS (ESI, m/e): 466 [M+H]⁺

Ausgehend von den nachfolgend beschriebenen Vorstufen werden durch Umsetzung der entsprechenden 4-Alkoxy-substituierten Verbindungen der allgemeinen Formel 8 mit Piperazin bzw. Homopiperazin analog Beispiel 1 folgende Verbindungen (2-11) erhalten, wobei die Reinigung z.T. durch Flash-Chromatographie (Kieselgel) erfolgt:

### Beispiel 2

### 4-Ethoxy-7-(4-methoxyphenyl)-9-methyl-2-piperazin-1-yl-pyrido[3',2':4,5]thieno[3,2-d]pyrimidin (Verbindung 2; allgemeine Formel 1: R₁=4-MeO-Ph, R₂=H, R₃=Me, R₄=Piperazin-1-yl, R₅=OEt, Y=S)

Analog Beispiel 1 aus 2-Chlor-4-ethoxy-7-(4-methoxyphenyl)-9-methyl-1-yl-pyrido[3',2':4,5]thieno[3,2-*d*]pyrimidin (Beispiel 8h; allgemeine Formel 8: R₁=4-MeO-Ph, R₂=H, R₃=Me, R₅=OEt, Y=S) und Piperazin (s. Tab. 1)

### Beispiel 3

### 4-Ethoxy-7-(4-fluorphenyl)-9-methyl-2-piperazin-1-yl-pyrido[3',2':4,5]thieno[3,2-d]pyrimidin (Verbindung 3; allgemeine Formel 1: R₁=4-F-Ph, R₂=H, R₃=Me, R₄=Piperazin-1-yl, R₅=OEt, Y=S)

Analog Beispiel 1 aus 2-Chlor-4-ethoxy-7-(4-fluorphenyl)-9-methyl-pyrido[3',2':4,5]-thieno[3,2-*d*]pyrimidin (Beispiel 8i; allgemeine Formel 8: R₁=4-F-Ph, R₂=H, R₃=Me, R₅=OEt, Y=S) und Piperazin (s. Tab. 1)

### Beispiel 4

### 4-Isopropoxy-9-methyl-7-phenyl-2-piperazin-1-yl-pyrido[3',2':4,5]thieno[3,2-d]pyrimidin

### (Verbindung 4; allgemeine Formel 1: R₁=Ph, R₂=H, R₃=Me, R₄=Piperazin-1-yl, R₅=OiPr, Y=S)

Analog Beispiel 1 aus 2-Chlor-4-isopropoxy-9-methyl-7-phenyl-pyrido[3',2':4,5]thieno[3,2-*d*]pyrimidin (Beispiel 8o; allgemeine Formel 8: R₁=Ph, R₂=H, R₃=Me, R₅=O*i*Pr, Y=S) und Piperazin (s. Tab. 1)

### Beispiel 5

### 4-Cyclopropylmethoxy-9-methyl-7-phenyl-2-piperazin-1-yl-pyrido[3',2':4,5]thieno[3,2-d]pyrimidin (Verbindung 5; allgemeine Formel 1: R₁=Ph, R₂=H, R₃=Me, R₄=Piperazin-1-yl, R₅=OCH₂Cypr, Y=S)

Analog Beispiel 1 aus 2-Chlor-4-cyclopropylmethoxy-9-methyl-7-phenyl-pyrido[3',2':4,5]thieno[3,2-*d*]pyrimidin (Beispiel 8p; allgemeine Formel 8: R₁=Ph, R₂=H, R₃=Me, R₅=OCH₂Cypr, Y=S) und Piperazin (s. Tab. 1)

### Beispiel 6

### 4-(2,2-Difluorethoxy)-9-methyl-7-phenyl-2-piperazin-1-yl-pyrido[3',2':4,5]thieno[3,2-d]pyrimidin (Verbindung 6; allgemeine Formel 1: R₁=Ph, R₂=H, R₃=Me, R₄=Piperazin-1-yl, R₅=OCH₂CHF₂, Y=S)

Analog Beispiel 1 aus 2-Chlor-4-(2,2-difluorethoxy)-9-methyl-7-phenyl-pyrido[3',2':4,5]thieno[3,2-*d*]pyrimidin (Beispiel 8q; allgemeine Formel 8: R₁=Ph, R₂=H, R₃=Me, R₅=OCH₂CHF₂, Y=S) und Piperazin (s. Tab. 1)

### Beispiel 7

### 4-(2,2,2-Trifluorethoxy)-9-methyl-7-phenyl-2-piperazln-1-yl-pyrido[3',2':4,5]thieno[3,2-d]pyrirnidin (Verbindung 7; allgemeine Formel 1: R₁=Ph, R₂=H, R₃=Me, R₄=Piperazin-1-yl, R₅=OCH₂CF₃, Y=S)

Analog Beispiel 1 aus 2-Chlor-4-(2,2,2-trifluorethoxy)-9-methyl-7-phenyl-pyrido[3',2':4,5]thieno[3,2-*d*]pyrimidin (Beispiel 8r; allgemeine Formel 8: R₁=Ph, R₂=H, R₃=Me, R₅=OCH₂CF₃, Y=S) und Piperazin (s. Tab. 1)

### Beispiel 8

### 4-Ethoxy-7-methyl-9-phenyl-2-piperazin-1-yl-pyrido[3',2':4,5]thieno[3,2-dJpyrimidin

### (Verbindung 8; allgemeine Formel 1: R₁=Me, R₂=H, R₃=Ph, R₄=Piperazin-1-yl, R₅=OEt, Y=S)

Analog Beispiel 1 aus 2-Chlor-4-ethoxy-7-methyl-9-phenyl-pyrido[3',2':4,5]thieno[3,2-*d*]pyrimidin (Beispiel 8k; allgemeine Formel 8: R₁=Me, R₂=H, R₃=Ph, R₅=OEt, Y=S) und Piperazin (s. Tab. 1)

### Beispiel 9

### 4-Ethoxy-7,9-diphenyl-2-piperazin-1-yl-pyrido[3',2':4,5]thieno[3,2-d]pyrimidin (Verbindung 9; allgemeine Formel 1: R₁=Ph, R₂=H, R₃=Ph, R₄=Piperazin-1-yl, R₅=OEt, Y=S)

Analog Beispiel 1 aus 2-Chlor-4-ethoxy-7,9-diphenyl-pyrido[3',2':4,5]thieno[3,2-*d*]pyrimidin (Beispiel 81; allgemeine Formel 8: R₁=Ph, R₂=H, R₃=Ph, R₅=OEt, Y=S) und Piperazin (s. Tab. 1)

### Beispiel 10

### 4-Ethoxy-7,9-dimethyl-8-(4'-nitrobenzyl)-2-piperazin-1-yl-pyrido[3',2':4,5]thieno[3,2-d]pyrimidin (Verbindung 10; allgemeine Formel 1: R₁=Me, R₂=4'-NO₂-Ph-CH₂, R₃=Me, R₄=Piperazin-1-yl, R₅=OEt, Y=S)

Analog Beispiel 1 aus 2-Chlor-4-ethoxy-7,9-dimethyl-8-(4'-nitrobenzyl)-pyrido[3',2':4,5]thieno[3,2-*d*]pyrimidin (Beispiel 8m; allgemeine Formel 8: R₁=Me, R₂=4'-NO₂-Ph-CH₂, R₃=Me, R₅=Et, Y=S) und Piperazin (s. Tab. 1)

### Beispiel 11

### 4-Ethoxy-2-homopiperazin-1-yl-9-methyl-7-phenyl-pyrido[3',2':4,5]thieno[3,2-d]pyrimidin

### (Verbindung 11; allgemeine Formel 1: R₁=Ph, R₂=H, R₃=Me, R₄=Homopiperazin-1-yl, R₅=OEt, Y=S)

Analog Beispiel 1 aus 2-Chlor-4-ethoxy-9-methyl-7-phenyl-pyrido[3',2':4,5]thieno[3,2-*d*]pyrimidin (Beispiel 8j; allgemeine Formel 8: R₁=Ph, R₂=H, R₃=Me, R₅=OEt, Y=S) und Homopiperazin (s. Tab. 1)

### Beispiel 12

### 2-Ethoxy-7-(3,4-dimethoxyphenyl)-9-methyl-4-piperazin-1-yl-pyrido[3',2':4,5]thieno[3,2-d]pyrimidin (Verbindung 12; allgemeine Formel 1: R₁=3,4-(MeO)₂-Ph, R₂=H, R₃=Me, R₄=OEt, R₅=Piperazin-1-yl, Y=S)

0.1 g (0.22 mmol) 2-Chlor-7-(3,4-dimethoxyphenyl)-9-methyl-4-piperazin-1-yl-pyrido-[3',2':4,5]thieno[3,2-*d*]pyrimidin (Beispiel 8a; allgemeine Formel 8: R₁=3,4-(MeO)₂₋Ph, R₂=H, R₃=Me, R₅=Piperazin-1-yl, Y=S) und 0.22 ml (0.44 mmol) Kaliumethylat-Tetrahydrofuran-Lösung (2M) werden in 10 ml abs. Dioxan 3 Stunden unter Rückfluß erhitzt. Anschließend wird das Lösungsmittel entfernt und der Rückstand in wenig Wasser suspendiert. Die wässrige Suspension wird mit Dichlormethan extrahiert, die organische Phase über Natriumsulfat getrocknet und das Lösungsmittel entfernt.
Ausbeute: 50 mg (49 %); HPLC t_{R}: 16.3 min, Reinheit: 97.5 % (s. a in Tab. 1);
MS (ESI, m/e): 466 [M+H]⁺

Ausgehend von den nachfolgend beschriebenen Vorstufen werden durch Umsetzung der entsprechenden 4-Amino-substituierten Verbindungen der allgemeinen Formel 8 mit Alkalialkanolat-Lösungen, welche aus Natriumhydrid bzw. Kalium und Methanol, Ethanol, iso-Propanol, 2,2-Difluorethanol, 2,2,2-Trifluorethanol und Ethylenglycol, ggf. unter Verwendung von Tetrahydrofuran, hergestellt werden, analog Beispiel 12 folgende Verbindungen (13-22) erhalten, wobei die Reinigung z.T. durch Flash-Chromatographie (Kieselgel) erfolgt:

### Beispiel 13

### 2-Ethoxy-7-(4-methoxyphenyl)-9-methyl-4-piperazin-1-yl-pyrido[3',2':4,5]thieno[3,2-d]pyrimidin (Verbindung 13; allgemeine Formel 1: R₁=4-MeO-Ph, R₂=H, R₃=Me, R₄=OEt, R₅=Piperazin-1-yl, Y=S)

Analog Beispiel 12 aus 2-Chlor-7-(4-methoxyphenyl)-9-methyl-4-piperazin-1-yl-pyrido[3',2':4,5]thieno[3,2-*d*]pyrimidin (Beispiel 8b; allgemeine Formel 8: R₁=4-MeO-Ph, R₂=H, R₃=Me, R₅=Piperazin-1-yl, Y=S) und Ethanol (s. Tab. 1)

### Beispiel 14

### 2-Ethoxy-9-methyl-7-phenyl-4-piperazin-1-yl-pyrido[3',2':4,5]thieno[3,2-d]pyrimidin

### (Verbindung 14; allgemeine Formel 1: R₁=Ph, R₂=H, R₃=Me, R₄=OEt, R₅=Piperazin-1-yl, Y=S)

Analog Beispiel 12 aus 2-Chlor-9-methyl-7-phenyl-4-piperazin-1-yl-pyrido[3',2':4,5]thieno[3,2-*d*]pyrimidin (Beispiel 8c; allgemeine Formel 8: R₁=Ph, R₂=H, R₃=Me, R₅=Piperazin-1-yl, Y=S) und Ethanol (s. Tab. 1)

### Beispiel 15

### 2-Isopropoxy-9-methyl-7-phenyl-4-piperazin-1-yl-pyrido[3',2':4,5]thieno[3,2-d]pyrimidin

### (Verbindung 15; allgemeine Formel 1: R₁=Ph, R₂=H, R₃=Me, R₄=OiPr, R₅=Piperazin-1-yl, Y=S)

Analog Beispiel 12 aus 2-Chlor-9-methyl-7-phenyl-4-piperazin-1-yl-pyrido[3',2':4,5]thieno[3,2-dlpyrimidin (Beispiel 8c; allgemeine Formel 8: R₁=Ph, R₂=H, R₃=Me, R₅=Piperazin-1-yl, Y=S) und iso-Propanol (s. Tab. 1)

### Beispiel 16

### 2-Methoxy-9-methyl-7-phenyl-4-piperazin-1-yl-pyrido[3',2':4,5]thieno[3,2-d]pyrimidin

### (Verbindung 16; allgemeine Formel 1: R₁=Ph, R₂=H, R₃=Me, R₄=OMe, R₅=Piperazin-1-yl, Y=S)

Analog Beispiel 12 aus 2-Chlor-9-methyl-7-phenyl-4-piperazin-1-yl-pyrido[3',2':4,5]thieno[3,2-*d*]pyrimidin (Beispiel 8c; allgemeine Formel 8: R₁=Ph, R₂=H, R₃=Me, R₅=Piperazin-1-yl, Y=S) und Methanol (s. Tab. 1)

### Beispiel 17

### 2-(2,2-Difluorethoxy)-9-methyl-7-phenyl-4-piperazin-1-yl-pyrido[3',2':4,5]thieno[3,2-d]pyrimidin (Verbindung 17; allgemeine Formel 1: R₁=Ph, R₂=H, R₃=Me, R₄=OCH₂CHF₂, R₅=Piperazin-1-yl, Y=S)

Analog Beispiel 12 aus 2-Chlor-9-methyl-7-phenyl-4-piperazin-1-yl-pyrido[3',2':4,5]thieno[3,2-*d*]pyrimidin (Beispiel 8c; allgemeine Formel 8: R₁=Ph, R₂=H, R₃=Me, R₅=Piperazin-1-yl, Y=S) und 2,2-Difluorethanol (s. Tab. 1)

### Beispiel 18

### 2-(2,2,2-Trifluorethoxy)-9-methyl-7-phenyl-4-piperazin-1-yl-pyrido[3',2':4,5]thieno[3,2-d]pyrimidin (Verbindung 18; allgemeine Formel 1: R₁=Ph, R₂=H, R₃=Me, R₄=OCH₂CF₃, R₅=Piperazin-1-yl, Y=S)

Analog Beispiel 12 aus 2-Chlor-9-methyl-7-phenyl-4-piperazin-1-yl-pyrido[3',2':4,5]thieno[3,2-*d*]pyrimidin (Beispiel 8c; allgemeine Formel 8: R₁=Ph, R₂=H, R₃=Me, R₅=Piperazin-1-yl, Y=S) und 2,2,2-Trifluorethanol (s. Tab. 1)

### Beispiel 19

### 2-Methoxy-7 -methyl-9-phenyl-4-piperazin-1-yl-pyrido[3',2' :4,5]thieno[3,2-d]pyrimidin

### (Verbindung 19; allgemeine Formel 1: R₁=Me, R₂=H, R₃=Ph, R₄=OMe, R₅=Piperazin-1-yl, Y=S)

Analog Beispiel 12 aus 2-Chlor-7-methyl-9-phenyl-4-piperazin-1-yl-pyrido[3',2':4,5]thieno[3,2-*d*]pyrimidin (Beispiel 8d; allgemeine Formel 8: R₁=Me, R₂=H, R₃=Ph, R₅=Piperazin-1-yl, Y=S) und Methanol (s. Tab. 1)

### Beispiel 20

### 2-Ethoxy-7,9-diphenyl-4-piperazin-1-yl-pyrido[3',2':4,5]thieno[3,2-dJpyrimidin (Verbindung 20; allgemeine Formel 1: R₁=Ph, R₂=H, R₃=Ph, R₄=OEt, R₅=Piperazin-1-yl, Y=S)

Analog Beispiel 12 aus 2-Chlor-7,9-diphenyl-4-piperazin-1-yl-pyrido[3',2':4,5]thieno[3,2-d]pyrimidin (Beispiel 8e; allgemeine Formel 8: R₁=Ph, R₂=H, R₃=Ph, R₅=Piperazin-1-yl, Y=S) und Ethanol (s. Tab. 1)

### Beispiel 21

### 2-Methoxy-7,9-dimethyl-4-piperazin-1-yl-pyrido[3',2':4,5]thieno[3,2-d]pyrimidin (Verbindung 21; allgemeine Formel 1: R₁=Me, R₂=H, R₃=Me, R₄=OMe, R₅=Piperazin-1-yl, Y=S)

Analog Beispiel 12 aus 2-Chlor-7,9-dimethyl-4-piperazin-1-yl-pyrido[3',2':4,5]thieno[3,2-*d*]pyrimidin (Beispiel 8s; allgemeine Formel 8: R₁=Me, R₂=H, R₃=Me, R₅=Piperazin-1-yl, Y=S) und Methanol (s. Tab. 1)

### Beispiel 22

### 2-Ethoxy-7,9-dimethyl-7-(4'-nitrophenyl)-4-piperazin-1-yl-pyrido[3',2':4,5]thieno[3,2-d]pyrimidin (Verbindung 22; allgemeine Formel 1: R₁=Me, R₂=4'-NO₂-Ph-CH₂, R₃=Me, R₄=OEt, R₅=Piperazin-1-yl, Y=S)

Analog Beispiel 12 aus 2-Chlor-7,9-dimethyl-7-(4'-nitrophenyl)-4-piperazin-1-yl pyrido[3',2':4,5]thieno[3,2-*d*]pyrimidin (Beispiel 8f; allgemeine Formel 8: R₁=Me, R₂=4'-NO₂-Ph-CH₂, R₃=Me, R₅=Piperazin-1-yl, Y=S) und Ethanol (s. Tab. 1)

### Beispiel 23

### 2-Ethylthio-9-methyl-7-phenyl-4-piperazin-1-yl-pyrido[3',2':4,5]thieno[3,2-djpyrimidin

### (Verbindung 23; allgemeine Formel 1: R₁=Ph, R₂=H, R₃=Me, R₄=SEt, R₅=Piperazin-1-yl, Y=S)

0.63 g (1.7 mmol) 4-Chlor-2-ethylthio-9-methyl-7-phenyl-pyrido[3',2':4,5]thieno[3,2-dlpyrimidin (Beispiel 17a; allgemeine Formel 17: R₁=Ph, R₂=H, R₃=Me, Alk*=Et, Y=S) werden mit 0.29 g (3.4 mmol) Piperazin in 10 ml Tetrahydrofuran 1 Stunde bei Raumtemperatur gerührt. Der Niederschlag wird abgesaugt, mit wenig Wasser gewaschen und trocken gesaugt. Anschließend wird der Niederschlag in ca. 300 ml Dichlormethan suspendiert, ausgerührt, filtriert und das Lösungsmittel des Filtrats entfernt.
Ausbeute: 0.67 g (93 %); HPLC t_{R}: 17.7 min, Reinheit: 99.7 % (s. a in Tab. 1);
MS (ESI, m/e): 422 [M+H]⁺; Schmp.: 150-154 °C

### Beispiel 24

### 9-Chlormethyl-2-ethylthio-7-phenyl-4-piperazin-1-yl-pyrido[3',2':4,5]thieno[3,2-d]pyrimidin (Verbindung 24; allgemeine Formel 1: R₁=Ph, R₂=H, R₃=ClCH₂, R₄=SEt, R₅=Piperazin-1-yl, Y=S)

Analog Beispiel 23 aus 4-Chlor-9-chlormethyl-2-ethylthio-7-phenyl-pyrido[3',2':4,5]thieno[3,2-dlpyrimidin (Beispiel 17b; allgemeine Formel 17: R₁=Ph, R₂=H, R₃=CICH₂, Alk*=Et, Y=S) und Piperazin (s. Tab. 1)

### Beispiel 25

### 2-Ethylsulfinyl-9-methyl-7-phenyl-4-piperazin-1 -yl-pyrido[3',2':4,5]thieno[3,2-d]pyrimidin

### (Verbindung 25; allgemeine Formel 1: R₁=Ph, R₂=H, R₃=Me, R₄=S(O)Et, R₅=Piperazin-1-yl, Y=S)

85 mg (0.2 mmol) 2-Ethylthio-9-methyl-7-phenyl-4-piperazin-1-yl-pyrido[3',2':4,5]thieno[3,2-*d*]pyrimidin (Verbindung 23; allgemeine Formel 1: R₁=Ph, R₂=H, R₃=Me, R₄=SEt, R₅=Piperazin-1-yl, Y=S), 3 ml Eisessig und 0.04 ml Wasserstoffperoxid (60 %) werden 48 Stunden bei Raumtemperatur gerührt. Nach Entfernen des Lösungsmittels wird der Rückstand durch Flash-Chromatographie (Kieselgel, Methanol/Dichlormethan 1:20 - 1:3) gereinigt.
Ausbeute: 50 mg (57 %); HPLC t_{R}: 15.0 min, Reinheit: 99.0 % (s. a in Tab. 1);
MS (ESI, m/e): 438 [M+H]⁺

### Beispiel 26

### 2-Ethylsulfonyl-9-methyl-7-phenyl-4-piperazin-1-yl-pyrido[3',2':4,5]thieno[3,2-djpyrimidin

### (Verbindung 26; allgemeine Formel 1: R₁=Ph, R₂=H, R₃=Me, R₄=S(O)₂Et, R₅=Piperazin-1-yl, Y=S)

85 mg (0.2 mmol) 2-Ethylthio-9-methyl-7-phenyl-4-piperazin-1-yl-pyrido[3',2':4,5]thieno[3,2-*d*]pyrimidin (Verbindung **23**; allgemeine Formel 1: R₁=Ph, R₂=H, R₃=Me, R₄=SEt, R₅=Piperazin-1-yl, Y=S), 3 ml Eisessig und 0.12 ml Wasserstoffperoxid (60 %) werden 96 Stunden bei Raumtemperatur gerührt. Nach Entfernen des Lösungsmittels wird der Rückstand durch Flash-Chromatographie (Kieselgel, Methanol/Dichlormethan 1:20 - 1:10) gereinigt.
Ausbeute: 16 mg (18 %); HPLC t_{R}: 15.0 min, Reinheit: 95.9 % (s. a in Tab. 1);
MS (ESI, m/e): 454 [M+H]⁺

### Beispiel 27

### 2-Ethyl-9-methyl-7-phenyl-4-piperazin-1-yl-pyrido[3',2':4,5]thieno[3,2-d]pyrimidin

### (Verbindung 27; allgemeine Formel 1: R₁=Ph, R₂=H, R₃=Me, R₄=Et, R₅=Piperazin-1-yl, Y=S)

0.24 g (0.7 mmol) 4-Chlor-2-ethyl-9-methyl-7-phenyl-pyrido[3',2':4,5]thieno[3,2-*d*]pyrimidin (Beispiel 13a; allgemeine Formel 1: R₁=Ph, R₂=H, R₃=Me, R₄=Et, Y=S) werden mit 0.27 g (3.1 mmol) Piperazin in 6 ml Tetrahydrofuran 2.5 Stunden bei Raumtemperatur gerührt. Das Lösungsmittel wird entfernt und der Rückstand mit ca. 10 ml Wasser aufgenommen, abgesaugt, mit ca. 10 ml Wasser gewaschen und trocken gesaugt. Der Rückstand wird durch Flash-Chromatographie (Kieselgel, Methanol/Chloroform 1:20) gereinigt.
Ausbeute: 0.20 g (73 %); HPLC t_{R}: 16.8 min, Reinheit: 99.3 % (s. a in Tab. 1);
MS (ESI, m/e): 390 [M+H]⁺

### Beispiel 28

### 8-(4'-Aminobenzyl)-4-ethoxy-7,9-dimethyl-2-piperazin-1-yl-pyrido[3',2':4,5]thieno[3,2-d]pyrimidin (Verbindung 28; allgemeine Formel 1: R₁=Me, R₂=4'-NH2-Ph-CH₂, R₃=Me, R₄=Piperazin-1-yl, R₅=OEt, Y=S)

Zu 0.36 g (0.8 mmol) 4-Ethoxy-7,9-dimethyl-8-(4'-nitrobenzyl)-2-piperazin-1-yl-pyrido[3',2':4,5]thieno[3,2-*d*]pyrimidin (Verbindung 10; allgemeine Formel 1: R₁=Me, R₂=4'-NO₂-Ph-CH₂, R₃=Me, R₄=Piperazin-1-yl, R₅=OEt, Y=S) in 3 ml Ethanol werden 40 mg Palladium/Aktivkohle (10% Pd) und 0.5 ml (10 mmol) Hydraziniumhydroxid gegeben. Es wird 10 Stunden unter Rückfluß erhitzt, das Lösungsmittel entfernt und der Rückstand mit 50 ml Chloroform aufgenommen. Nach Filtrieren wird das Lösungsmittel entfernt und der Rückstand aus Chloroform umkristallisiert, wobei mit Cyclohexan ausgefällt wird.
Ausbeute: 0.16 g (45 %); HPLC t_{R}: 12.9 min, Reinheit: 94.5 % (s. a in Tab. 1);
MS (ESI, m/e): 449 [M+H]⁺

### Beispiel 29

### 4-Propoxy-9-methyl-7-phenyl-2-piperazin-1-yl-pyrido[3',2':4,5]thieno[3,2-d]pyrimidin

### (Verbindung 29; allgemeine Formel 1: R₁=Ph, R₂=H, R₃=Me, R₄=Piperazin-1-yl, R₅=0nPr, Y=S)

Analog Beispiel 1 aus 2-Chlor-4-propoxy-9-methyl-7-phenyl-pyrido[3',2':4,5]thieno[3,2-dJpyrimidin (Beispiel 8n; allgemeine Formel 8: R₁=Ph, R₂=H, R₃=Me, R₅=OnPr, Y=S) und Piperazin (s. Tab. 1 )

### Beispiel 30

### 4-Ethoxy-9-methyl-7-phenyl-2-piperazin-1-yl-pyrido[3',2':4,5]furo[3,2-d]pyrimidin

### (Verbindung 30; allgemeine Formel 1: R₁=Ph, R₂=H, R₃=Me, R₄=Piperazin-1-yl, R₅=OEt, Y=O)

Analog Beispiel 1 aus 2-Chlor-4-ethoxy-9-methyl-7-phenyl-pyrido[3',2':4,5]furo[3,2-d]pyrimidin (Beispiel 8t; allgemeine Formel 8: R₁=PH, R₂=H, R₃=Me, R₅=OEt, Y=O) und Piperazin (s. Tab. 1)

### Beispiel 31

### 9-Methyl-7-phenyl-4-piperazin-1-yl-2-propyl-pyrido[3',2':4,5]thieno[3,2-d]pyrimidin

### (Verbindung 31; allgemeine Formel 1: R₁=Ph, R₂=H, R₃=Me, R₄=nPr, R₅=Piperazin-1-yl, Y=S)

Analog Beispiel 27 aus 4-Chlor-9-methyl-7-phenyl-2-propyl-pyrido[3',2':4,5]thieno[3,2-*d*]pyrimidin (Beispiel 13b; allgemeine Formel 1: R₁=PH, R₂=H, R₃=Me, R₄=*n*Pr, Y=S) und Piperazin
Zur Reinigung wird der Rückstand mit Ether gewaschen.

### Beispiel 32

### 7-(3,4-Dimethoxyphenyl)-9-methyl-4-piperazin-1-yl-2-propyl-pyrido[3',2':4,5]thieno[3,2-d]pyrimidin (Verbindung 32; allgemeine Formel 1: R₁=3,4-(MeO)₂-Ph, R₂=H, R₃=Me, R₄=nPr, R₅=Piperazin-1-yl, Y=S)

Analog Beispiel 27 aus 4-Chlor-7-(3,4-dimethoxyphenyl)-9-methyl-2-propyl-pyrido[3',2':4,5]thieno[3,2-dlpyrimidin (Beispiel 13c; allgemeine Formel 1: R₁=3,4-(MeO)₂-Ph, R₂=H, R₃=Me, R₄=*n*Pr, Y=S) und Piperazin
Der Rückstand wird in ca. 50 ml Dichlormethan suspendiert und filtriert. Das Filtrat wird mit insgesammt 30 ml Wasser gewaschen, über Natriumsulfat getrocknet und das Lösungsmittel entfernt. Der Rückstand wird aus Essigsäureethylester umkristallisiert.

### Beispiel 33

### 4-(2-Hydroxyethoxy)-9-methyl-7-phenyl-2-piperazin-1-yl-pyrido[3',2':4,5]thieno[3,2-d]pyrimidin (Verbindung 33; allgemeine Formel 1: R₁=Ph, R₂=H, R₃=Me, R₄=Piperazin-1-yl, R₅=OCH₂CH₂OH, Y=S)

Analog Beispiel 1 aus 2-Chlor-4-(2-hydroxyethoxy)-9-methyl-7-phenyl-pyrido[3',2':4,5]thieno[3,2-*d*]pyrimidin (Beispiel 8u; allgemeine Formel 8: R₁=Ph, R₂=H, R₃=Me, R₅=OCH₂CH₂OH, Y=S) und Piperazin (s. Tab. 1)

### Beispiel 34

### 2-(2-Hydroxyethoxy)-9-methyl-7-phenyl-4-piperazin-1 -yl-pyrido[3",2":4,5]thieno[3,2-d]pyrimidin (Verbindung 34; allgemeine Formel 1: R₁=Ph, R₂=H, R₃=Me, R₄=OCH₂CH₂OH, R₅= Piperazin-1-yl, Y=S)

Analog Beispiel 12 aus 2-Chlor-9-methyl-7-phenyl-4-piperazin-1-yl-pyrido[3',2':4,5]thieno[3,2-*d*]pyrimidin (Beispiel 8c; allgemeine Formel 8: R₁=Pₕ, R₂=H, R₃=Me, R₅=Piperazin-1-yl, Y=S) und Ethylenglycol (s. Tab. 1)

### Beispiel 35

### 2-Ethoxy-9-methyl-7-phenyl-4-piperazin-1 -yl-pyrido[3',2':4,5]furo[3,2-d]pyrimidin

### (Verbindung 35; allgemeine Formel 1: R₁=Ph, R₂=H, R₃=Me, R₄=OEt, R₅=Piperazin-1-yl, Y=O)

Analog Beispiel 12 aus 2-Chlor-4-piperazin-1-yl-9-methyl-7-phenyl-pyrido[3',2':4,5]furo[3,2-*d*]pyrimidin (Beispiel 8v; allgemeine Formel 8: R₁=Ph, R₂=H, R₃=Me, R₅=Piperazin-1-yl, Y=O) und Ethanol (s. Tab. 1)
Die Reinigung erfolgt durch Flash-Chromatographie (Kieselgel, Ethanol/Chloroform 2:8).

### Beispiel 36

### 2-Ethoxy-4-homopiperazin-1-yl-9-methyl-7-phenyl-pyrido[3',2':4,5]thieno[3,2-d]pyrimidin

### (Verbindung 36; allgemeine Formel 1: R₁=Ph, R₂=H, R₃=Me, R₄=OEt, R₅=Homopiperazin-1-yl, Y=S)

Analog Beispiel 12 aus 2-Chlor-4-homopiperazin-1-yl-9-methyl-7-phenyl-pyrido[3',2':4,5]thieno[3,2-*d*]pyrimidin (Beispiel 8w; allgemeine Formel 8: R₁=Ph, R₂=H, R₃=Me, R₅=Homo-piperazin-1-yl, Y=S) und Ethanol (s. Tab. 1)

### Beispiel 37

### 4-Ethoxy-7-(3,4-dimethoxyphenyl)-9-methyl-2-piperazin-1-yl-pyrido[3',2':4,5]thieno[3,2-d]pyrimidin-hydrochlorid (Verbindung 1-HCl;

### allgemeine Formel 1: R₁=3,4-(MeO)₂-Ph, R₂=H, R₃=Me, R₄=Piperazin-1-yl, R₅=OEt, Y=S, x HCl)

19 mg (41.6µmol) 4-Ethoxy-7-(3,4-dimethoxyphenyl)-9-methyl-2-piperazin-1-yl-pyrido[3',2':4,5]thieno[3,2-d]pyrimidin (Verbindung 1; allgemeine Formel 1: R₁=3,4-(MeO)₂-Ph, R₂=H, R₃=Me, R₄=Piperazin-1-yl, R₅=OEt, Y=S) werden in 1 ml Dichlormethan gelöst. Es werden 34 µl ethanolischer Salzsäure (1.25 M) zugegeben und der gebildete Niederschlag abgesaugt und i.Vak. über Kaliumhydroxid getrocknet.
Ausbeute: 9 mg (43 %)

Ausgehend von den nachfolgend beschriebenen Vorstufen werden durch Umsetzung der entsprechenden Verbindungen der allgemeinen Formel 1 a bzw. 1 b mit ethanolischer Salzsäure folgende Verbindungen (2-HCl, 4-HCl, 9-HCl, 14-HCl, 15-HCl) erhalten:

### Beispiel 38

### 7-(4-Methoxyphenyl)-4-ethoxy-9-methyl-2-piperazin-1-yl-pyrido[3',2':4,5]thieno-[3,2-d]pyrimidin-hydrochlorid (Verbindung 2-HCl; allgemeine Formel 1: R₁=4-MeO-Ph, R₂=H, R₃=Me, R₄=Piperazin-1-yl, R₅=OEt, Y=S, x HCl)

Analog Beispiel 2-HCl aus 4-Ethoxy-7-(4-methoxyphenyl)-9-methyl-2-piperazin-1-yl-pyrido[3',2':4,5]thieno[3,2-*d*]pyrimidin (Verbindung 2; allgemeine Formel 1: R₁=4-MeO-Ph, R₂=H, R₃=Me, R₄=Piperazin-1-yl, R₅=OEt, Y=S) und ethanolischer Salzsäure (s. Tab. 1)

### Beispiel 39

### 4-Isopropoxy-9-methyl-7-phenyl-2-piperazin-1-yl-pyrido[3',2':4,5]thieno[3,2-d]pyrimidin-hydrochlorid (Verbindung 4-HCI; allgemeine Formel 1: R₁=Ph, R₂=H, R₃=Me, R₄=Piperazin-1-yl, R₅=OiPr, Y=S, x HCl)

Analog Beispiel 1-HCI aus 4-Isopropoxy-9-methyl-7-phenyl-2-piperazin-1-yl-pyrido[3',2':4,5]thieno[3,2-*d*]pyrimidin (Verbindung 4; allgemeine Formel 1: R₁=Ph, R₂=H, R₃=Me, R₄=Piperazin-1-yl, R₅=O*i*Pr, Y=S) und ethanolischer Salzsäure (s. Tab. 1)

### Beispiel 40

### 4-Ethoxy-7,9-diphenyl-2-piperazin-1 -yl-pyrido[3',2':4,5]thieno[3,2-d]pyrimidin-hydrochlorid (Verbindung 9-HCl; allgemeine Formel 1: R₁=Ph, R₂=H, R₃=Ph, R₄=Piperazin-1-yl, R₅=OEt, Y=S, x HCl)

Analog Beispiel 1-HCl aus 4-Ethoxy-7,9-diphenyl-2-piperazin-1-yl-pyrido[3',2':4,5]thieno[3,2-*d*]pyrimidin (Verbindung 9; allgemeine Formel 1: R₁=Ph, R₂=H, R₃=Ph, R₄=Piperazin-1-yl, R₅=OEt, Y=S) und ethanolischer Salzsäure (s. Tab. 1)

### Beispiel 41

### 2-Ethoxy-9-methyl-7-phenyl-4-piperazin-1-yl-pyrido[3',2':4,5]thieno[3,2-d]pyrimidin-hydrochlorid (Verbindung 14-HCl; allgemeine Formel 1: R₁=Ph, R₂=H, R₃=Me, R₄=OEt, R₅=Piperazin-1-yl, Y=S, x HCl)

Analog Beispiel 1-HCl aus 2-Ethoxy-9-methyl-7-phenyl-4-piperazin-1-yl-pyrido[3',2':4,5]thieno[3,2-d]pyrimidin (Verbindung 14; allgemeine Formel 1: R₁=Ph, R₂=H, R₃=Me, R₄=OEt, R₅=Piperazin-1-yl, Y=S) und ethanolischer Salzsäure (s. Tab. 1)

### Beispiel 42

### 2-Isopropoxy-9-rnethyl-7-phenyl-4-piperazin-1-yl-pyrido[3',2':4,5]thieno[3,2-d]pyrimidin-hydrochlorid (Verbindung 15-HCl; allgemeine Formel 1: R₁=Ph, R₂=H, R₃=Me, R₄=OiPr, R₅=Piperazin-1-yl, Y=S, x HCl)

Analog Beispiel 1-HCl aus 2-lsopropoxy-9-methyl-7-phenyl-4-piperazin-1-yl-pyrido[3',2':4,5]thieno[3,2-*d*]pyrimidin (Verbindung 15; allgemeine Formel 1: R₁=Ph, R₂=H, R₃=Me, R₄=O*i*Pr, R₅=Piperazin-1-yl, Y=S) und ethanolischer Salzsäure (s. Tab. 1)

Die nachfolgenden Beispiele beschreiben die Herstellung von Vorstufen der Verbindungen 1, 1 a bzw. 1 b

### Beispiel 43

### Beispiele zur Herstellung von Verbindungen der allgemeinen Formel 2

Methode A: Darstellung von 5-unsubstituierten Verbindungen der allgemeinen Formel 2

### 2-Mercapto-6-(3,4-dimethoxyphenyl)-4-methyl-pyridin-3-carbonitril (Experiment 2a; allgemeine Formel 2: R₁=3,4-(MeO)₂-Ph, R₂=H, R₃=Me, Y=S)

5.0 g (22.5 mmol) 1-(3,4-Dimethoxyphenyl)butan-1,3-dion*, 2.9 g (29.2 mmol) Cyanthioacetamid und 4.0 g (29.2 mmol) Kaliumcarbonat werden bei Raumtemperatur 21 Stunden in 170 ml Aceton gerührt. Anschließend wird das Lösungsmittel entfernt und der Rückstand in Ethanol/Wasser (1:1) gerade gelöst. Nach Ansäuern mit Eisessig wird der erhaltene Niederschlag abgesaugt, mit wenig Wasser gewaschen, trocken gesaugt und i. Vak. bei 50 °C getrocknet.
Ausbeute: 6.34 g (98 %); HPLC t_{R}: 12.9 min, Reinheit: 87.5 % (s. a in Tab. 3);
MS (ESI, m/e): 287 [M+H]⁺
(* Nicht kommerziell verfügbare1,3-Diketone werden nach bekannten Verfahren erhalten und sind in der Fachliteratur beschrieben.)

### Methode B: Darstellung von 5-Benzyl-substituierten Verbindungen der allgemeinen Formel 2

### 2-Mercapto-4,6-dimethyl-5-(4'-nitrobenzyl)-pyridin-3-carbonitril (Experiment 2h; allgemeine Formel 2: R₁=Me, R2=4'-NO₂-Ph-CH₂, R₃=Me, Y=S)

Zu einer Lösung von 1.28 g (55.7 mmol) Natrium in 50 ml abs. Ethanol werden 5.70 ml (55.2 mmol) Acetylaceton und 0.75 g (5.0 mmol) Natriumiodid gegeben. Nach Eintragen einer Suspension von 13.20 g (61.1 mmol) 4'-Nitrobenzylbromid in 60 ml abs. Ethanol wird 3 Stunden unter Rückfluß erhitzt. Nach Abkühlen und Filtrieren werden 5.23 g (52.2 mmol) Cyanthioacetamid und 5 ml (36.1 mmol) Triethylamin zum Filtrat hinzugefügt. Anschließend wird 4 Stunden unter Rückfluß erhitzt. Der nach Kühlen erhaltene Niederschlag wird abgesaugt, mit ca. 50 ml Ethanol und ca. 10 ml Ether rasch gewaschen, trocken gesaugt und bei i. Vak. bei 50 °C getrocknet.
Ausbeute: 5.30 g (32 %); HPLC t_{R}: 13.3 min, Reinheit: 95.9 % (s. a in Tab. 3);
MS (ESI, m/e): 300 [M+H]⁺

Analog dargestellte Beispiele der allgemeinen Formel 2 sind in Tab. 3 aufgeführt.

### Beispiel 44

### Beispiele zur Herstellung von Verbindungen der allgemeinen Formel 4

### 3-Amino-6-(3,4-dimethoxyphenyl)-4-methyl-thieno[2,3-b]pyridin-2-carbonsäureamid

### (Experiment 4a; allgemeine Formel 4: R₁=3,4-(MeO)₂-Ph, R₂=H, R₃=Me, Y=S)

2.1 g (31.4 mmol) Natriumethylat werden in 40 ml abs. Ethanol gelöst. Es werden 3.0 g (10.5 mmol) 2-Mercapto-6-(3,4-dimethoxyphenyl)-4-methyl-pyridin-3-carbonitril (Beispiel 2a; allgemeine Formel 2: R₁=3,4-(MeO)₂-Ph, R₂=H, R₃=Me, Y=S) und 1.2 g (12.6 mmol) 2-Chloracetamid zugegeben und 1 Stunde unter Rückfluß erhitzt. Nach dem Abkühlen werden 10 ml Wasser zugegeben und der Niederschlag abgesaugt, trocken gesaugt und i. Vak. bei 50 °C getrocknet.
Ausbeute: 3.4 g (94 %); HPLC t_{R}: 14.0 min, Reinheit: 99.8 % (s. a in Tab. 4);
MS (ESI, m/e): 344 [M+H]⁺

Analog dargestellte Beispiele der allgemeinen Formel 4 sind in Tab. 4 aufgeführt.

### Beispiel 45

### Beispiele zur Herstellung von Verbindungen der allgemeinen Formel 6

### Methode A:

### 7-(3,4-Dimethoxyphenyl)-9-methyl-pyrido[3',2':4,5]thieno[3,2-d]pyrimidin-2,4-dion

### (Experiment 6a; allgemeine Formel 6: R₁=3,4-(MeO)₂-Ph, R₂=H, R₃=Me, Y=S)

3.0 g (8.7 mmol) 3-Amino-6-(3,4-dimethoxyphenyl)-4-methyl-thieno[2,3-*b*]pyridin-2-carbonsäureamid (Beispiel 4a; allgemeine Formel 4: R₁=3,4-(CH₃O)₂-Ph, R₂=H, R₃=Me, Y=S) werden in 100 ml abs. Dioxan suspendiert. Anschließend werden 1.1 ml (8.7 mmol) Chlorameisensäure-trichlormethylester (Diphosgen) zugegeben und 3 Stunden unter Rückfluß erhitzt. Nach Abkühlen wird der Niederschlag abgesaugt und anschließend in 50 ml Wasser suspendiert. Die Suspension wird mit konz. Ammoniak-Lösung auf pH 8 eingestellt und der Niederschlag abgesaugt, mit wenig Wasser gewaschen, trocken gesaugt und i. Vak. bei 50 °C getrocknet.
Ausbeute: 3.08 g (96 %); HPLC t_{R}: 13.8 min, Reinheit: 99.9 % (s. a in Tab. 5);
MS (ESI, m/e): 368 [M-H]⁻

### Methode B

### 9-Methyl-7-phenyl-pyrido[3',2':4,5]furo[3,2-d]pyrimidin-2,4-dion (Experiment 6i; allgemeine Formel 6: R₁=Ph, R₂=H, R₃=Me, Y=O)

Zu 1.0 g (3.4 mmol) 3-Amino-4-methyl-6-phenyl-furo[2,3-b]pyridin-2-carbonsäureethylester (allgemeine Formel 14: R₁=Ph, R₂=H, R₃=Me, Y=O)* in 20 ml abs. Aceton werden 1.0 g (6.8 mmol) Benzoylisocyanat gegeben und 30 Minuten bei Raumtemperatur gerührt. Das Lösungsmittel wird entfernt und der nach Zugabe von Wasser ausgefallene Niederschlag abgesaugt und trocken gesaugt. Der Niederschlag wird in 6.8 ml (6.8 mmol) Natriumhydroxid-Lösung (1 M) und 20 ml Ethanol 15 Minuten unter Rückfluss erhitzt. Das Lösungsmittel wird entfernt und der Rückstand mit 100 ml abs. Dichlormethan aufgenommen. Nach Zugabe von 0.44 ml (5.1 mmol) Oxalylchlorid wird 1 Stunde bei Raumtemperatur gerührt. Das Lösungsmittel wird entfernt und der Rückstand mit Wasser suspendiert. Der entstandene Niederschlag wird abgesaugt, mit wenig Ethanol gewaschen, trocken gesaugt und i. Vak. bei 50 °C getrocknet.
Ausbeute: 0.85 g (85 %); HPLC t_{R}: 15.0 min, Reinheit: 85.1 % (s. a in Tab. 5);
MS (ESI, m/e): 294 [M+H]⁺

### (* beschrieben in Wagner G., Prantz J. Pharmazie (1990); 45, 213-214)

Analog dargestellte Beispiele der allgemeinen Formel 6 sind in Tab. 5 aufgeführt.

### Beispiel 46

### Beispiele zur Herstellung von Verbindungen der allgemeinen Formel 7

### 2,4-Dichlor-7-(3,4-dimethoxyphenyl)-9-methyl-pyrido[3',2':4,5]thieno[3,2-d]pyrimidin

### (Experiment 7a; allgemeine Formel 7: R₁=3,4-(MeO)₂-Ph, R₂=H, R₃=Me, Y=S)

3.0 g (8.1 mmol) 7-(3,4-Dimethoxyphenyl)-9-methyl-pyrido[3',2':4,5]thieno[3,2-*d*]-pyrimidin-2,4-dion (Beispiel 6a; allgemeine Formel 6: R₁=3,4-(MeO)₂-Ph, R₂=H, R₃=Me, Y=S) und 6.8 ml (48.7 mmol) Dichlorphenylphosphinoxid werden 6 Stunden bei 200 °C erhitzt. Anschließend wird auf ca. 20 g Eiswasser gegossen und mit gesättigter Natriumhydrogencarbonat-Lösung neutralisiert. Der Niederschlag wird abgesaugt, mit ca. 100 ml Wasser gewaschen, trocken gesaugt und i. Vak. bei 50 °C getrocknet.
Ausbeute: 3.08 g (94 %), HPLC t_{R}: 21.8 min, Reinheit: 85.5 % (s. a in Tab. 6);
MS (ESI, m/e): 407 [M+H]⁺

Analog dargestellte Beispiele der allgemeinen Formel 7 sind in Tab. 6 aufgeführt.

### Beispiel 47

### Beispiele zur Herstellung von Verbindungen der allgemeinen Formel 8

Methode A: Darstellung von 4-Amino-substituierten Verbindungen der allgemeinen Formel 8

### 2-Chlor-7-(3,4-dimethoxyphenyl)-9-methyl-4-piperazin-1-yl-pyrido[3',2':4,5]thieno[3,2-d]pyrimidin (Experiment 8a; allgemeine Formel 8: R₁=3,4-(MeO)₂-Ph, R₂=H, R₃=Me, R,₅=Piperazin-1-yl, Y=S)

0.5 g (1.2 mmol) 2,4-Dichlor-7-(3,4-dimethoxyphenyl)-9-methyl-pyrido[3',2':4,5]-thieno[3,2-d]pyrimidin (Beispiel 7a; allgemeine Formel 7: R₁=3,4-(MeO)₂-Ph, R₂=H, R₃=Me, Y=S), 0.13 g (1.5 mmol) Piperazin und 0.2 ml (1.5 mmol) Triethylamin werden in 20 ml abs. Tetrahydrofuran 6 Stunden bei Raumtemperatur gerührt. Anschließend wird das Lösungsmittel entfernt, der Rückstand in Wasser suspendiert, abgesaugt, mit ca. 40 ml Wasser gewaschen, trocken gesaugt und i. Vak. bei 50 °C getrocknet.
Ausbeute: 0.49 g (90 %); HPLC t_{R}: 16.2 min, Reinheit: 81.4 % (s. a in Tab. 7);
MS (ESI, m/e): 456 [M+H]⁺

Analog dargestellte 4-Amino-substituierte Verbindungen der allgemeinen Formel 8 sind in Tab. 7 aufgeführt.

Methode B: Darstellung von 4-Alkoxy-substituierten Verbindungen der allgemeinen Formel 8

### 2-Chlor-4-ethoxy-7-(3,4-dimethoxyphenyl)- 9-methyl-pyrido[3',2':4,5]thieno[3,2-d]pyrimidin (Experiment 8g; allgemeine Formel 8: R₁=3,4-(MeO)₂-Ph, R₂=H, R₃=Me, R₅=OEt, Y=S)

1.5 g (3.7 mmol) 2,4-Dichlor-7-(3,4-dimethoxyphenyl)-9-methyl-pyrido[3',2':4,5]thieno[3,2-*d*]pyrimidin (Beispiel 7a; allgemeine Formel 7: R₁=3,4-(MeO)₂-Ph, R₂=H, R₃=Me, Y=S) und 0.7 g (11.1 mmol) Natriumethylat werden in 50 ml abs. Ethanol 24 Stunden bei Raumtemperatur gerührt. Anschließend wird der Niederschlag abgesaugt, mit ca. 40 ml Wasser gewaschen, trocken gesaugt und i. Vak. bei 50 °C getrocknet
Ausbeute: 1.38 g (90 %); HPLC t_{R}: 21.5 min, Reinheit: 99.9 % (s. a in Tab. 7);
MS (ESI, m/e): 416 [M+H]⁺

In analoger Weise erfolgt die Darstellung von 4-Alkoxy-substituierten Verbindungen der allgemeinen Formel 8 unter Verwendung von Natriumethylat-Lösung oder Natriumalkanolat-Lösungen, welche aus Natriumhydrid und *n*-Propanol, iso-Propanol, Cyclopropylmethanol, 2,2-Difluorethanol, 2,2,2-Trifluorethanol und Ethylenglycol, ggf. unter Verwendung von Tetrahydrofuran, hergestellt werden. Gegebenenfalls wird durch Flash-Chromatographie (Kieselgel) gereinigt.

Analog dargestellte 4-Alkoxy-substituierte Verbindungen der allgemeinen Formel 8 sind in Tab. 7 aufgeführt.

### Beispiel 48

### Beispiele zur Herstellung von Verbindungen der allgemeinen Formel 12

### 2-Ethyl-9-methyl-7-phenyl-pyrido[3',2':4,5]thieno[3,2-d]pyrimidin-4-on (Experiment 12a; allgemeine Formel 12: R₁=Ph, R₂=H, R₃=Me, R₄=Et, Y=S)

Es werden 0.86 g (3.0 mmol) 3-Amino-4-methyl-6-phenyl-thieno[2,3-*b*]pyridin-2-carbonsäureamid (Beispiel 4d; allgemeine Formel 4: R₁=Ph, R₂=H, R₃=Me, Y=S) und 5.0 ml (39.2 mmol) Propionsäureanhydrid in 20 ml Toluol 2 Stunden unter Rückfluß erhitzt. Nach Abkühlen wird der Niederschlag abgesaugt, mit ca. 5 ml Ethanol gewaschen und trocken gesaugt. Der Rückstand wird in 5 ml (15.0 mmol) Natriumhydroxid-Lösung (3N) 15 Minuten unter Rückfluß erhitzt. Nach Abkühlen werden 5 ml Wasser zugegeben und es wird mit Essigsäure (10 %) neutralisiert. Anschließend wird der Niederschlag abgesaugt, mit ca. 10 ml Wasser gewaschen, trocken gesaugt und i. Vak. bei 50 °C getrocknet.
Ausbeute: 0.29 g (30 %); HPLC t_{R}: 17.6 min, Reinheit: 96.2 % (s. a in Tab. 7); MS (ESI, m/e): 322 [M+H]⁺

### 9-Methyl-7-phenyl-2-propyl-pyrido[3',2':4,5]thieno[3,2-d]pyrimidin-4-on (Experiment 12b; allgemeine Formel 12: R₁=Ph, R₂=H, R₃=Me, R₄=nPr, Y=S)

Analog Verbindung 12a aus 3-Amino-4-methyl-6-phenyl-thieno[2,3-b]pyridin-2-carbonsäureamid (Beispiel 4d; allgemeine Formel 4: R₁=Ph, R₂=H, R₃=Me, Y=S) und Buttersäureanhydrid
Es wird bei 170 °C gerührt. Nach Abkühlen wird mit Natriumhydroxid alkalisiert und kurz erwärmt. Der Niederschlag wird abgesaugt, mit Wasser gewaschen, trocken gesaugt und i. Vak. bei 50 °C getrocknet.
Ausbeute: 0.55 g (91 %); HPLC t_{R}: 20.3 min, Reinheit: 46.8 % (s. a in Tab. 7); MS (ESI, m/e): 336 [M+H]⁺

### 7-(3,4-Dimethoxyphenyl)-9-methyl-2-propyl-pyrido[3',2':4,5]thieno[3,2-d]pyrimidm-4-on (Experiment 12c; allgemeine Formel 12: R₁=3,4-(MeO)₂-Ph, R₂=H, R₃=Me, R₄=nPr, Y=S)

Analog Verbindung 12a aus 3-Amino-6-(3,4-dimethoxyphenyl)-4-methyl-thieno[2,3-b]pyridin-2-carbonsäureamid (Beispiel 4a; allgemeine Formel 4: R₁=3,4-(MeO)₂-Ph, R₂=H, R₃=Me, Y=S) und Buttersäureanhydrid
Der Rückstand wird in 5 ml (15.0 mmol) Natriumhydroxid-Lösung (3N) und 5 ml Ethanol 6 Stunden unter Rückfluß erhitzt. Nach Ansäuern mit Salzsäure (10 %) wird der Niederschlag mit Wasser gewaschen, trocken gesaugt und i. Vak. bei 50 °C getrocknet.
Ausbeute: 0.59 g (47 %); HPLC t_{R}: 17.4 min, Reinheit: 89.8 % (s. a in Tab. 7);
MS (ESI, m/e): 396 [M+H]⁺

### Beispiel 49

### Beispiele zur Herstellung von Verbindungen der allgemeinen Formel 13

### 4-Chlor-2-ethyl-9-methyl-7-phenyl-pyrido[3',2':4,5]thieno[3,2-d]pyrimidin (Experiment 13a; allgemeine Formel 13: R₁=Ph, R₂=H, R₃=Me, R₄=Et, Y=S)

0.29 g (0.9 mmol) 2-Ethyl-9-methyl-7-phenyl-pyrido[3',2':4,5]thieno[3,2-d]pyrimidin-4-on (Beispiel 12a; allgemeine Formel 12: R₁=Ph, R₂=H, R₃=Me, R₄=Et, Y=S) werden in 5 ml (54 mmol) Phosphoroxychlorid 6 Stunden unter Rückfluß erhitzt. Anschließend wird auf ca. 20 g Eiswasser gegossen und mit Natriumhydrogencarbonat neutralisiert. Der Niederschlag wird abgesaugt, mit ca. 20 ml Wasser gewaschen, trocken gesaugt und i. Vak. bei 50 °C getrocknet.
Ausbeute: 0.24 g (78 %); HPLC t_{R}: 23.2 min; Reinheit: 94.2 % (s. a in Tab. 7);
MS (ESI, m/e): 340 [M+H]⁺

### 4-Chlor-9-methyl-7-phenyl-2-propyl-pyrido[3',2':4,5]thieno[3,2-d]pyrimidin (Experiment 13b; allgemeine Formel 13: R₁=Ph, R₂=H, R₃=Me, R₄=nPr, Y=S)

Analog Verbindung 13a aus 9-Methyl-7-phenyl-2-propyl-pyrido[3',2':4,5]thieno[3,2-*d*]pyrimidin-4-on (Beispiel 12b; allgemeine Formel 12: R₁=Ph, R₂=H, R₃=Me, R₄=*n*Pr, Y=S) und Phosphoroxychlorid
Es wird durch Flash-Chromatographie (Kieselgel, Chloroform) gereinigt .
Ausbeute: 0.25 g (44 %); HPLC t_{R}: 23.9 min; Reinheit: 89.8 % (s. a in Tab. 7);
MS (ESI, m/e): 354 [M+H]⁺

### 4-Chlor-7-(3,4-dimethoxyphenyl)-9-methyl-2-propyl-pyrido[3',2':4,5]thieno[3,2-d]pyrimidin (Experiment 13c; allgemeine Formel 13: R₁=3,4-(MeO)₂-Ph, R₂=H, R₃=Me, R₄=nPr, Y=S)

Analog Verbindung 13a aus 7-(3,4-Dimethoxyphenyl)-9-methyl-2-propyl-pyrido[3',2':4,5]thieno[3,2-*d*]pyrimidin-4-on (Beispiel 12c; allgemeine Formel 12: R₁=3,4-(MeO)₂-Ph, R₂=H, R₃=Me, R₄=*n*Pr, Y=S) und Phosphoroxychlorid
Ausbeute: 0.58 g (96 %); HPLC t_{R}: 22.5 min; Reinheit: 98.1 % (s. a in Tab. 7);
MS (ESI, m/e): 414 [M+H]⁺

### Beispiel 50

### Beispiele zur Herstellung von Verbindungen der allgemeinen Formel 14

### 3-Amino-4-methyl-6-phenyl-thieno[2,3-b]pyridin-2-carbonsäureethylester (Experiment 14a; allgemeine Formel 14: R₁=Ph, R₂=H, R₃=Me, Y=S)

6.45 g (28.5 mmol) 2-Mercapto-4-methyl-6-phenyl-pyridin-3-carbonitril (Beispiel 2d; allgemeine Formel 2: R₁=Ph, R₂=H, R₃=Me, Y=S) werden in 100 ml abs. Ethanol vorgelegt und 5.3 ml (50.0 mmol) 2-Chloressigsäureethylester sowie 10 ml (72.1 mmol) Triethylamin hinzugefügt. Es wird 1 Stunde unter Rückfluß erhitzt und der nach Kühlen ausgefallene Niederschlag abgesaugt, mit wenig Wasser und Ethanol gewaschen, trocken gesaugt und i. Vak. bei 50 °C getrocknet.
Anschließend wird der Niederschlag in einer Lösung aus 0.14 g (1.6 mmol) Natriumethylat in abs. Ethanol 1 Stunde unter Rückfluß erhitzt. Der nach Abkühlen ausgefallene Niederschlag wird abgesaugt, mit 30 ml Wasser und 15 ml Ethanol gewaschen, trocken gesaugt und i. Vak. bei 50 °C getrocknet.
Ausbeute: 5.0 g (91 %); HPLC t_{R}: 18.0 min, Reinheit: 98.4 % (s. a in Tab. 7);
MS (ESI, m/e): 313 [M+H]⁺

### Beispiel 51

### Beispiele zur Herstellung von Verbindungen der allgemeinen Formel 15

### 3-(3-Benzoylthioureido)-4-methyl-6-phenyl-thieno[2,3-b]pyridin-2-carbonsäureethylester (Experiment 15a; allgemeine Formel 15: R₁=Ph, R₂=H, R₃=Me, Y=S)

Zu 2.78 g (8.9 mmol) 3-Amino-4-methyl-6-phenyl-thieno[2,3-*b*]pyridin-2-carbonsäure-ethylester (Beispiel 14a; allgemeine Formel 14: R₁=Ph, R₂=H, R₃=Me, Y=S) in 10 ml Aceton werden 5 ml (13.3 mmol) acetonischer Benzoylisothiocyanat-Lösung (2M) gegeben und 2 Stunden unter Rückfluß erhitzt. Der nach Abkühlen ausgefallene Niederschlag wird abgesaugt, mit wenig Wasser und Ethanol gewaschen, trocken gesaugt und i. Vak. bei 50 °C getrocknet.
Ausbeute: 3.93 g (93 %); HPLC t_{R}: 19.3 min, Reinheit: 99.9 % (s. a in Tab. 7);
MS (ESI, m/e): 476 [M+H]⁺

### Beispiel 52

### Beispiele zur Herstellung von Verbindungen der allgemeinen Formel 16

### 2-Ethylthio-9-methyl-7-phenyl-pyrido[3',2':4,5]thieno[3,2-d]pyrimidin-4-on (Experiment 16a; allgemeine Formel 16: R₁=Ph, R₂=H, R₃=Me, Alk*=Et, Y=S)

Zu 2.00 g (4.2 mmol) 3-(3-Benzoylthioureido)-4-methyl-6-phenyl-thieno[2,3-*b*]pyridin-2-carbonsäureethylester (Beispiel 15a; allgemeine Formel 15: R₁=Ph, R₂=H, R₃=Me, Y=S) in 25 ml Ethanol werden 10.5 ml (10.5 mmol) Natriumhydroxid-Lösung (1 N) gegeben und 30 Minuten unter Rückfluß erhitzt. Nach Zugabe von 10 ml N,N-Dimethylformamid, 5 ml (5 mmol) Natriumhydroxid-Lösung (1 N) und 0,50 ml lodethan (6.2 mmol) wird 30 Minuten bei Raumtemperatur gerührt. Der nach Ansäuern mit verd. Salzsäure ausgefallene Niedeschlag wird abgesaugt, mit Wasser gewaschen, trocken gesaugt und i. Vak. bei 50 °C getrocknet.
Ausbeute: 1.23 g (83 %), HPLC t_{R}: 18.5 min, Reinheit: 98.5 % (s. a in Tab. 7);
MS (ESI, m/e): 354 [M+H]⁺

### Beispiel 53

### Beispiele zur Herstellung von Verbindungen der allgemeinen Formel 17

### 4-Chlor-2-ethylthio-9-methyl-7-phenyl-pyrido[3',2':4,5]thieno[3,2-d]pyrimidin (Experiment 17a; allgemeine Formel 17: R₁=Ph, R₂=H, R₃=Me, Alk*=Et, Y=S)

1.23 g (3.5 mmol) 2-Ethylthio-9-methyl-7-phenyl-pyrido[3',2':4,5]thieno[3,2-*d*]pyrimidin-4-on (Beispiel 16a; allgemeine Formel 16: R₁=Ph, R₂=H, R₃=Me, Alk*=Et, Y=S) werden in 10 ml (10.9 mol) Phosphoroxychlorid 6 Stunden unter Rückfluß erhitzt. Anschließend wird auf ca. 100 g Eiswasser gegossen und mit gesättigter. Natriumhydrogencarbonat-Lösung neutralisiert. Der Niederschlag wird abgesaugt, mit ca. 100 ml Wasser gewaschen, getrocknet und durch Flash-Chromatographie (Kieselgel, Dichlormethan/Petrolether 5:1) gereinigt, wobei nach Elution der 1. Fraktion (s. Beispiel 17b) die Zielverbindung erhalten wird.
Ausbeute: 0.29 g (22 %); HPLC t_{R}: 23.8 min, Reinheit: 99.9 % (s. a in Tab. 7);
MS (ESI, m/e): 372 [M+H]⁺

### 4-Chlor-9-chlormethyl-2-ethylthio-7-phenyl-pyrido[3',2':4,5]thieno[3,2-d]pyrimidin

### (Experiment 17b; allgemeine Formel 17: R₁=Ph, R₂=H, R₃=CICH₂, Alk*=Et, Y=S)

Die Darstellung erfolgt analog Verbindung 17a. Die Zielverbindung wird durch Flash-Chromatographie (Kieselgel, Dichlormethan/Petrolether 5:1) als 1. Fraktion erhalten.
Ausbeute: 70 mg (5 %); HPLC t_{R}: 23.5 min, Reinheit: 99.7 % (s. a in Tab. 7);
MS (ESI, m/e): 406 [M+H]⁺

Die Verbindungen der allgemeinen Formel 1, 1 a bzw. 1 b sind in Tab. 1 zusammenfassend aufgeführt. Von ausgewählten Verbindungen der allgemeinen Formel 1a bzw. 1b sind ¹H-NMR-Daten in Tab. 2 aufgeführt.

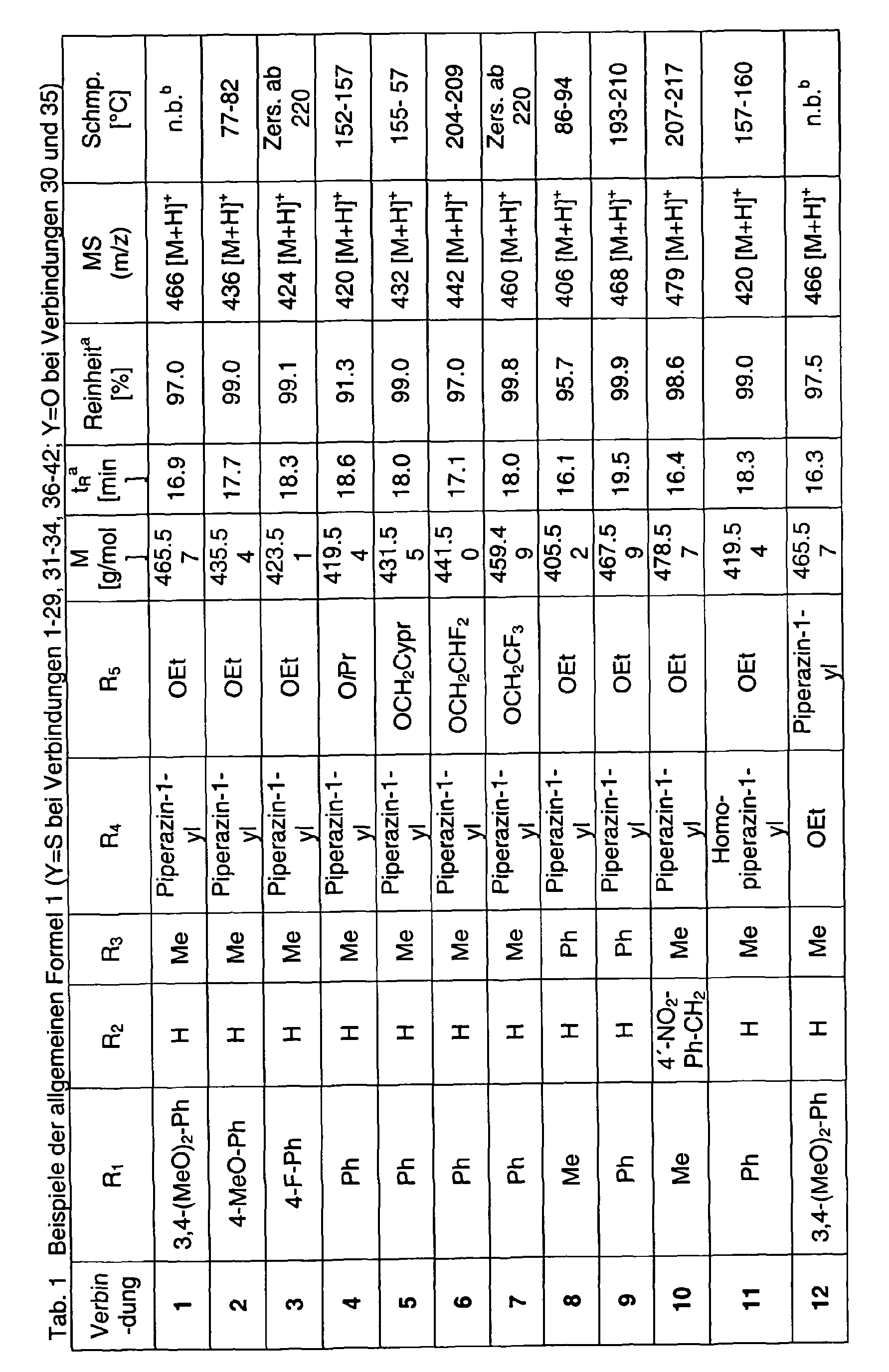

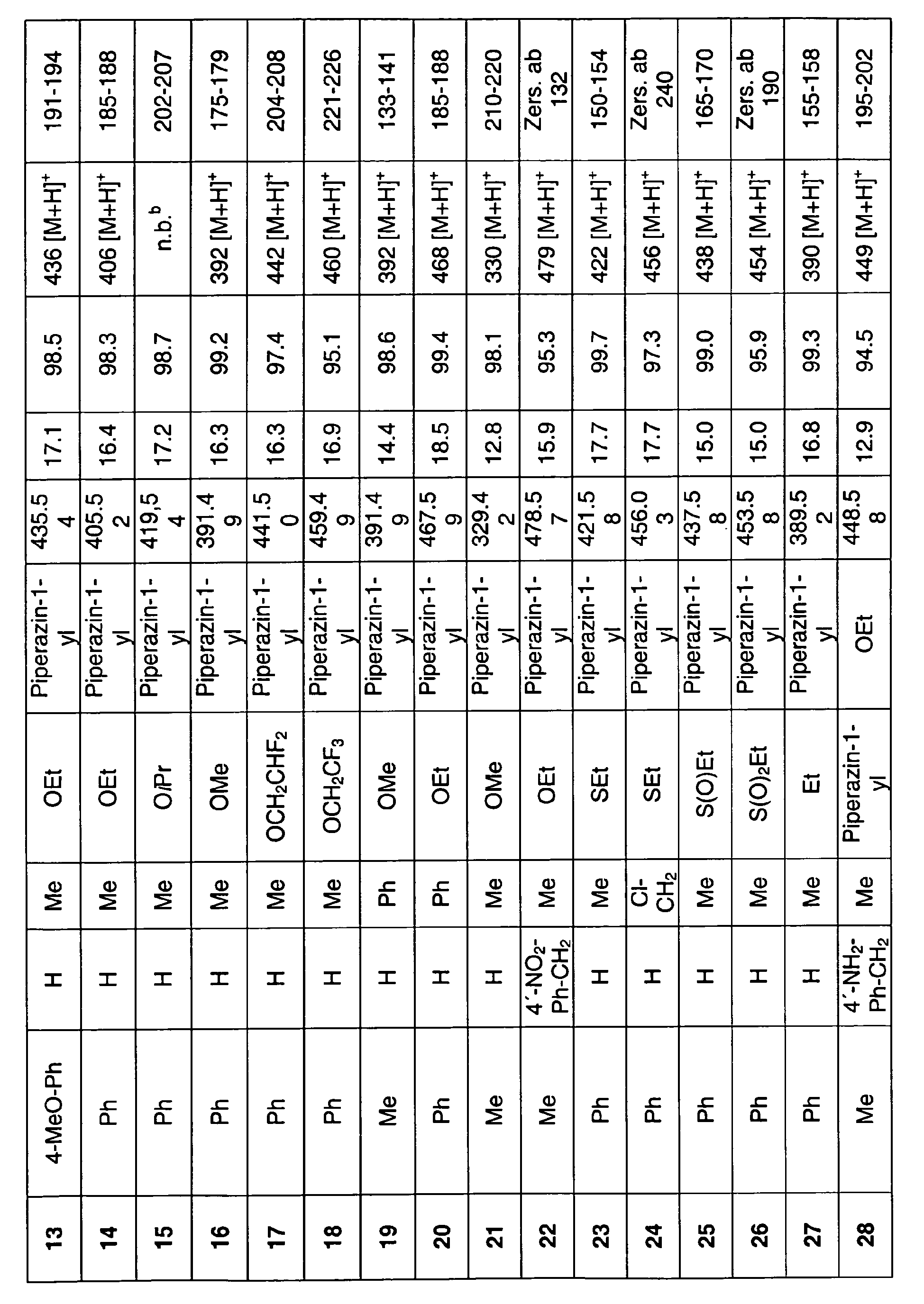

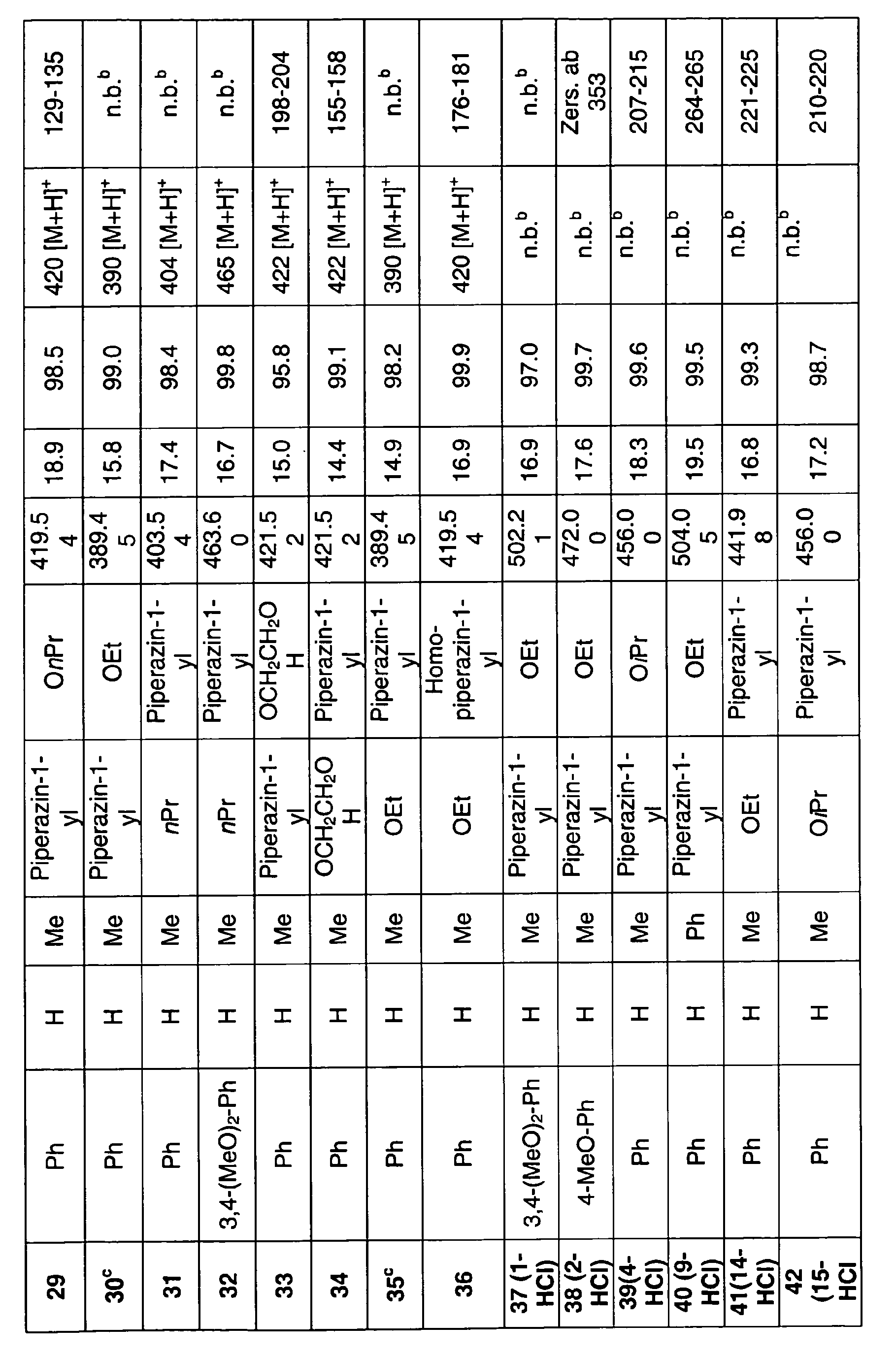

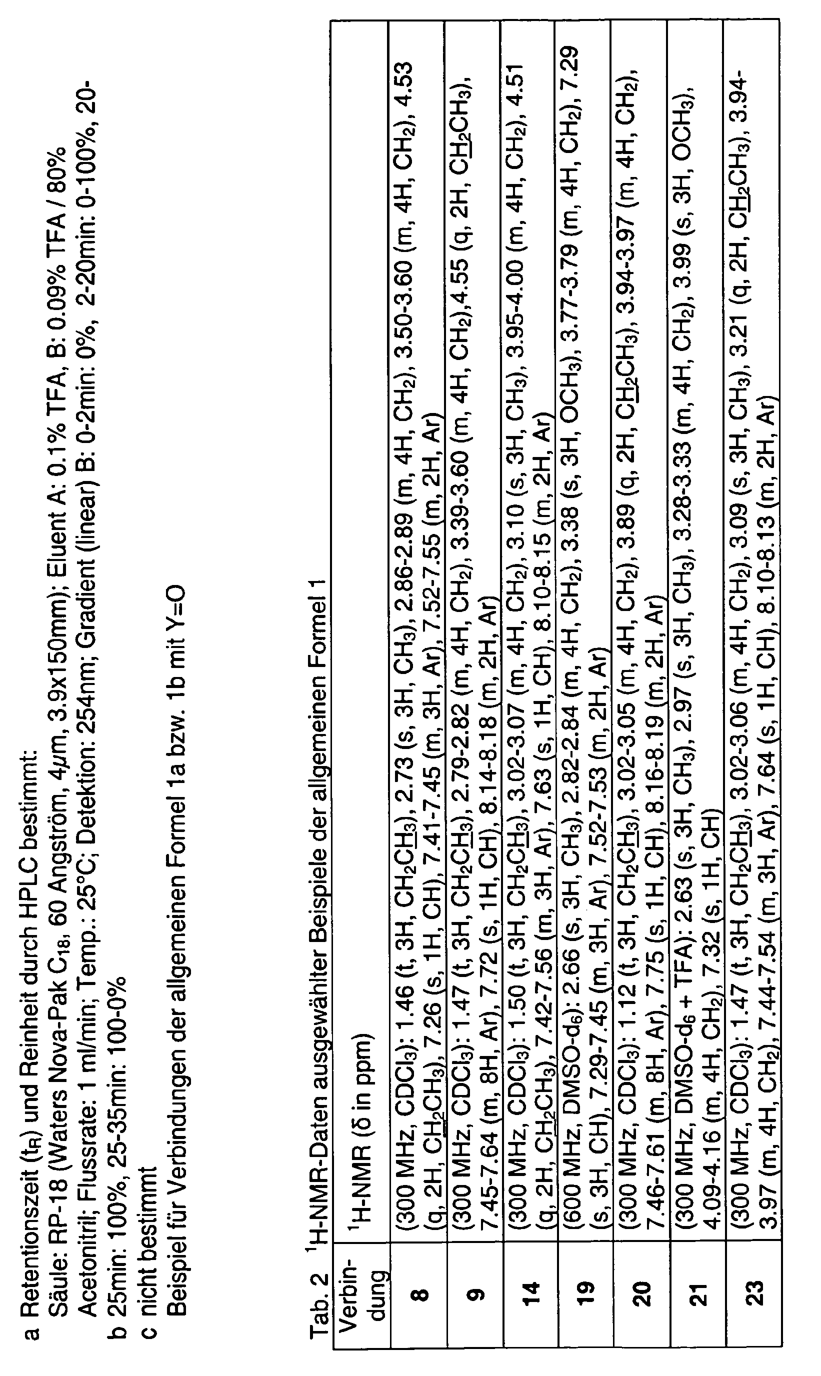

**Tab. 3 Beispiele der allgemeinen Formel 2 (Y=S)**

| Exp. | R₁ | R₂ | R₃ | M [g/mol] | t_{R}^{a} [min] | Reinheit^{a} [%] | MS (m/z) |
|---|---|---|---|---|---|---|---|
| 2a | 3,4-(MeO)₂-Ph | H | Me | 286.35 | 12.9 | 87.5 | 287 [M+H]⁺ |
| 2b^{b,c} | 4-MeO-Ph | H | Me | 256.32 | 13.4 | 89.0 | 257 [M+H]⁺ |
| 2c^{b,d} | 4-F-Ph | H | Me | 244.29 | 12.2 | 89.7 | 245 [M+H]⁺ |
| 2d^{b,e} | Ph | H | Me | 226.30 | 12.0 | 87.9 | 227 [M+H]⁺ |
| 2e^{f,g} | Me | H | Ph | 226.30 | 11.7 | 52.0 | 227 [M+H]⁺ |
| 2f^{b,h} | Ph | H | Ph | 288.37 | 15.8 | 88.5 | 289 [M+H]⁺ |
| 2g^{b,i} | Me | H | Me | 164.23 | 9.1 | 35.6 | 165 [M+H]⁺ |
| 2h^{j} | Me | 4'-NO₂-Ph-CH₂ | Me | 299.35 | 13.3 | 95.5 | 300 [M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9 x 150mm); Eluent A: 0.1 % TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: | | | | | | | |
| b 100%, 25-35min: 100-0% Darstellung erfolgt nach Methode A: Erhitzen unter Rückfluß für 3-4 Stunden; | | | | | | | |
| c nach Abkühlen wird der Ansatz ggf. filtriert und das Filtrat weiterverarbeitet | | | | | | | |
| d Beispiel beschrieben in *Vieweg H., Leistner S., Wagner G. Pharmazie (1986); 41, 827-830* | | | | | | | |
| e Beispiel beschrieben in *Vieweg H., Krasselt U., Boehm N., Prantz J., Wagner G.,* | | | | | | | |
| f *Pharmazie (1990); 45, 731-733* Beispiel beschrieben in *Schmidt U., Kubitzek H., Chem Ber (1960); 93, 1559-* | | | | | | | |
| g *1565* Darstellung erfolgt nach Methode A: Erhitzen unter Rückfluß für 3-4 Stunden; | | | | | | | |
| h nach Abkühlen wird der Niederschlag abgesaugt und analog dem Rückstand | | | | | | | |
| i weiterverarbeitet. | | | | | | | |
| j Beispiel beschrieben in *Krauze A., Bomika Z., Shestopalov A.M., Rodinovskaya L.A., Pelcers J., Duburs G., Sharanin Y.A., Promonenkov V.K. Khim Geterotsikl Soedin (1981); 377-382* Beispiel beschrieben in *Guerrera F., Siracusa M.A., Tornetta B. Farmaco, Ediz Scientif (1976); 31, 21-30* Beispiel beschrieben in *Wallenfels K., Schuly H., Ann (1959); 621, 215-221* Darstellung erfolgt nach Methode B, in Anlehnung an *Wagner G., Vieweg H., Leistner S., Böhm N., Krasselt U., Hanfeld V., Prantz J., Grupe R. Pharmazie (1990); 45, 102-109* | | | | | | | |

**Tab. 4 Beispiele der allgemeinen Formel 4 (Y=S)**

| Exp. | R₁ | R₂ | R₃ | M [g/mol] | t_{R}^{a} [min] | Reinheit^{a} [%] | MS (m/z) |
|---|---|---|---|---|---|---|---|
| 4a | 3,4-(MeO)₂-Ph | H | Me | 343.40 | 14.0 | 99.8 | 344 [M+H]⁺ |
| 4b | 4-MeO-Ph | H | Me | 313.37 | 14.8 | 99.9 | 314 [M+H]⁺ |
| 4c | 4-F-Ph | H | Me | 301.34 | 15.5 | 99.5 | 302 [M+H]⁺ |
| 4d^{b} | Ph | H | Me | 283.35 | 14.8 | 99.5 | 284 [M+H]⁺ |
| 4e^{b,c} | Me | H | Ph | 283.35 | 14.2 | 99.3 | 284 [M+H]⁺ |
| 4f^{d} | Ph | H | Ph | 345.42 | 17.5 | 99.2 | 346 [M+H]⁺ |
| 4g^{e} | Me | H | Me | 221.28 | 9.5 | 98.4 | 222 [M+H]⁺ |
| 4h | Me | 4'-NO₂-Ph-CH₂ | Me | 356.40 | 13.5 | 98.2 | 357[M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9x150mm); Eluent A: 0.1 % TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: | | | | | | | |
| b 100%, 25-35min: 100-0% Beispiel beschrieben in *Litvinov V.P., Sharanin Y.A., Rodinovskaya L.A.,* | | | | | | | |
| c *Shestopalov A.M., Mortikov V. Yu., Promonenkov V.K. Ser Khimich (1984); 12, 2760-2765* | | | | | | | |
| d Beispiel beschrieben in *Sharanin Y.A., ShestopalovA.M., Promonenkov V.K.* | | | | | | | |
| *e Zhurnal Organ Khimii (1984); 20, 2012-2020* Beispiel beschrieben in *Shestopalov A.M., Sharanin Y.A. Zhurnal Organ Khimii (1984); 20, 1991-2002* Beispiel beschrieben in *Tornetta B., Siracusa M.A., Ronsisvalle G., Guerrera F. Gazz. Chim. ltal. (1978); 108, 57-62* und *Shvedov V.l., Sycheva T.P., Sakovich T. V. Khimiya Geterot Soedin (1979); 1331-1335* | | | | | | | |

**Tab. 5 Beispiele der allgemeinen Formel 6 (Y=S bei a-h; Y= O bei i))**

| Exp. | R₁ | R₂ | R₃ | M [g/mol] | t_{R}^{a} [min] | Reinheit^{a} [%] | MS (m/z) |
|---|---|---|---|---|---|---|---|
| 6a^{b} | 3,4-(MeO)₂-Ph | H | Me | 369.39 | 13.8 | 99.9 | 368 [M-H]⁺ |
| 6b^{b} | 4-MeO-Ph | H | Me | 339.37 | 14.8 | 96.8 | 340 [M+H]⁺ |
| 6c^{b} | 4-F-Ph | H | Me | 327.33 | 15.4 | 99.8 | 328 [M+H]⁺ |
| 6d^{b} | Ph | H | Me | 309.34 | 14.2 | 98.3 | 310 [M+H]⁺ |
| 6e^{b} | Me | H | Ph | 309.34 | 13.0 | 99.5 | 310 [M+H]⁺ |
| 6f ^{b} | Ph | H | Ph | 371.41 | 17.3 | 98.6 | 372 [M+H]⁺ |
| 6g^{b,c} | Me | H | Me | 247.27 | 9.3 | 95.2 | 248 [M+H]⁺ |
| 6h^{b} | Me | 4'-NO₂-Ph-CH₂ | Me | 382.39 | 13.9 | 97.2 | 383 [M+H]⁺ |
| 6i^{d} | Ph | H | Me | 293.28 | 15.0 | 85.1 | 294 [M+H]⁺ |

| | | | | | | | |
|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9x150mm); Eluent A: 0.1 % TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | | | |
| b dargestellt nach Methode A | | | | | | | |
| c Beispiel beschrieben in Wagner, G., Böhm N., Pharmazie (1993), 48, 95-99 | | | | | | | |
| d dargestellt nach Methode B, Beispiel für Verbindungen der allgemeinen Formel 6 mit Y=O | | | | | | | |

**Tab. 6 Beispiele der allgemeinen Formel 7 (Y=S bei a - h; Y = O bei i)**

| Exp. | R₁ | R₂ | R₃ | M [g/mol | t_{R}^{a} [min] | Reinheit^{a} [%] | MS (m/z) |
|---|---|---|---|---|---|---|---|
| 7a | 3,4-(MeO)₂-Ph | H | Me | 406.29 | 21.8 | 85.5 | 407 [M+H]^{+b} |
| 7b | 4-MeO-Ph | H | Me | 376.26 | 22.8 | 95.5 | 376 [M+H]^{+b} |
| 7c | 4-F-Ph | H | Me | 364.22 | 22.7 | 89.4 | 364 [M+H]^{+b} |
| 7d | Ph | H | Me | 346.23 | 23.8 | 98.7 | 346 [M+H]^{+b} |
| 7e | Me | H | Ph | 346.23 | 20.6 | 98.2 | 346 [M+H]^{+b} |
| 7f | Ph | H | Ph | 408.30 | 23.7 | 99.7 | 408 [M+H]^{+b} |
| 7g | Me | H | Me | 284.16 | 18.2 | 99.4 | 284 [M+H]^{+b} |
| 7h | Me | 4'-NO₂-Ph-CH2 | Me | 419.28 | 20.3 | 79.5 | 419 [M+H]^{+b} |
| 7i^{c} | Ph | H | Me | 330.18 | 19.4 | 73.5 | 330 [M+H]^{+b} |

| | | | | | | | |
|---|---|---|---|---|---|---|---|
| a Retentionszeit (tR) und Reinheit durch HPLC bestimmt: Säule: RP-18 (Waters Nova-Pak C18, 60 Angström, 4µm, 3.9x150mm); Eluent A: 0.1 % TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: | | | | | | | |
| 100%, 25-35min: 100-0% | | | | | | | |
| b unter Berücksichtigung des reinen Isotopenpeaks | | | | | | | |
| c Beispiel für Verbindungen der allgemeinen Formel 7 mit Y=O | | | | | | | |

**Tab. 7 Beispiele der allgemeinen Formel 8 (Y=S bei a-s, u, w; Y=O bei t und v)**

| Exp. | R₁ | R₂ | R₃ | R₅ | M [g/mol] | t_{R}^{a} [min] | Reinheit^{a} [%] | MS (m/z) |
|---|---|---|---|---|---|---|---|---|
| 8a^{b} | 3,4-(MeO)₂-Ph | H | Me | Piperazin-1-yl | 455.96 | 16.2 | 81.4 | 456 [M+H]^{+c} |
| 8b^{b} | 4-MeO-Ph | H | Me | Piperazin-1-yl | 425.93 | 16.9 | 98.0 | 426 [M+H]^{+c} |
| 8c^{b} | Ph | H | Me | Piperazin-1-yl | 395.91 | 16.6 | 97.2 | 396 [M+H]^{+c} |
| 8d^{b} | Me | H | Ph | Piperazin- | 395.91 | 15.2 | 76.5 | n.b.^{d} |
| 8e^{b} | Ph | H | Ph | Piperazin-1-yl | 457.98 | 17.8 | 99.7 | 458 [M+H]^{+c} |
| 8f^{b} | Me | 4'-NO₂-Ph-CH₂ | Me | Piperazin-1-yl | 468.96 | 15.9 | 89.7 | 469 [M+H]^{+c} |
| 8g^{e} | 3,4-(MeO)₂- | H | Me | Oet | 415.89 | 21.5 | 99.9 | 416 [M+H]^{+c} |
| 8he | 4-MeO-Ph | H | Me | Oet | 385.87 | 23.7 | 95.5 | 386 [M+H]^{+c} |
| 8i^{e} | 4-F-Ph | H | Me | Oet | 373.83 | 23.3 | 97.1 | 374 [M+H]^{+c} |
| 8j^{e} | Ph | H | Me | Oet | 355.84 | 23.3 | 98.1 | 356 [M+H]^{+c} |
| 8k^{e} | Me | H | Ph | Oet | 355.84 | 21.4 | 98.6 | 356 [M+H]^{+c} |
| 8l^{e} | Ph | H | Ph | Oet | 417.91 | 24.5 | 94.5 | 418 [M+H]^{+c} |
| 8m^{e} | Me | 4'-NO₂-Ph-CH₂ | Me | Oet | 428.89 | 20.7 | 65.5 | 430 [M+H]^{+c} |
| 8n^{e} | Ph | H | Me | O*n*Pr | 369.87 | 26.1 | 96.3 | 370[M+H]^{+c} |
| 8o^{e} | Ph | H | Me | O*i*Pr | 369.87 | 24.5 | 87.0 | n.b.^{d} |
| 8p^{e} | Ph | H | Me | OCH₂Cypr | 381.89 | 23.7 | 99.0 | 382 [M+H]^{+c} |
| 8q^{e} | Ph | H | Me | OCH₂CHF₂ | 391.82 | 21.2 | 91.0 | 392 [M+H]^{+c} |
| 8r^{e} | Ph | H | M e | OCH₂CF₃ | 409.80 | 22.2 | 99.3 | 410 [M+H]^{+c} |
| 8s^{b} | Me | H | M e | Piperazin-1-yl | 332.83 | 13.6 | 98.2 | 334 [M+H]^{+c} |
| 8t^{e,f} | Ph | H | M e | Oet | 339.78 | 19.8 | 98.5 | 340 [M+H]^{+c} |
| 8u^{e} | Ph | H | M e | OCH₂CH₂ OH | 371.84 | 18.6 | 86.9 | 372 [M+H]^{+c} |
| 8v^{b,f} 8 | Ph Ph | H H | M e | Piperazin-1-yl | 379.84 | 14.9 14.9 99.0 | 99.0 | 380 [M+H]^{+c} |
| 8w^{b} | Ph | H | M | Homopiperazin-1-yl | 409.10 | 17.0 | 95.0 | 410 [M+H]^{+c} |

| | | | | | | | | |
|---|---|---|---|---|---|---|---|---|
| a Retentionszeit (t_{R}) und Reinheit durch HPLC bestimmt: Säule: RP-18 (Waters Nova-Pak C₁₈, 60 Angström, 4µm, 3.9x150mm); Eluent A: 0.1 % TFA, B: 0.09% TFA / 80% Acetonitril; Flussrate: 1 ml/min; Temp.: 25°C; Detektion: 254nm; Gradient (linear) B: 0-2min: 0%, 2-20min: 0-100%, 20-25min: 100%, 25-35min: 100-0% | | | | | | | | |
| b dargestellt nach Methode A | | | | | | | | |
| c unter Berücksichtigung des reinen Isotopenpeaks | | | | | | | | |
| d nicht bestimmt | | | | | | | | |
| e dargestellt nach Methode B | | | | | | | | |
| f Beispiel für Verbindungen der allgemeinen Formel 1 mit Y=O | | | | | | | | |

## Patentansprüche

1. Substituierte Pyrido[3',2':4,5]thieno[3,2-d]pyrimidine und Pyrido[3',2':4,5]furo[3,2-d]pyrimidine der allgemeinen Formel 1 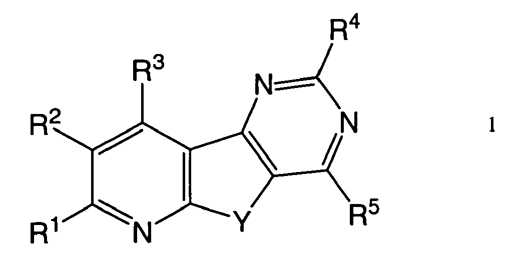
worin bedeuten:
Y Schwefel oder Sauerstoff und
R¹ und R³, gleich oder ungleich unabhängig voneinander,
- Wasserstoff,
- C₁₋₁₀Alkyl (ggf. mit R^{§} substituiert),
- C_{2-!2} Alkenyl und C_{2-!2} Alkinyl (jeweils ggf. mit R^{§} substituiert ),
- Difluormethyl, Trifluormethyl,
- Benzyl (ggf. mit R^{§} substituiert), Phenyl-(C₂₋₆)alkyl , Phenyl, 4-R^{§} -Phenyl, 3-R -Phenyl, 2-R^{§}- Phenyl, 3-R^{§},4-R^{§}-Phenyl, 3-R^{§},4-R^{§}-,5-R^{§} -Phenyl, 2R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, lsochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- Wasserstoff,
- C₁₋₁₂ Alkyl,
- C₂₋₁₂ Alkenyl und C₂₋₁₂ Alkinyl,
- Benzyl, Phenyl(C₂₋₆)alkyl (ggf. mit R^{§} ein oder mehrmals, gleich oder ungleich am aromatischen und/oder aliphatischen Molekülteil substituiert);
- Phenacyl (ggf. mit R^{§} ein oder mehrmals, gleich oder ungleich am aromatischen Molekülteil substituiert);
- Carboxyl, C₁₋₄ Alkoxycarbonyl, CONH₂, CONHAlk und CONAlk₂ (mit "Alk" jeweils C₁₋₆),
- R*CO- (worin R* Wasserstoff, C₁-C₁₂Alkyl bedeuten sowie ggf. mit R^{§} substituiert),
- Cyano, Nitro, Amino, C₁₋₆Alkylamino, Di(C₁₋₆ )alkylamino, -N=N-C₆H₅, -N=N-C₆H₄-R^{§},
- 1,3-Diphenyl-pyrazol-4-yl, Thiazolin-2-yl, Imidazolin-2-yl und 3,4,5,6-Tetrahydro-pyrimidinyl;
R⁴
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten aliphatischen Reste mit R§ substituiert);
- Phenyl, 4-R^{§} -Phenyl, 3-R^{§} -Phenyl, 2-R^{§}-Phenyl (jedoch ausgenommen: 2-NO₂-Phenyl), 3-R^{§},4-R^{§}-Phenyl, 3-R^{§},4-R^{§}-,5-R^{§} -Phenyl,
- 1-Naphthyl, 2-Naphthyl (jeweils ggf. mit R^{§} substituiert);
- S-Alkyl, SO-Alkyl, SO₂-Alkyl, (jeweils C₁-C₈),
S-Alkenyl, SO-Alkenyl, SO₂-Alkenyl (jeweils C₂-C₈)
S-Alkinyl, SO-Alkinyl, SO₂-Alkinyl (jeweils C₂-C₆)
(ggf. jeweils am C-Skelett der vorgenannten aliphatischen Reste mit -OH, -CN, -SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert;
- OR⁶, worin R⁶ bedeutet
- CH₃, C₂H₅, (CH₂) ₂CH₃, (CH₂) ₃CH₃, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃-₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
- C₂-₅Alkenyl und C₂-₅Alkinyl,
- C₃-₇ Cycloalkenyl,
- Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
- 1-Naphthyl, 2-Naphthyl (ggf. jeweils mit R^{§} substituiert),
- Pyridyl, Isochinolinyl, Chinolinyl, Acridinyl;
- NR⁷R⁸, worin dieser Substituent insgesamt bedeutet:
- Morpholino, Thiomorpholino, Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
- weitere Amino-Reste sekundärer, mono- oder polycyclischer cycloaliphatischer Amine mit insgesamt 5-14 Ringatomen, einschließlich der am C-Skelett mit R^{§} substituierte Vertreter;
- Amino-Reste sekundärer aliphatischer und aromatischer Amine, R⁷R⁸NH,
worin R⁷ und R⁸ unabhängig voneinander gleich oder ungleich sein können und bedeuten:
- C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
- Aminoreste primärer Amine, R⁷NH₂, ( worin R⁷ dasselbe wie oben bedeutet),
- Amino, NH₂, mit der Einschränkung, dass dann R⁵ nur die Bedeutung von OR⁶ hat ;
R⁵
- OR⁶, wobei R⁶ dasselbe wie oben bedeutet,
- NR⁷R⁸, mit gleicher Bedeutung wie oben, jedoch mit der Einschränkung, dass R⁴ dabei nicht ebenfalls die Bedeutung von NR ⁷R⁸ aufweist,
- Azido, Hydroxylamino, O-(C₁-₃)Alkylhydroxylamino,
- N-(C₁-₃)Alkylhydroxylamino, N,N-Di(C₁-₃)alkylhydroxylamino,
- Hydrazino, (C₁-₄)Alkyl- und Di(C₁-₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;
R^{§}: -OH, -SH, -O-C₁₋₈Alkyl, -O-C₆₋₁₄Aryl, -S-C₁₋₄Alkyl, -S-C₆₋₁₄Aryl, -SO-C₁₋₄Alkyl, -SO-C₆₋₁₄Aryl, -SO₂-C₁₋₄Alkyl, -SO₂-C₆₋₁₄Aryl, -SO₃H, -OSO₂C₁₋₈Alkyl, -OSO₂C₆₋₁₄Aryl, -COOH, -COOC₁₋₈Alkyl, -(CO)C₁₋₈Alkyl, -COOH, -COOC_{1...8}Alkyl, -CONH₂, -CONHC₁₋₆Alkyl, -CON(C₁₋₆Alkyl)₂, -NH₂, -NHC₁₋₆Alkyl, -N(C₁₋₆Alkyl)₂, -NHC₆₋₁₄Aryl, -NH-Hetaryl, -N(C₆₋₁₄Aryl)₂, -N(C₁₋₆Alkyl)(C₆₋₁₄Aryl), -CH₃, -CHF₂, -CF₃, -C₂H₅, -C(CH₃)₂, -(CH₂) ₂CH₃, -(CH₂) ₃CH₃, -CH₂CH₂OH, -CH₂CH₂SH, -CH₂CH₂SCH₃, -CH₂CF₃, -CH₂CCl₃, -CH₂CHF₂, -CH₂CHCl₂, -CH₂CH₂F, -CH₂CH₂CI, -CH₂CH₂Br, -Cylopropyl, -Cylopropylmethyl, -Cylobutyl, -Cyclobutylmethyl, -Cyclopentyl, -Cyclopentylmethyl, -Cylohexyl, -Cyclohexylmethyl, -F, -Cl, -Br, -1, -CN, -NO₂, und -SCN
sowie pharmazeutisch verträgliche Salze, Solvate, aktive Metabolite, Tautomere und Prodrugs dieser Verbindungen,
wobei folgende Verbindung ausgenommen ist:
Y = S, R¹ = OC₂H₅ , R² = CN, R³ = C₆H₅ , R⁴ = N(C₂H₅)₂, R⁵ = OC₂H₅ .

2. Substituierte Pyrido[3',2':4,5]thieno[3,2-d]pyrimidine und Pyrido[3',2':4,5]furo[3,2-d]pyrimidine nach Anspruch 1 mit der allgemeinen Formel 1 a oder 1 b : 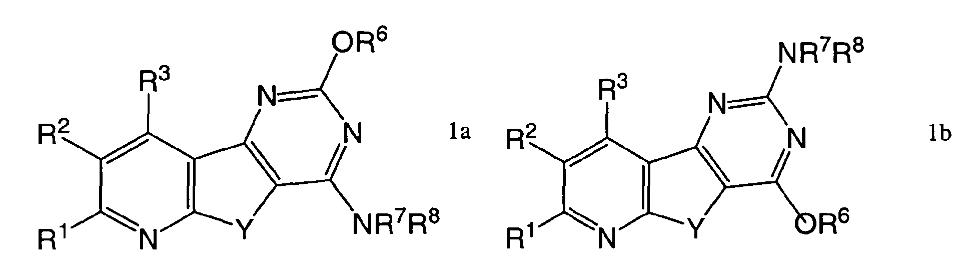
worin R¹, R², R³, R⁶, R⁷, R⁸ und Y die oben genannten Bedeutungen besitzen.

3. Substituierte Pyrido[3',2':4,5]thieno[3,2-d]pyrimidine und Pyrido[3',2':4,5]furo[3,2-d]pyrimidine nach Anspruch 1, wobei Y = S und die Substituenten R¹, R², R³, R⁴, R⁵ die folgenden Bedeutungen haben:
| R₁ | R₂ | R₃ | R₄ | R₅ |
|---|---|---|---|---|
| 3,4-(MeO)₂-Ph | H | Me | Piperazin-1-yl | OEt |
| 4-MeO-Ph | H | Me | Piperazin-1-yl | OEt |
| 4-F-Ph | H | Me | Piperazin-1-yl | OEt |
| Ph | H | Me | Piperazin-1-yl | O*i*Pr |
| Ph | H | Me | Piperazin-1-yl | OCH₂Cypr |
| Ph | H | Me | Piperazin-1- | OCH₂CHF₂ |
| Ph | H | Me | Piperazin-1-yl | OCH₂CF₃ |
| Me | H | Ph | Piperazin-1-yl | OEt |
| Ph | H | Ph | Piperazin-1-yl | OEt |
| Me | 4'-NO₂-Ph-CH₂ | Me | Piperazin-1-yl | OEt |
| Ph | H | Me | Homopiperazin-1-yl | OEt |
| 3,4-(MeO)₂-Ph | H | Me | OEt | Piperazin-1-yl |
| 4-Me0-Ph | H | Me | OEt | Piperazin-1-yl |
| Ph | H | Me | OEt | Piperazin-1-yl |
| Ph | H | Me | O*i*Pr | Piperazin-1-yl |
| Ph | H | Me | OMe | Piperazin-1-yl |
| Ph | H | Me | OCH₂CHF₂ | Piperazin-1-yl |
| Ph | H | Me | OCH₂CF₃ | Piperazin-1-yl |
| Me | H | Ph | OMe | Piperazin-1-yl |
| Ph | H | Ph | OEt | Piperazin-1-yl |
| Me | H | Me | OMe | Piperazin-1-yl |
| Me | 4'-NO₂-Ph-CH₂ | Me | OEt | Piperazin-1-yl |
| Ph | H | Me | SEt | Piperazin-1- |
| Ph | H | ClCH₂ | SEt | Piperazin-1-yl |
| Ph | H | Me | S(O)Et | Piperazin-1-yl |
| Ph | H | Me | S(O)₂Et | Piperazin-1-yl |
| Ph | H | Me | Et | Piperazin-1-yl |
| Me | 4'-NH₂-Ph-CH₂ | Me | Piperazin-1-yl | OEt |
| Ph | H | Me | Piperazin-1-yl | O*n*Pr |
| Ph | H | Me | Piperazin-1-yl | OEt |
| Ph | H | Me | *n*Pr | Piperazin-1-yl |
| 3,4-(MeO)₂-Ph | H | Me | *n*Pr | Piperazin-1-yl |
| Ph | H | Me | Piperazin-1-yl | OCH₂CH₂OH |
| Ph | H | Me | OCH₂CH₂OH | Piperazin-1-yl |
| Ph | H | Me | OEt | Piperazin-1-yl |
| Ph | H | Me | OEt | Homopiperazin-1-yl |
| 3,4-(MeO)₂-Ph | H | Me | Piperazin-1-yl | OEt |
| 4-MeO-Ph | H | Me | Piperazin-1-yl | OEt |
| Ph | H | Me | Piperazin-1-yl | O*i*Pr |
| Ph | H | Ph | Piperazin-1-yl | OEt |
| Ph | H | Me | OEt | Piperazin-1-yl |
| Ph | H | Me | O*i*Pr | Piperazin-1-yl |

4. Substituierte Pyrido[3',2':4,5]thieno[3,2-d]pyrimidine und Pyrido[3',2':4,5]furo[3,2-d]pyrimidine nach Anspruch 1, wobei Y = O und die Substituenten R¹, R², R³, R⁴, R⁵ die folgenden Bedeutungen haben:
| R₁ | R₂ | R₃ | R₄ | R₅ |
|---|---|---|---|---|
| Ph | H | Me | Piperazin-1-yl | oEt |
| Ph | H | Me | OEt | Piperazin-1-yl |

5. Substituierte Pyrido[3',2':4,5]thieno[3,2-d]pyrimidine und Pyrido[3',2':4,5]furo[3,2-d]pyrimidine nach Anspruch 1, wobei diese sind:
4-Ethoxy-7-morpholino-9-phenyl-2-(piperazin-1-yl)-pyrido[3',2':4,5]thieno[3,2-d]pyrimidin;
4-Ethoxy-7-(4-trifluo-rmethyl-phenyl)-9-methyl-2-(piperazin-1-yl)-pyrido[3',2':4,5]thieno[3,2-d]pyrimidin;
8-(4-Amino-benzyl)-4-ethoxy-7,9-dimethyl-2-(piperazin-1-yl)-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;
9-Methyl-7-phenyl-2-(piperazin-1-yl)-4-n-propoxy-pyrido[3',2':4,5]thieno[3,2-d]pyrimidin;
4-Ethoxy-7-thien-2-yl-9-trifluor-methyl-2-(piperazin-1-yl)-pyrido[3',2':4,5]thieno[3,2-d]pyrimidin;
2-Ethoxy-7-(4-trifluor-methyl-phenyl)-9-methyl-4-(piperazin-1-yl)-pyrido[3',2':4,5]thieno[3,2-d]pyrimidin;
2-(2-Hydroxy-ethoxy)-9-methyl-7-phenyl-4-(piperazin-1-yl)-pyrido[3',2':4,5]thieno[3,2-d]pyrimidin;
2-Ethoxy-7-methyl-9-phenyl-4-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin-hydrochlorid;
4-Ethoxy-7-(4-methoxy-phenyl)-9-methyl-2-(piperazin-1-yl)-pyrido[3',2':4,5]thieno[3,2-d]pyrimidin;
4-Ethoxy-7-phenyl-9-methyl-2-(piperazin-1-yl)-pyrido[3',2':4,5]furo[3,2-d]pyrimidin;
2-(2,2-Difluor-ethoxy)-9-methyl-7-phenyl-4-(piperazin-1-yl)-pyrido[3',2':4,5]thieno[3,2-d]pyrimidin;
2-Ethoxy-7,9-di-*iso*-propyl-4-(piperazin-1-yl)-pyrido[3',2':4,5]thieno[3,2-d]pyrimidin;
7-(3,4-Dimethoxy-phenyl)-9-methyl-4-(piperazin-1-yl)-2-n-propyl-pyrido[3',2':4,5]thieno[3,2-d]pyrimidin;
2-Ethoxy-9-methyl-7-(4-methyl-phenyl)-4-(piperazin-1-yl)-pyrido[3',2':4,5]thieno[3,2-d]pyrimidin;
9-Methyl-7-phenyl-2-(piperazin-1-yl)-4-iso-propoxy-pyrido[3',2':4,5]thieno[3,2-d]pyrimidin;
4-(2-Hydroxy-ethoxy)-9-methyl-7-phenyl-2-(piperazin-1-yl)-pyrido[3',2':4,5]thieno[3,2-d]pyrimidin;
2-Ethoxy-7-thien-2-yl-9-trifluor-methyl-4-(piperazin-1-yl)-pyrido[3',2':4,5]thieno[3,2-d]pyrimidin;
2-Ethoxy-7-(3,4-dimethoxy-phenyl)-9-methyl-4-(piperazin-1-yl)-pyrido[3',2':4,5]thieno[3,2-d]pyrimidin;
2-Ethoxy-7-(4-methoxy-phenyl)-9-methyl-4-(piperazin-1-yl)-pyrido[3',2':4,5]thieno[3,2-d]pyrimidin;
2-Ethoxy-9-methyl-7-thien-2-yl-9-methyl-4-(piperazin-1-yl)-pyrido[3',2':4,5]thieno[3,2-d]pyrimidin;
4-Ethoxy-9-methyl-7-(4-methyl-phenyl)-2-(piperazin-1-yl)-pyrido[3',2':4,5]thieno[3,2-d]pyrimidin;
4-Ethoxy-7,9-dimethyl-8-(4-nitro-benzyl)-2-(piperazin-1-yl)-pyrido[3',2':4,5]thieno[3,2-d]pyrimidin;
4-Ethoxy-7-(4-fluor-phenyl)-9-methyl-2-(piperazin-1-yl)-pyrido[3',2':4,5]thieno[3,2-d]pyrimidin;
2-Ethoxy-7,9-dimethyl-8-(4-nitro-benzyl)-4-(piperazin-1-yl)-pyrido[3',2':4,5]thieno[3,2-d]pyrimidin;
4-Ethoxy-7,9-diphenyl-2-(piperazin-1-yl)-pyrido[3',2':4,5]thieno[3,2-d]pyrimidin;
4-Ethoxy-7-methyl-9-phenyl-2-(piperazin-1-yl)-pyrido[3',2':4,5]thieno[3,2-d]pyrimidin;
2-Ethoxy-9-trifluor-methyl-7-phenyl-4-(piperazin-1-yl)-pyrido[3',2':4,5]thieno[3,2-d]pyrimidin;
4-Ethoxy-7,9-di-iso-propyl-2-(piperazin-1-yl)-pyrido[3',2':4,5]thieno[3,2-d]pyrimidin;
9-Methyl-7-phenyl-2-(piperazin-1-yl)-4-iso-propoxy-pyrido[3',2':4,5]thieno[3,2-d]pyrimidin-hydrochlorid;
9-Methyl-7-phenyl-4-(piperazin-1-yl)-2-*iso*-propoxy-pyrido[3',2':4,5]thieno[3,2-d]pyrimidin-hydrochlorid;
9-Methyl-7-phenyl-4-(piperazin-1-yl)-2-n-propyl-pyrido[3',2':4,5]thieno[3,2-d]pyrimidin;
4-Ethoxy-9-methyl-8-(4-nitro-benzyl)-7-phenyl-2-(piperazin-1-yl)-pyrido[3',2':4,5]thieno[3,2-d]pyrimidin;
4-Ethoxy-9-methyl-7-thien-2-yl-2-(piperazin-1-yl)-pyrido[3',2':4,5]thieno[3,2-d]pyrimidin;
4-Ethoxy-9-trifluor-methyl-7-phenyl-2-(piperazin-1-yl)-pyrido[3',2':4,5]thieno[3,2-d]pyrimidin;
4-(2,2-Difluor-ethoxy)-9-methyl-7-phenyl-2-(piperazin-1-yl)-pyrido[3',2':4,5]thieno[3,2-d]pyrimidin;
4-Ethoxy-7-(3,4-dimethoxy-phenyl)-9-methyl-2-(piperazin-1-yl)-pyrido[3',2':4,5]thieno[3,2-d]pyrimidin;
4-Ethoxy-7,9-diphenyl-2-(piperazin-1-yl)-pyrido[3',2':4,5]thieno[3,2-d]pyrimidin-hydrochlorid;
2-Ethoxy-7-methyl-9-phenyl-4-(piperazin-1-yl)-pyrido[3',2':4,5]thieno[3,2-d]pyrimidin-hydrochlorid;
4-Ethoxy-7-(4-methoxy-phenyl)-9-methyl-2-(piperazin-1-yl)-pyrido[3',2':4,5]thieno[3,2-d]pyrimidin-hydrochlorid.

6. Pharmazeutische Zusammensetzung enthaltend mindestens eine der Verbindungen nach einem der Ansprüche 1-5 sowie ggf. pharmazeutisch verträgliche Hilfsstoffe und/oder Träger.

7. Pharmazeutische Zusammensetzung nach Anspruch 6 enthaltend zusätzlich einen oder mehrere der folgenden Wirkstoffe:
- β₂-Adrenoceptor Agonisten
- Dinatriumcromoglycat
- Korticosteroide
- Leukotrien-Antagonisten (entweder Enzym-Inhibitoren [wie 5-Lipoxygenaseinhibitoren oder Arachidonsäure-Enzyminhibitoren] oder Rezeptorantagonisten) ,
- Antihistaminika (bevorzugt solche mit Mastzellen-stabilisierenden Eigenschaften oder Leukotrien-antagonisierenden Aspekten)
- Theophyllin
- Muscarinrezeptor-Antagonisten
- (monoklonale) Antikörper gegen TNF-alpha oder andere Wirkstoffe, die die Bildung bzw. Freisetzung von TNF-alpha oder die Aktivität von TNF-alpha hemmen

8. Verwendung der substituierten Pyrido[3',2':4,5]thieno[3,2-d]pyrimidine und Pyrido[3',2':4,5]furo[3,2-d]pyrimidine nach einem der Ansprüche 1-5 zur Herstellung eines Arzneimittels zur Inhibierung des Enzyms Phosphodiesterase 4 (PDE4) und/oder TNF alpha-Freisetzung.

9. Verwendung nach Anspruch 8 zur Behandlung von Asthma bronchiale, COPD, Rheumatoide Arthritis (RA), Osteoarthritis, Multiple Sklerose, Guillain-Barré Syndrom, Mb. Crohn, Colitis ulcerosa, Psoriasis, atopische Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ 1, neurodegenerative Erkrankungen, insbesondere Mb. Alzheimer und Mb. Parkinson, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen, virale Erkrankungen.
